(19) Europäisches Patentamt
European Patent Office
Office européen des brevets

(11) EP 4 349 813 A1

# (12) EUROPEAN PATENT APPLICATION
published in accordance with Art. 153(4) EPC

(43) Date of publication:
**10.04.2024 Bulletin 2024/15**

(21) Application number: **22810638.1**

(22) Date of filing: **26.05.2022**

(51) International Patent Classification (IPC):
***C07D 211/60*** (2006.01) ***C07D 225/02*** (2006.01)
***C07D 401/06*** (2006.01) ***A61K 31/452*** (2006.01)
***A61P 23/02*** (2006.01)

(52) Cooperative Patent Classification (CPC):
**A61K 31/452; A61P 23/02; C07D 211/60; C07D 225/02; C07D 401/06**

(86) International application number:
**PCT/CN2022/095336**

(87) International publication number:
**WO 2022/247914 (01.12.2022 Gazette 2022/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.05.2021 CN 202110587550**

(71) Applicant: **Yichang Humanwell Pharmaceutical Co., Ltd.**
**Yichang, Hubei 443005 (CN)**

(72) Inventors:
- **ZHOU, Hao**
  **Yichang, Hubei 443005 (CN)**
- **LIANG, Dali**
  **Yichang, Hubei 443005 (CN)**
- **LIU, Zewen**
  **Yichang, Hubei 443005 (CN)**
- **ZHOU, Xiujie**
  **Yichang, Hubei 443005 (CN)**
- **CHEN, Lei**
  **Yichang, Hubei 443005 (CN)**
- **LIAO, Zongquan**
  **Yichang, Hubei 443005 (CN)**
- **LV, Jinliang**
  **Yichang, Hubei 443005 (CN)**
- **LI, Lie**
  **Yichang, Hubei 443005 (CN)**
- **TIAN, Luanyuan**
  **Yichang, Hubei 443005 (CN)**

(74) Representative: **Modiano, Gabriella Diana et al**
**Modiano & Partners (DE)**
**Steinsdorfstrasse, 14**
**80538 München (DE)**

## (54) QUATERNARY AMMONIUM SALT COMPOUND, PREPARATION METHOD THEREFOR AND USE THEREOF

(57) Provided are a quaternary ammonium salt compound, a preparation method therefor and a use thereof. The compound represented by general formula (I), or an isomer or pharmaceutically acceptable salt thereof, and a composition thereof can be used for the preparation of anesthetic or analgesic drugs. Each substituent of general formula (I) is the same as in the definition of the description.

EP 4 349 813 A1

## Description

Cross-Reference to Related Application

The present application claims priority to Chinese invention patent application No. CN202110587550.8, filed on May 27, 2021 and titled "Quaternary Ammonium Salt Compound, Preparation Method Therefor and Use Thereof", the content of which is hereby incorporated by reference in its entirety.

Technical Field

The present application relates to, but is not limited to, the technical field of medicinal chemistry, in particular to a quaternary ammonium salt compound, preparation method therefor and use thereof.

Background

Local anesthetics are a kind of drugs which are used locally around nerve trunks or nerve endings, and they are capable of temporarily, completely, and reversibly blocking generation and conduction of nerve impulses to cause a temporary loss of localized pain sensation. The mechanism of action of local anesthetics is to reduce inward flow of $Na^+$ in cell membrane and change the membrane potential by bonding to the binding site of sodium channel on nerve cell membrane, thus preventing conduction of nerve impulses and achieving anesthetic effect. Due to the advantages of definite effect, being less prone to hyperalgesia, convenient local administration, low blood concentration, and fewer systemic side effects, etc., local anesthetics are still pain treatment means that are relatively frequently used in clinical practice.

At present, clinically used local anesthetics are mainly a kind of compounds formed by linking aromatic groups and amino groups through ester bonds or amide bonds, such as procaine, tetracaine, lidocaine, bupivacaine, and ropivacaine. Although bupivacaine and ropivacaine are considered as new long-acting local anesthetics, their analgesic duration usually does not exceed 8h for a single dose ("Local anesthetics: review of pharmacological considerations", Becker D E. et al., Anesth Prog. 2012, 59(2): 90-102.). Although it can meet the needs of most surgical or invasive operations, it is far from fully meeting the needs of postsurgical pain, chronic pain, etc.

QX-314, a quaternary ammonium salt derivative of lidocaine, can effectively block sodium ion current and produce lasting anesthetic effect after passing through cell membrane, but it is difficult to actively pass through lipid-soluble cell membrane because it is permanently positively charged ("Mechanism of frequency-dependent inhibition of sodium currents in frog myelinated nerve by the lidocaine derivative GEA", Courtney KR. J Pharmacol Exp Ther.1975, 195:225-236). Studies have shown that QX-314 combined with local anesthetics or transient receptor channel (TRP) agonists can penetrate cell membrane and produce long-lasting local anesthetic effect, but it will lead to increased local neurotoxicity and systemic toxicity ("Anti-nociceptive and desensitizing effects of olvanil on capsaicin-induced thermal hyperalgesia in the rat", Alsalem M. et al., BMC Pharmacol Toxicol. 2016, 17(1): 31.).

Summary

A first aspect of the present application provides a quaternary ammonium salt compound represented by Formula (I), or a tautomer, geometric isomer, enantiomer, diastereomer thereof, or a mixture thereof, or a pharmaceutically acceptable salt thereof:

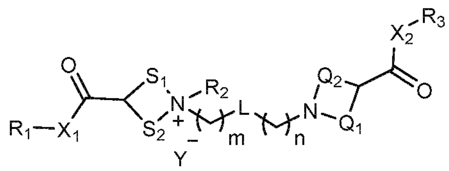

(I)

wherein:

$R_1$ is selected from an aromatic hydrocarbon group and heteroaryl;

$R_2$ is selected from $C_{1-18}$ alkyl, $C_{3-12}$ cycloalkyl;

$R_3$ is selected from $C_{1-8}$ alkyl, $C_{3-12}$ cycloalkyl, an aromatic hydrocarbon group, heteroaryl, and heterocycloalkyl;

$X_1$ and $X_2$ are each independently selected from O, S, and $NR_4$, wherein $R_4$ is hydrogen, deuterium, $C_{1-8}$ alkyl, $C_{3-8}$ cycloalkyl, $C_{1-8}$ alkoxy $C_{1-8}$ alkyl, or, $NR_4$ and $R_1$ or $R_3$ connected thereto together form an azacycloalkyl;

m and n are each independently selected from an integer of 0-8, and m and n may be the same or different;

L is selected from $C_{1-8}$ alkylene, $C_{2-8}$ alkenylene, $C_{2-8}$ alkynylene, and $C_{3-8}$cycloalkylene;

$S_1$, $S_2$, $Q_1$, and $Q_2$ are each independently selected from a single bond (i.e., atoms connected thereto are directly bond-connected) and $C_{1-6}$ alkylene, wherein a carbon atom on the main chain of $C_{1-6}$ alkylene is optionally substituted by a heteroatom selected from O, S, and N, wherein N may be substituted by $R_6$, $R_6$ is selected from hydrogen and deuterium, and provided that $S_1$ and $S_2$ are not single bonds at the same time (i.e., atoms connected thereto are directly bond-connected), $Q_1$ and $Q_2$ are not single bonds at the same time (i.e., atoms connected thereto are directly bond-connected); and

$Y^-$ represents a pharmaceutically acceptable anion.

A second aspect of the present application relates to a preparation method for the compound of General Formula (I) of the present application, a tautomer, geometric isomer, enantiomer, diastereomer thereof, or a mixture thereof, or a pharmaceutically acceptable salt thereof, the preparation method including the following steps:

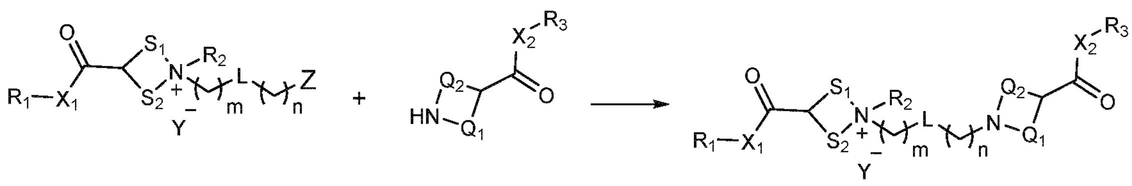

(II) (III) (I)

reacting a compound of Formula (II) with a compound of Formula (III) to obtain the compound of Formula (I);

wherein Z in Formula (II) is an electron-withdrawing leaving group, optionally the leaving group is bromine, chlorine, or sulfonate, and definitions of groups in Formula (II) and Formula (III) are the same as those in Formula (I).

A third aspect of the present application relates to a pharmaceutical composition, including the compound of General Formula (I) of the present application, a tautomer, geometric isomer, enantiomer, diastereomer thereof, or a mixture thereof, or a pharmaceutically acceptable salt thereof, and a pharmaceutically acceptable carrier excipient or a diluent.

A fourth aspect of the present application relates to the compound of General Formula (I) of the present application, a tautomer, geometric isomer, enantiomer, diastereomer thereof, or a mixture thereof, or a pharmaceutically acceptable salt thereof, for use as a medication.

A fifth aspect of the present application relates to use of the compound of General Formula (I) of the present application, a tautomer, geometric isomer, enantiomer, diastereomer thereof, or a mixture thereof, or a pharmaceutically acceptable salt thereof in the preparation of anesthetic or analgesic drugs.

A sixth aspect of the present application relates to a method for anesthesia or analgesia, including administering the compound of General Formula (I) of the present application, a tautomer, geometric isomer, enantiomer, diastereomer thereof, or a mixture thereof, or a pharmaceutically acceptable salt thereof, or the pharmaceutical composition to a patient in need thereof.

Detailed Description

In view of the problems of QX-314, more and more researchers are showing great interest in finding novel, long-acting, and safe local anesthetics. For example, CN110156665B discloses a new class of quaternary ammonium salt compounds with both long-acting local anesthetic effect and a selective local anesthetic effect (sensory nerve block time is longer than motor nerve block time), which have the advantages of long local anesthetic effect time, good local anesthetic selectivity, less nerve injury and high safety, as compared with the existing QX314, QX314 compositions and

long-chain compounds with surfactant structural features. CN110156666A discloses a novel quaternary ammonium cationic compound with both long-acting local anesthetic effect and a selective local anesthetic effect (sensory nerve block time is longer than motor nerve block time), which have the advantages of long-acting local anesthetic effect time, good local anesthetic selectivity, less nerve injury and high safety, as compared with the existing QX314, QX314 compositions and long-chain compounds with surfactant structural features. CN101050200A discloses a class of alkene-substituted amide derivatives and use thereof in the field of local anesthesia. Results show that they have certain local anesthetic effect, and their acute toxicity is less than that of levobupivacaine hydrochloride.

The present application provides a new kind of quaternary ammonium salt compounds, a preparation method including the compound, and use thereof, which have the advantages of having a novel structure, maintaining long-term local anesthesia after a single dose, and causing no motor nerve injuries.

The first aspect of the present application provides a quaternary ammonium salt compound represented by Formula (I), or a tautomer, geometric isomer, enantiomer, diastereomer thereof, or a mixture thereof, or a pharmaceutically acceptable salt thereof:

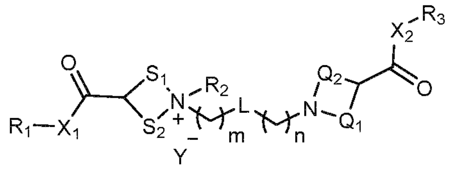

(I)

wherein:

$R_1$ is selected from an aromatic hydrocarbon group and heteroaryl, herein, the aromatic hydrocarbon group and heteroaryl are optionally substituted by one or more of the following groups: $C_{1-6}$ alkyl, $C_{1-6}$ alkoxy, cyano, halogen, hydroxyl, amino, nitro, an ester group, mono $C_{1-6}$ alkyl amino, di-$C_{1-6}$ alkyl amino, $C_{2-4}$ alkenyl, $C_{2-4}$ alkynyl, $C_{1-6}$ haloalkyl, and $C_{1-6}$ haloalkoxy;

$R_2$ is selected from $C_{1-18}$ alkyl and $C_{3-12}$ cycloalkyl, herein, the $C_{1-18}$ alkyl and $C_{3-12}$ cycloalkyl are optionally substituted by one or more of the following groups: $C_{1-6}$ alkyl, $C_{1-6}$ alkoxy, $C_{1-6}$ haloalkyl, $C_{3-6}$ cycloalkyl, halogen, hydroxyl, cyano, and amino;

$R_3$ is selected from $C_{1-8}$ alkyl, $C_{3-12}$ cycloalkyl, an aromatic hydrocarbon group, heteroaryl, and heterocycloalkyl, wherein optionally, herein, the $C_{1-8}$ alkyl, $C_{3-12}$ cycloalkyl, an aromatic hydrocarbon group, heteroaryl, and heterocycloalkyl are optionally substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, $C_{2-4}$ alkynyl, an optionally substituted aromatic hydrocarbon group, optionally substituted heteroaryl, optionally substituted heterocycloalkyl; herein, the optionally substituted aromatic hydrocarbon group, optionally substituted heteroaryl, optionally substituted heterocycloalkyl refer to an unsubstituted aromatic hydrocarbon group, unsubstituted heteroaryl, and unsubstituted heterocycloalkyl; or the aromatic hydrocarbon group, heteroaryl, and heterocycloalkyl are substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, and $C_{2-4}$ alkynyl;

$X_1$ and $X_2$ are each independently selected from O, S, and $NR_4$, wherein $R_4$ is hydrogen, deuterium, $C_{1-8}$ alkyl, $C_{3-8}$ cycloalkyl, $C_{1-8}$ alkoxy $C_{1-8}$ alkyl, or $NR_4$ and $R_1$ or $R_3$ connected thereto together form azacycloalkyl;

m and n are each independently selected from an integer of 0-8, and m and n may be the same or different;

L is selected from $C_{1-8}$ alkylene, $C_{2-8}$ alkenylene, $C_{2-8}$ alkynylene, and $C_{3-8}$ cycloalkylene, wherein optionally, herein, a carbon atom on the main chain of the $C_{1-8}$ alkylene and $C_{3-8}$ cycloalkylene is optionally substituted by 1-3 heteroatoms selected from O, S, and N, wherein N may be substituted by $R_5$, $R_5$ is selected from hydrogen, deuterium, $C_{1-4}$ alkyl; the $C_{1-8}$ alkylene, $C_{2-8}$ alkenylene, $C_{2-8}$ alkynylene, and $C_{3-8}$ cycloalkylene are optionally substituted by one or more of the following groups: $C_{1-4}$ alkyl, $C_{1-4}$ alkoxy, $C_{1-4}$haloalkyl, $C_{1-4}$haloalkoxy, $C_{3-6}$ cycloalkyl, halogen, hydroxyl, cyano, amino, an ester group, nitro, mono $C_{14}$ alkyl amino, di-$C_{1-4}$ alkyl amino, $C_{2-4}$ alkenyl, and $C_{2-4}$ alkynyl;

$S_1$, $S_2$, $Q_1$, and $Q_2$ are each independently selected from a single bond (i.e., atoms connected thereto are directly

bond-connected) and $C_{1-6}$ alkylene, herein, a carbon atom on the main chain of $C_{1-6}$ alkylene is optionally substituted by a heteroatom selected from O, S and N, wherein N may be substituted by $R_6$, $R_6$ is selected from hydrogen and deuterium, and it is specified that $S_1$ and $S_2$ are not single bonds at the same time (i.e., atoms connected thereto are directly bond-connected), $Q_1$ and $Q_2$ are not single bonds at the same time (i.e., atoms connected thereto are directly bond-connected); and

$Y^-$ represents a pharmaceutically acceptable anion.

In some embodiments of the present application, in the compound of Formula (I), or a tautomer, geometric isomer, enantiomer, diastereomer thereof, or a mixture thereof, or a pharmaceutically acceptable salt thereof provided by the present application, $R_1$ is phenyl or naphthyl; herein, the phenyl and naphthyl are optionally substituted by one or more of the following groups: $C_{1-6}$ alkyl, $C_{1-6}$ alkoxy, cyano, halogen, hydroxyl, amino, nitro, an ester group, mono $C_{1-6}$ alkyl amino, di-$C_{1-6}$ alkyl amino, $C_{2-4}$ alkenyl, $C_{2-4}$ alkynyl, $C_{1-4}$ haloalkyl, $C_{1-6}$ haloalkoxy.

In some embodiments of the present application, in the compound of Formula (I), or a tautomer, geometric isomer, enantiomer, diastereomer thereof, or a mixture thereof, or a pharmaceutically acceptable salt thereof provided by the present application, $R_1$ is phenyl, optionally substituted by one or more of the following groups: methyl, ethyl, n-propyl, isopropyl, n-butyl, sec-butyl, tert-butyl, trifluoromethyl, methoxy, trifluoromethoxy, fluorine, chlorine, bromine, iodine, hydroxyl, amino, nitro, methyl ester, ethyl ester; more preferably, $R_1$ is phenyl, 2-methylphenyl, 2-methoxyphenyl, 4-methylphenyl, 4-methoxyphenyl, 4-fluorophenyl, 2-chlorophenyl, 4-chlorophenyl, 2-bromophenyl, 3-bromophenyl, 4-bromophenyl, 3-hydroxyphenyl, 4-trifluoromethylphenyl, 2,6-dimethylphenyl, 2,6-dimethoxyphenyl, 3-nitrophenyl, 2,6-difluorophenyl, 3-chloro-2-methylphenyl, 2,3-dichlorophenyl, 4-hydroxyphenyl, 2,4,6-trimethylphenyl, 2,4,6-trimethoxyphenyl, and 2,4,6-trifluorophenyl.

In some embodiments of the present application, in the compound of Formula (I), or a tautomer, geometric isomer, enantiomer, diastereomer thereof, or a mixture thereof, or a pharmaceutically acceptable salt thereof provided by the present application, $R_1$ is phenyl, 2-methylphenyl, 2-methoxyphenyl, 4-methylphenyl, 4-methoxyphenyl, 4-fluorophenyl, 4-trifluoromethylphenyl, 2-chlorophenyl, 4-chlorophenyl, 2-bromophenyl, 3-bromophenyl, 4-bromophenyl, 3-hydroxyphenyl, 2,6-dimethylphenyl, 2,6-dimethoxyphenyl, 3-nitrophenyl, 2,6-difluorophenyl, 3-chloro-2-methylphenyl, 2,3-dichlorophenyl, 4-hydroxyphenyl, 2,4,6-trimethylphenyl, 2,4,6-trimethoxyphenyl, 2,4,6-trifluorophenyl, preferably, $R_1$ is 4-fluorophenyl, 4-methylphenyl, 2,4,6-trimethylphenyl, 2,6-dimethylphenyl, 4-methoxyphenyl, 4-trifluoromethylphenyl, 3-hydroxyphenyl, more preferably, 2,6-dimethylphenyl.

In some embodiments of the present application, in the compound of Formula (I), or a tautomer, geometric isomer, enantiomer, diastereomer thereof, or a mixture thereof, or a pharmaceutically acceptable salt thereof provided by the present application, $R_2$ is $C_{1-8}$ alkyl or $C_{3-8}$ cycloalkyl; the $C_{1-8}$ alkyl or $C_{3-8}$ cycloalkyl is optionally substituted by one or more of the following groups: $C_{1-6}$ alkyl, $C_{1-6}$ alkoxy, $C_{1-6}$ haloalkyl, $C_{3-6}$ cycloalkyl, halogen, hydroxyl, cyano, and amino.

In some embodiments of the present application, in the compound of Formula (I), or a tautomer, geometric isomer, enantiomer, diastereomer thereof, or a mixture thereof, or a pharmaceutically acceptable salt thereof provided by the present application, $R_2$ is methyl, ethyl, n-propyl, isopropyl, n-butyl, sec-butyl, tert-butyl, n-pentyl, isopentyl, n-hexyl, cyclopropyl, cyclobutyl, cyclopropylmethylene, cyclobutylmethylene, cyclopentylmethylene, n-octyl, n-heptyl; preferably, ethyl, n-propyl, n-butyl, n-pentyl, n-hexyl, n-octyl, n-heptyl; more preferably, n-butyl.

In some embodiments of the present application, in the compound of Formula (I), or a tautomer, geometric isomer, enantiomer, diastereomer thereof, or a mixture thereof, or a pharmaceutically acceptable salt thereof provided by the present application, $R_3$ is $C_{1-8}$ alkyl, $C_{3-7}$ cycloalkyl, an aromatic hydrocarbon group, heteroaryl, three- to eight-membered heterocycloalkyl, or $R_3$ and $X_2$, which is $NR_4$, together form azacycloalkyl, here, the $C_{1-8}$ alkyl, $C_{3-7}$ cycloalkyl, an aromatic hydrocarbon group, heteroaryl, or three- to eight-membered heterocycloalkyl is optionally substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, $C_{2-4}$ alkynyl, optionally substituted phenyl; herein, the optionally substituted phenyl refers to unsubstituted phenyl, or phenyl substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, and $C_{2-4}$ alkynyl.

In some embodiments of the present application, in the compound of Formula (I), or a tautomer, geometric isomer, enantiomer, diastereomer thereof, or a mixture thereof, or a pharmaceutically acceptable salt thereof provided by the present application, $R_3$ is methyl, ethyl, propyl, cyclopropyl, cyclobutyl, cyclopentyl, cyclohexyl, methoxyethyl, methoxypropyl, phenyl, phenylethyl, benzyl, 4-fluorobenzyl, 2-methylphenyl, 2-methoxyphenyl, 2-fluorophenyl, 2-chlorophenyl, 2-bromophenyl, 2-hydroxyphenyl, 3-methylphenyl, 3-methoxyphenyl, 3-fluorophenyl, 3-chlorophenyl, 3-bromophenyl, 3-hydroxyphenyl, 4-methylphenyl, 4-methoxyphenyl, 4-fluorophenyl, 4-chlorophenyl, 4-bromophenyl, 4-hydroxyphenyl, 4-trifluoromethylphenyl, 2,4-dimethylphenyl, 2,4-dimethoxyphenyl, 2,4-difluorophenyl, 2,4-dichlorophenyl, 2,4-dihydroxyphenyl, 2,6-dimethylphenyl, 2,6-dimethoxyphenyl, 2,6-difluorophenyl, 2,6-dichlorophenyl, 2,6-dihydroxyphenyl, 2,4,6-trimethylphenyl, 2,4,6-trimethoxyphenyl, 2,4,6-trifluorophenyl, or $R_3$ and $X_2$, which is $NR_4$, together form piperidine or pyrrolidine, preferably, phenyl, 4-methylphenyl, 4-trifluoromethylphenyl, 2-hydroxyphenyl, ethyl, 2-methoxyethyl, 2-phenethyl, 2,6-dimethylphenyl, cyclopropyl, benzyl, 4-fluorobenzyl, or $R_3$ and $X_2$, which is $NR_4$, together form piperidine or pyrrolidine.

In some embodiments of the present application, in the compound of Formula (I), or a tautomer, geometric isomer, enantiomer, diastereomer thereof, or a mixture thereof, or a pharmaceutically acceptable salt thereof provided by the present application, m and n are each independently selected from 0, 1, and 2, and m and n may be the same or may be different.

In some embodiments of the present application, in the compound of Formula (I), or a tautomer, geometric isomer, enantiomer, diastereomer thereof, or a mixture thereof, or a pharmaceutically acceptable salt thereof provided by the present application, L is selected from $C_{1-6}$ alkylene, $C_{2-4}$ alkenylene, $C_{2-4}$ alkynylene, and $C_{3-6}$ cycloalkylene; herein, carbon atoms on the main chain of the $C_{1-6}$ alkylene and $C_{3-6}$ cycloalkylene are optionally substituted by 1-3 heteroatoms selected from O, S, N, wherein N may be substituted by $R_5$, $R_5$ is selected from hydrogen, deuterium, and $C_{1-4}$ alkyl; the $C_{1-6}$ alkylene, $C_{2-4}$ alkenylene, $C_{2-4}$ alkynylene, and $C_{3-6}$ cycloalkylene are optionally substituted by one or more of the following groups: $C_{1-4}$ alkyl, $C_{1-4}$ alkoxy, $C_{14}$ haloalkyl, $C_{14}$ haloalkoxy, $C_{3-6}$ cycloalkyl, halogen, hydroxyl, cyano, amino, an ester group, nitro, mono $C_{1-4}$ alkyl amino, di-$C_{1-4}$ alkyl amino, $C_{2-4}$ alkenyl, and $C_{2-4}$ alkynyl.

In some embodiments of the present application, in the compound of Formula (I), or a tautomer, geometric isomer, enantiomer, diastereomer thereof, or a mixture thereof, or a pharmaceutically acceptable salt thereof provided by the present application, L is selected from: $-CH_2-$, $-(CH_2)_2-$, $-(CH_2)_3-$, $-(CH_2)_4-$, $-(CH_2)_5-$, $-(CH_2)_6-$, -O-, $-CH_2OCH_2-$, $-OCH_2-$, $-CH_2O-$, $-OCH_2O-$, $-O(CH_2)_2O-$, $-O(CH_2)_3O-$, $-O(CH_2)_4O-$, $-CH_2OCH_2CH_2-$, $-CH_2CH_2OCH_2-$, $-OCH_2O-CH_2O-$, $-CH_2CH_2OCH_2CH_2-$, -S-, $-CH_2SCH_2-$, $-SCH_2-$, $-CH_2S-$, $-CH_2S-CH_2CH_2-$, $-CH_2CH_2SCH_2-$, $-CH_2CH_2SCH_2CH_2-$, -CH=CH-,

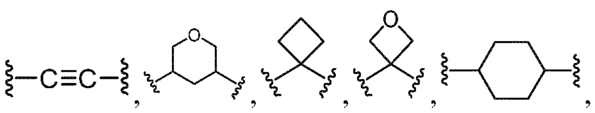
, , , , ,

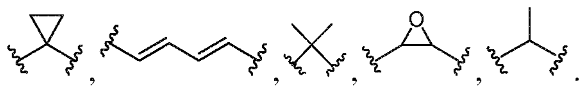
, , , , .

In some embodiments of the present application, in the compound of Formula (I), or a tautomer, geometric isomer, enantiomer, diastereomer thereof, or a mixture thereof, or a pharmaceutically acceptable salt thereof provided by the present application, $X_1$ and $X_2$ are each independently O, NH, $NCH_3$, $NCH_2CH_3$, $N(CH_2)_2CH_3$, or, when $X_2$ is $NR_4$, it forms piperidine together with $R_3$.

In some embodiments of the present application, in the compound of Formula (I), or a tautomer, geometric isomer, enantiomer, diastereomer thereof, or a mixture thereof, or a pharmaceutically acceptable salt thereof provided by the present application, when one of $S_1$ and $S_2$ is a single bond (i.e., atoms connected thereto are directly bond-connected), the other is $-(CH)_2)_3-$, $-(CH_2)_4-$,$-(CH_2)_5-$, $-(CH_2)_6-$, $-CH_2OCH_2CH_2-$, $-CH_2CH_2OCH_2-$, $-CH_2SCH_2CH_2-$.

In some embodiments of the present application, in the compound of Formula (I), or a tautomer, geometric isomer, enantiomer, diastereomer thereof, or a mixture thereof, or a pharmaceutically acceptable salt thereof provided by the present application, when one of $Q_1$ and $Q_2$ is a single bond (i.e., atoms connected thereto are directly bond-connected), the other is $-(CH)_2)_3-$, $-(CH_2)_4-$, $-(CH_2)_5-$, $-(CH_2)_6-$.

In some embodiments of the present application, in the compound of Formula (I), or a tautomer, geometric isomer, enantiomer, diastereomer thereof, or a mixture thereof, or a pharmaceutically acceptable salt thereof provided by the present application, $Y^-$ is halogen anion, sulfate, acetate, tartrate, p-toluenesulfonate, mesylate, citrate, preferably $Cl^-$, $Br^-$, $I^-$, $CH_3COO^-$; more preferably $Br^-$.

In some embodiments of the present application, in the compound of Formula (I), or a tautomer, geometric isomer, enantiomer, diastereomer thereof, or a mixture thereof, or a pharmaceutically acceptable salt thereof provided by the present application,

$R_1$ is selected from an aromatic hydrocarbon group; the aromatic hydrocarbon group is optionally substituted by one or more of the following groups: $C_{1-6}$ alkyl, $C_{1-6}$ alkoxy, cyano, halogen, hydroxyl, amino, nitro, an ester group, mono $C_{1-6}$ alkyl amino, di-$C_{1-6}$ alkyl amino, $C_{2-4}$ alkenyl, $C_{2-4}$ alkynyl, $C_{1-4}$haloalkyl, and $C_{1-6}$haloalkoxy;

$R_2$ is selected from $C_{1-8}$ alkyl and $C_{3-8}$ cycloalkyl, herein, the $C_{1-8}$ alkyl and $C_{3-8}$ cycloalkyl are optionally substituted by one or more of the following groups: $C_{1-6}$ alkyl, $C_{1-6}$ alkoxy, $C_{1-6}$ haloalkyl, $C_{3-6}$ cycloalkyl, halogen, hydroxyl,

cyano, and amino;

$R_3$ is selected from $C_{1-8}$ alkyl, $C_{3-7}$ cycloalkyl, an aromatic hydrocarbon group, heteroaryl, three- to eight-membered heterocycloalkyl, or $R_3$ and $X_2$, which is $NR_4$, together form azacycloalkyl, herein, the $C_{1-8}$ alkyl, $C_{3-7}$ cycloalkyl, an aromatic hydrocarbon group, heteroaryl, or three- to eight-membered heterocycloalkyl is optionally substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, $C_{2-4}$ alkynyl, optionally substituted phenyl; here, the optionally substituted phenyl is unsubstituted phenyl, or phenyl is substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino;

$X_1$ and $X_2$ are each independently selected from O, S, and $NR_4$, wherein $R_4$ is selected from hydrogen, deuterium, $C_{1-6}$ alkyl, $C_{1-6}$ alkoxy, $C_{1-6}$ alkyl, and $C_{3-6}$ cycloalkyl;

m and n are each independently selected from an integer of 0-4, and m and n may be the same or may be different;

L is selected from $C_{1-4}$ alkylene, $C_{2-4}$ alkenylene, $C_{2-4}$ alkynylene, and $C_{3-6}$ cycloalkylene, here, carbon atoms on the main chain of the $C_{1-4}$ alkylene and $C_{3-6}$ cycloalkylene are optionally substituted by 1-2 heteroatoms selected from O, S, N, wherein N may be substituted by $R_5$, $R_5$ is selected from hydrogen, deuterium, and $C_{1-4}$ alkyl; wherein the $C_{1-4}$ alkylene, $C_{2-4}$ alkenylene, $C_{2-4}$ alkynylene, and $C_{3-8}$ cycloalkylene are optionally substituted by one or more of the following groups: $C_{1-4}$ alkyl, $C_{1-4}$ alkoxy, $C_{1-4}$ haloalkyl, $C_{1-4}$ haloalkoxy, $C_{3-6}$ cycloalkyl, halogen, hydroxyl, cyano, amino, an ester group, nitro, mono $C_{1-4}$ alkyl amino, di-$C_{1-4}$ alkyl amino, $C_{2-4}$ alkenyl, and $C_{2-4}$ alkynyl;

$S_1$, $S_2$, $Q_1$, and $Q_2$ are each independently selected from a single bond (i.e., atoms connected thereto are directly bond-connected), and $C_{1-6}$ alkylene, here, carbon atoms on the main chain of the $C_{1-6}$ alkylene are optionally substituted by a heteroatom selected from O or S; wherein $S_1$ and $S_2$ cannot be single bonds at the same time (i.e., atoms connected thereto are directly bond-connected), $Q_1$ and $Q_2$ cannot be single bonds at the same time (i.e., atoms connected thereto are directly bond-connected); and

$Y^-$ is halogen anion, sulfate, acetate, tartrate, p-toluenesulfonate, mesylate, citrate.

In some embodiments of the present application, in the compound of Formula (I), or a tautomer, geometric isomer, enantiomer, diastereomer thereof, or a mixture thereof, or a pharmaceutically acceptable salt thereof provided by the present application,

$R_1$ is selected from phenyl, which may be optionally substituted by one or more of the following groups: methyl, ethyl, n-propyl, isopropyl, n-butyl, sec-butyl, tert-butyl, trifluoromethyl, methoxy, trifluoromethoxy, fluorine, chlorine, bromine, iodine, hydroxyl, amino, nitro, methyl ester, and ethyl ester;

$R_2$ is selected from methyl, ethyl, n-propyl, isopropyl, n-butyl, sec-butyl, tert-butyl, n-pentyl, isopentyl, n-hexyl, cyclopropyl, cyclobutyl, cyclopropylmethylene, cyclobutyl methylene, cyclopentylmethylene, n-octyl, n-heptyl;

$R_3$ is selected from $C_{1-8}$ alkyl, $C_{3-7}$ cycloalkyl, an aromatic hydrocarbon group, heteroaryl, three- to eight-membered heterocycloalkyl, or $R_3$ and $X_2$, which is $NR_4$, together form azacycloalkyl, herein, the $C_{1-8}$ alkyl, $C_{3-7}$ cycloalkyl, an aromatic hydrocarbon group, heteroaryl, or three- to eight-membered heterocycloalkyl is optionally substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, $C_{2-4}$ alkynyl, optionally substituted phenyl; herein, the optionally substituted phenyl refers to unsubstituted phenyl, or phenyl is substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, and $C_{2-4}$ alkynyl;

$X_1$ and $X_2$ are each independently selected from O, S, and $NR_4$; wherein $R_4$ is selected from hydrogen, deuterium, methyl, ethyl, n-propyl, isopropyl, n-butyl, sec-butyl, tert-butyl, n-amyl, isoamyl, n-hexyl, cyclopropyl, cyclobutyl, cyclopropylmethylene, and cyclobutyl methylene, or $NR_4$ and $R_1$ or $R_3$ connected thereto together form azacycloalkyl;

m and n are each independently selected from an integer of 0-2, and m and n may be the same or may be different;

L is selected from $C_{1-4}$ alkylene, $C_{2-4}$ alkenylene, $C_{2-4}$ alkynylene, and $C_{3-6}$ cycloalkylene, herein, carbon atoms on the main chain of the $C_{1-4}$ alkylene and $C_{3-6}$ cycloalkylene are optionally substituted by 1-2 heteroatoms selected from O, S, N, wherein N may be substituted by $R_5$, $R_5$ is selected from hydrogen, deuterium, and $C_{1-4}$ alkyl; wherein the $C_{1-4}$ alkylene, $C_{2-4}$ alkenylene, $C_{2-4}$ alkynylene, and $C_{3-8}$ cycloalkylene are optionally substituted by one or more of the following groups: $C_{1-4}$ alkyl, $C_{1-4}$ alkoxy, $C_{1-4}$ haloalkyl, $C_{1-4}$ haloalkoxy, $C_{3-6}$ cycloalkyl, halogen, hydroxyl, cyano, amino, an ester group, nitro, mono $C_{1-4}$ alkyl amino, di-$C_{1-4}$ alkyl amino, $C_{2-4}$ alkenyl, and $C_{2-4}$ alkynyl;

when one of $S_1$ and $S_2$ is a single bond (i.e., atoms connected thereto are directly bond-connected), the other is selected from $-(CH_2)_3-$, $-(CH_2)_4-$, $-(CH_2)_5-$, $-(CH_2)_6-$, $-CH_2OCH_2CH_2-$, $-CH_2CH_2OCH_2-$, $-CH_2SCH_2CH_2-$;

when one of $Q_1$ and $Q_2$ is a single bond (i.e., atoms connected thereto are directly bond-connected), the other is selected from $-(CH_2)_3-$, $-(CH_2)_4-$, $-(CH_2)_5-$, $-(CH_2)_6-$, $-CH_2OCH_2CH_2-$, $-CH_2CH_2OCH_2-$, $-CH_2SCH_2CH_2-$; and

Y is selected from halogen anion, sulfate, acetate, tartrate, p-toluenesulfonate, mesylate, citrate.

In some embodiments of the present application, in the compound of Formula (I), or a tautomer, geometric isomer, enantiomer, diastereomer thereof, or a mixture thereof, or a pharmaceutically acceptable salt thereof provided by the present application,

$R_1$ is selected from phenyl, 2-methylphenyl, 2-methoxyphenyl, 4-methylphenyl, 4-methoxyphenyl, 4-fluorophenyl, 2-chlorophenyl, 4-chlorophenyl, 2-bromophenyl, 3-bromophenyl, 4-bromophenyl, 3-hydroxyphenyl, 4-trifluoromethylphenyl, 2,6-dimethylphenyl, 2,6-dimethoxyphenyl, 3-nitrophenyl, 2,6-difluorophenyl, 3-chloro-2-methylphenyl, 2,3-dichlorophenyl, 4-hydroxyphenyl, 2,4,6-trimethylphenyl, 2,4,6-trimethoxyphenyl, and 2,4,6-trifluorophenyl;

$R_2$ is selected from methyl, ethyl, n-propyl, n-butyl, n-pentyl, n-hexyl, n-octyl and n-heptyl;

$R_3$ is selected from methyl, ethyl, propyl, cyclopropyl, cyclobutyl, cyclopentyl, cyclohexyl, methoxyethyl, methoxypropyl, phenyl, 2-methylphenyl, phenylethyl, benzyl, 4-fluorobenzyl, 2-methoxyphenyl, 2-fluorophenyl, 2-chlorophenyl, 2-bromophenyl, 2-hydroxyphenyl, 3-methylphenyl, 3-methoxyphenyl, 3-fluorophenyl, 3-chlorophenyl, 3-bromophenyl, 3-hydroxyphenyl, 4-methylphenyl, 4-methoxyphenyl, 4-fluorophenyl, 4-chlorophenyl, 4-chlorophenyl, 4-hydroxyphenyl, 4-trifluoromethylphenyl, 2,4-dimethylphenyl, 2,4-dimethoxyphenyl, 2,4-difluorophenyl, 2,4-dichlorophenyl, 2,4-dihydroxyphenyl, 2,6-dimethylphenyl, 2,6-dimethoxyphenyl, 2,6-difluorophenyl, 2,6-dichlorophenyl, 2,6-dihydroxyphenyl, 2,4,6-trimethylphenyl, 2,4,6-trimethoxyphenyl, 2,4,6-trifluorophenyl, or $R_3$ and $X_2$, which is $NR_4$, together form piperidine or pyrrolidine;

$X_1$ and $X_2$ are each independently selected from O, NH, $NCH_3$, $NCH_2CH_3$, N $(CH_2)_2CH_3$;

m and n are each independently selected from an integer of 0-2, and m and n may be the same or may be different;

L is selected from: $-CH_2-$, $-(CH_2)_2-$, $-(CH_2)_3-$, $-(CH_2)_4-$, $-(CH_2)_5-$, $-(CH_2)_6-$, -O-, $-CH_2OCH_2-$, $-OCH_2-$, $-CH_2O-$, $-OCH_2O-$, $-O(CH_2)_2O-$, $-O(CH_2)_3O-$, $-O(CH_2)_4O-$, $-CH_2OCH_2CH_2-$, $-CH_2CH_2OCH_2-$, $-OCH_2OCH_2O-$, $-CH_2CH_2OCH_2CH_2-$, -S-, $-CH_2SCH_2-$, $-SCH_2-$, $-CH_2S-$, $-CH_2SCH_2CH_2-$, $-CH_2CH_2SCH_2-$, $-CH_2CH_2SCH_2CH_2-$, -CH = CH-,

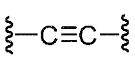, 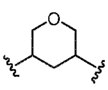, 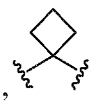, 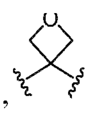, 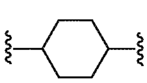, 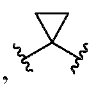, 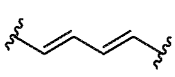, 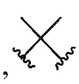, 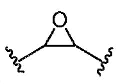, 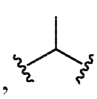;

when one of $S_1$ and $S_2$ is a single bond (i.e., atoms connected thereto are directly bond-connected), the other is $-(CH_2)_3-$, $-(CH_2)_4-$, $-(CH_2)_5-$, $-(CH_2)_6-$, $-CH_2OCH_2CH_2-$, $-CH_2CH_2OCH_2-$, or $-CH_2SCH_2CH_2-$;

when one of $Q_1$ and $Q_2$ is a single bond (i.e., atoms connected thereto are directly bond-connected), the other is $-(CH_2)_3-$, $-(CH_2)_4-$, $-(CH_2)_5-$, $-(CH_2)_6-$; and

$Y^-$ is halogen anion, sulfate, acetate, tartrate, p-toluenesulfonate, mesylate, citrate.

In some embodiments of the present application, in the compound of Formula (I), or a tautomer, geometric

isomer, enantiomer, diastereomer thereof, or a mixture thereof, or a pharmaceutically acceptable salt thereof provided by the present application, the compound of Formula (I) includes, but is not limited to, the following compounds:

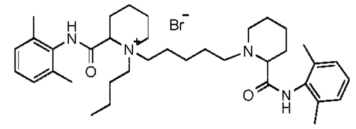
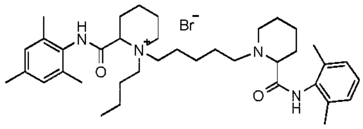
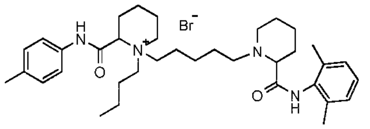

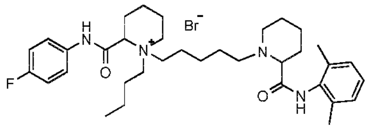
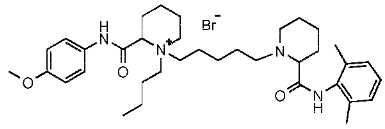
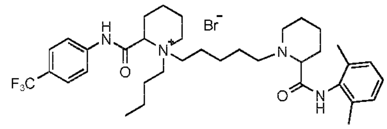

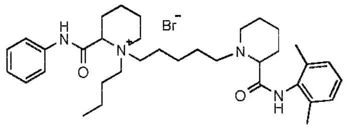
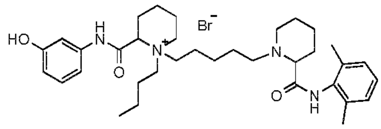
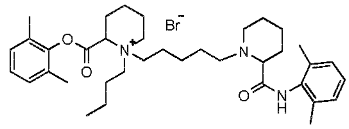

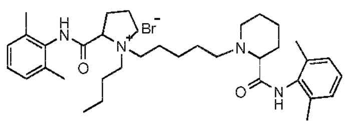
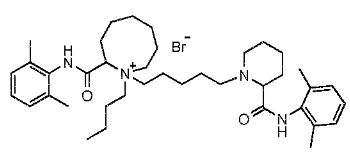
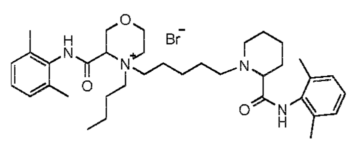

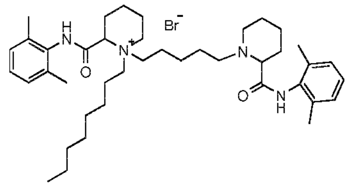
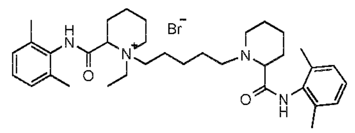
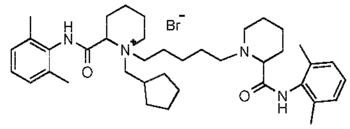

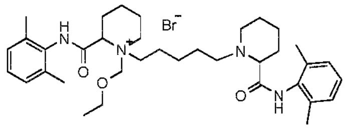
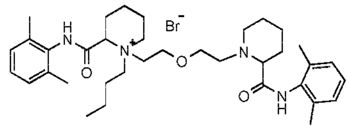
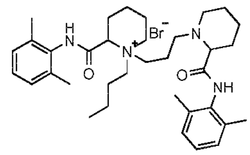

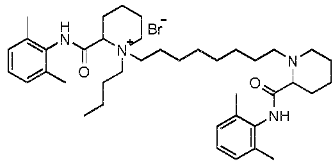
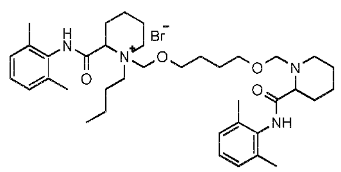
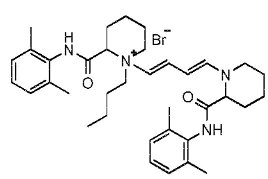

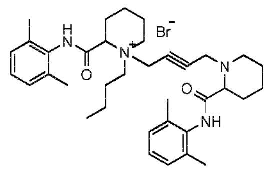
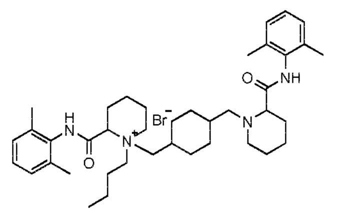
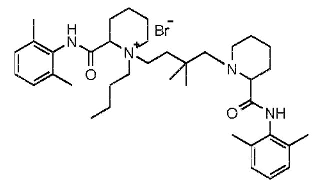

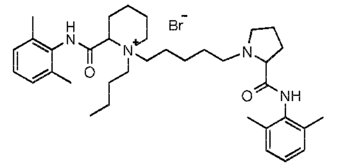
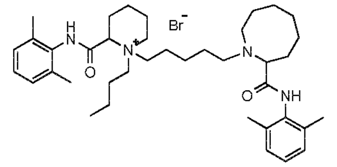
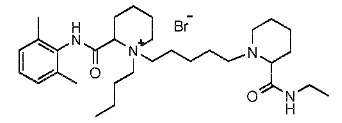

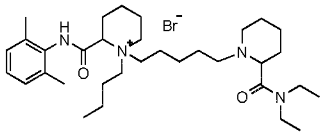
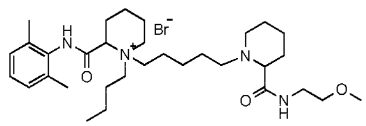
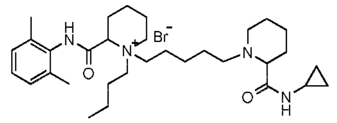

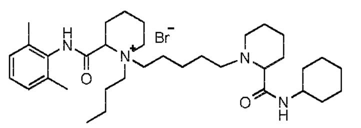
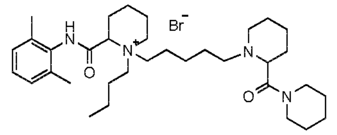
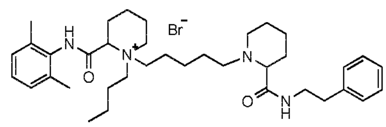

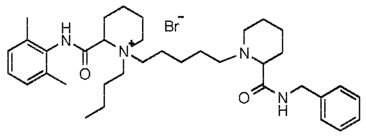
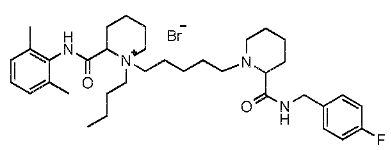
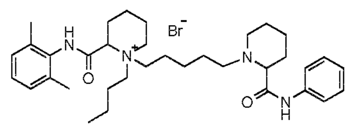

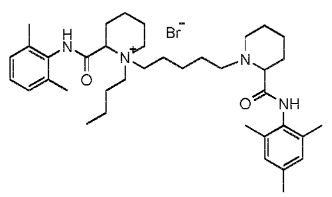
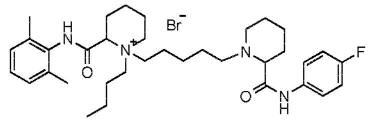
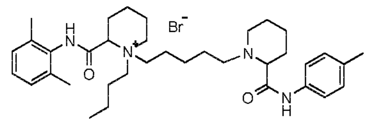

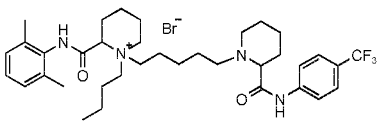
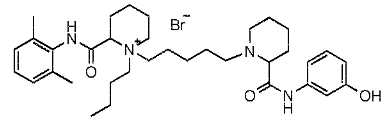
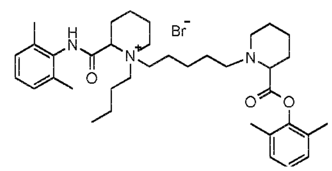

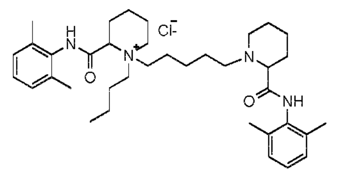
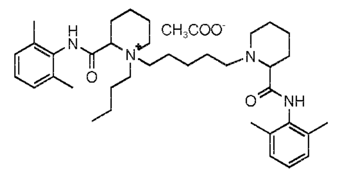

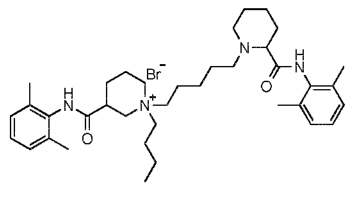

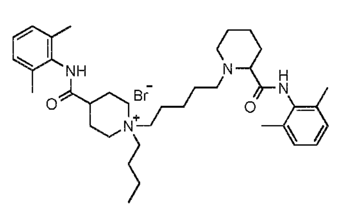

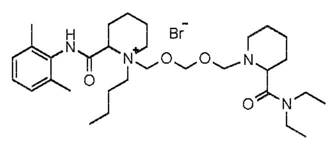

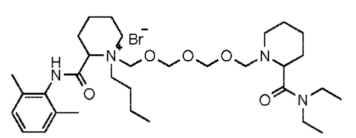

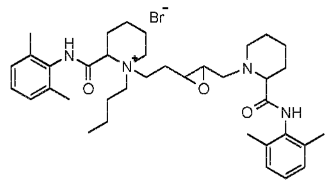

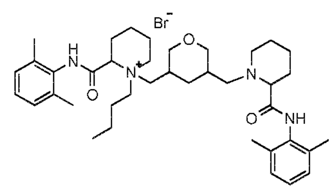

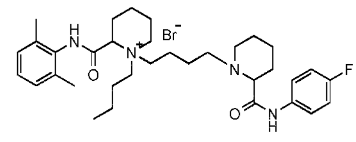

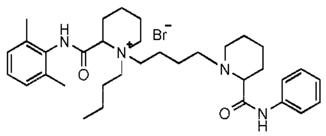

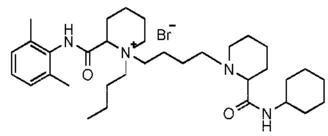

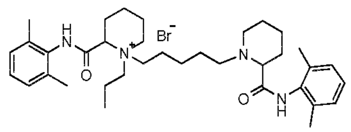

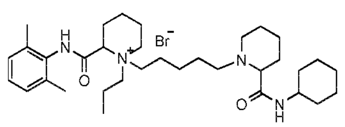

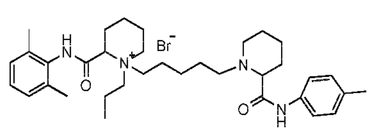

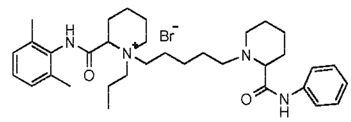

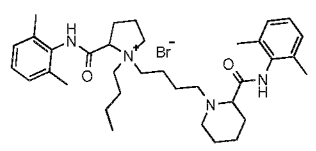

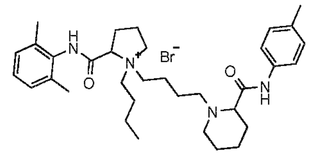

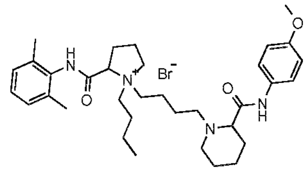

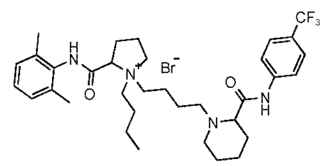

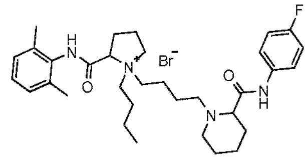

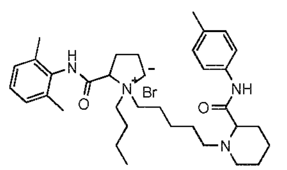

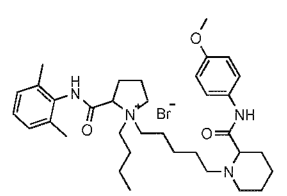

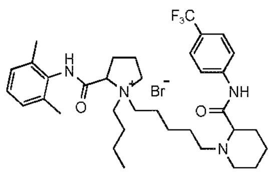
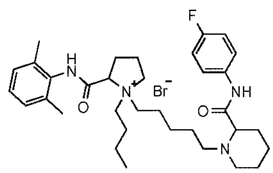
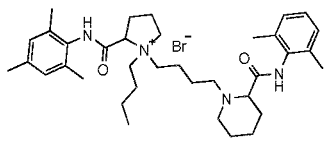
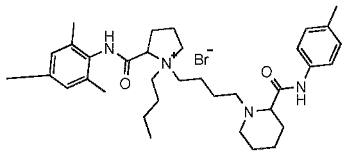
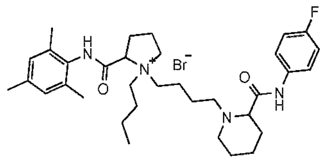
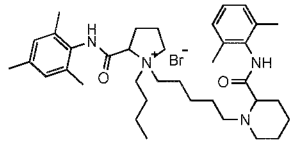
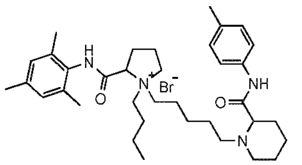
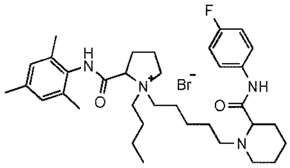

In some embodiments of the present application, the compound of General Formula (I), or a tautomer, geometric isomer, enantiomer, diastereomer thereof, or a mixture thereof, or a pharmaceutically acceptable salt thereof provided by the present application, the compound of General Formula (I) includes, but is not limited to, the following compounds:

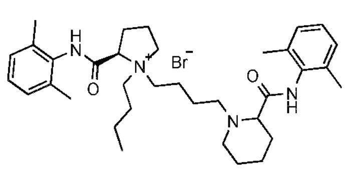
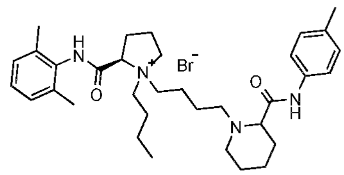
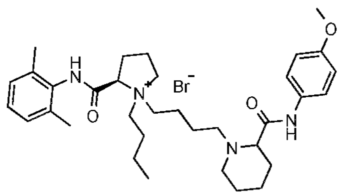
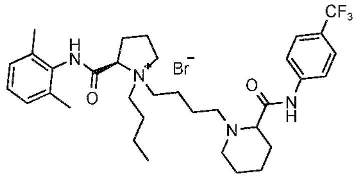
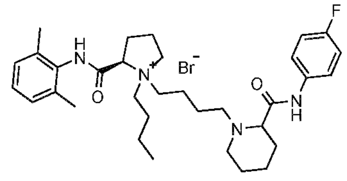
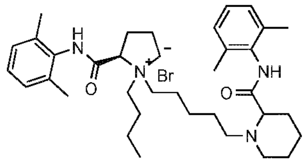
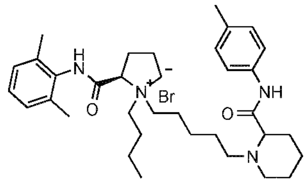
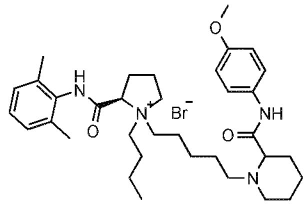
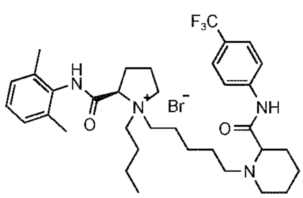
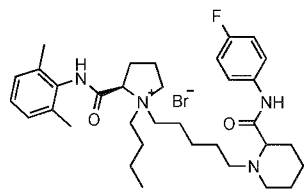
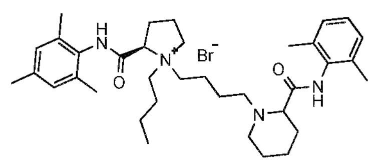

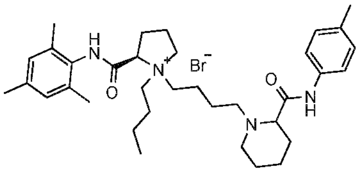

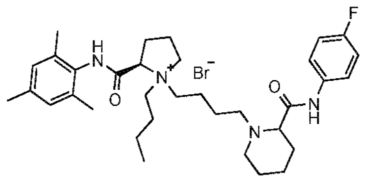

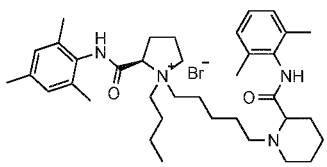

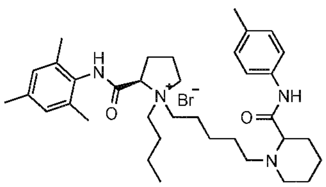

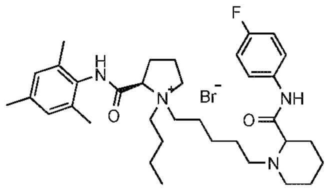

。

The second aspect of the present application provides a preparation method of the compound of General Formula (I) of the first aspect of the present application, a tautomer, geometric isomer, enantiomer, diastereomer thereof, or a mixture thereof, or a pharmaceutically acceptable salt thereof, the preparation method including the following steps:

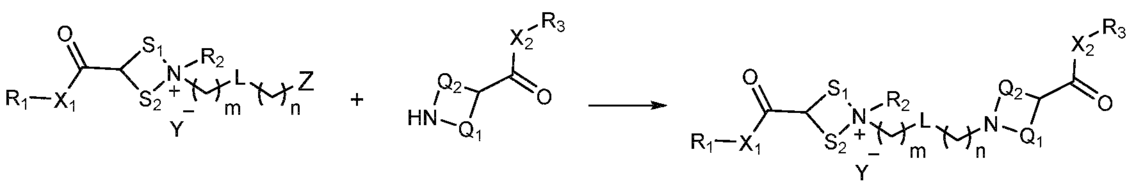

(II) (III) (I)

reacting a compound of Formula (II) with a compound of Formula (III) to obtain the compound of Formula (I);

herein, Z in Formula (II) is an electron-withdrawing leaving group, such as bromine, chlorine, or sulfonate, and definitions of groups in Formula (II) and Formula (III) are the same as those in Formula (I).

In the preparation method of the present application, the compound of Formula (II) may be prepared by the following method:

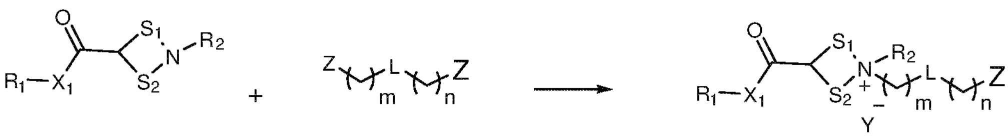

(II-1) (II-2) (II)

wherein definitions of the groups in Formula (II-1) and Formula (II-2) are the same as those set forth above.

The third aspect of the present application provides a pharmaceutical composition, including the compound of General Formula (I) of the first aspect of the present application, a tautomer, geometric isomer, enantiomer, diastereomer thereof, or a mixture thereof, or a pharmaceutically acceptable salt thereof, and a pharmaceutically acceptable carrier excipient or a diluent.

The fourth aspect of the present application provides the compound of General Formula (I) of the present application, a tautomer, geometric isomer, enantiomer, diastereomer thereof, or a mixture thereof, or a pharmaceutically acceptable salt thereof, for pharmaceutical use.

In some embodiments of the present application, the compound of General Formula (I), or a tautomer, geometric isomer, enantiomer, diastereomer thereof, or a mixture thereof, or a pharmaceutically acceptable salt thereof provided by the present application are for anesthetic or analgesic use.

In some embodiments of the present application, the compound of General Formula (I), or a tautomer, geometric isomer, enantiomer, diastereomer thereof, or a mixture thereof, or a pharmaceutically acceptable salt thereof provided by the present application are for anesthetic or analgesic use.

The fifth aspect of the present application provides use of the compound of General Formula (I) of the first aspect of the present application, a tautomer, geometric isomer, enantiomer, diastereomer thereof, or a mixture thereof, or a pharmaceutically acceptable salt thereof, or a pharmaceutical composition thereof in the preparation of anesthetic or analgesic drugs.

The use according to the fifth aspect of the present application, wherein the use is use in the preparation of drugs for local anesthesia or analgesia.

The use according to the fifth or fourth aspect of the present application, wherein the anesthesia is conduction anesthesia, topical anesthesia, or infiltration anesthesia; pain suitable for the analgesia is chronic pain, acute pain, inflammatory pain, cancer pain, neuropathic pain, musculoskeletal pain, primary pain, intestinal pain, or idiopathic pain.

The use according to the fifth or fourth aspect of the present application, wherein administration for anesthesia or analgesia is transdermal administration, subcutaneous administration, intradermal administration, intramuscular administration, near-nerve administration, dental pulp administration, intraspinal administration, epidural administration, intravenous administration, or topical, transmucosal administration such as eye drops.

The sixth aspect of the present application provides a method for anesthesia or analgesia, including administering the compound of General Formula (I) of the present application, a tautomer, geometric isomer, enantiomer, diastereomer thereof, or a mixture thereof, or a pharmaceutically acceptable salt thereof, or the pharmaceutical composition to a patient in need thereof.

The method according to the sixth aspect of the present application, wherein the anesthesia is local anesthesia, preferably conduction anesthesia, topical anesthesia, or infiltration anesthesia; pain suitable for analgesia is chronic pain, acute pain, inflammatory pain, cancer pain, neuropathic pain, musculoskeletal pain, primary pain, intestinal pain, or idiopathic pain.

The method according to the sixth aspect of the present application, wherein the administration is transdermal administration, subcutaneous administration, intradermal administration, intramuscular administration, near-nerve administration, dental pulp administration, intraspinal administration, epidural administration, intravenous administration, or topical, transmucosal administration such as eye drops.

The terms appearing in the specification and claims of the present application that are used to describe the present application are defined below. For a particular term, the meaning defined in the present application shall prevail if the meaning defined in the present application is inconsistent with that commonly understood by those skilled in the art; and if it is not defined in the present application, it shall have the meaning commonly understood by those skilled in the art.

In the present application, the name of the compound corresponds to its structural formula, and when the name of the compound is inconsistent with the structural formula, the structural formula shall prevail, or inference shall be made according to the specific situation of the present application combined with the knowledge of those skilled in the art.

The term "alkyl" as used in the present application refers to a linear or branched monovalent saturated hydrocarbon group.

The term "$C_{1-8}$ alkyl" means a linear or branched alkyl group having 1-8, i.e. 1, 2, 3, 4, 5, 6, 7, or 8 carbon atoms, typically methyl, ethyl, n-propyl, isopropyl, n-butyl, isobutyl, sec-butyl, tert-butyl, neopentyl, pentyl, or hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, etc. Similarly, the term "$C_{1-4}$ alkyl" means a linear or branched alkyl group having 1, 2, or 3 carbon atoms, i.e. methyl, ethyl, n-propyl, isopropyl, n-butyl, isobutyl, sec-butyl, or tert-butyl. Alkyl in the present application is preferably $C_{1-6}$ alkyl, more preferably $C_{1-4}$ alkyl.

The term "alkylene" used in the present application refers to a saturated, linear or branched aliphatic hydrocarbon group having two residues derived by removing two hydrogen atoms from the same carbon atom or two different carbon atoms of the parent alkane, which is a linear or branched group containing 1 to 16 carbon atoms, preferably alkylene containing 1 to 6 carbon atoms. Non-limiting examples of "alkylene" include, but are not limited, to $-CH_2-$, $-(CH_2)_2-$, $-(CH_2)_3-$, $-(CH_2)_4-$,

$-(CH_2)_5-$, $-(CH_2)_6-$, $-O-$, $-CH_2O\ CH_2-$, $-O\ CH_2-$, $-CH_2\ O-$,

$-OCH_2\ O-$, $-O\ (CH_2)_2\ O-$, $-O\ (CH_2)_3\ O-$, $-O\ (CH_2)_4\ O-$,

$-CH_2O\ CH_2\ CH_2-$, $-CH_2\ CH_2\ O\ CH_2-$, $-O\ CH_2O\ CH_2O-$,

$-CH_2\ CH_2\ O\ CH_2\ CH_2-$, $-S-$,$-CH_2S\ CH_2-$, $-S\ CH_2-$, $-CH_2S-$,

$-CH_2S\ CH_2\ CH_2-$, $-CH_2\ CH_2\ S\ CH_2-$, $-CH_2\ CH_2\ S\ CH_2\ CH_2-$.

The term "cycloalkylene" used in the present application refers to a group formed by formally eliminating two hydrogen atoms from saturated or partially unsaturated monocyclic or polycyclic cyclic hydrocarbon, which includes 3 to 8 carbon atoms. Non-limiting examples of "cycloalkylene" include, but are not limited to,

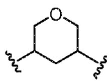, 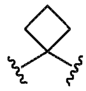, 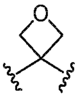, 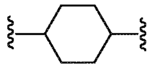, 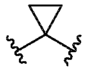

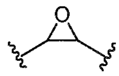.

The term "alkenylene" used in the present application refers to a group formed by formally eliminating two hydrogen atoms from an alkene, and non-limiting examples of "alkenylene" include, but are not limited to, -CH=CH-,

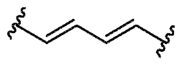.

The term "alkynylene" used in the present application refers to a group formed by formally eliminating two hydrogen atoms from an alkyne, and non-limiting examples of "alkenylene" include, but are not limited to,

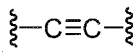.

The term "alkoxy" used in the present application refers to -O-(alkyl) and -O-(non-substituted cycloalkyl), wherein alkyl is defined as above. Non-limiting examples of "alkoxy" include, but are not limited to: methoxy, ethoxy, propoxy, butoxy, cyclopropoxy, cyclobutoxy, cyclopentyloxy, cyclohexyloxy.

The term "alkoxyalkyl" used in the present application refers to an alkyl group as defined above substituted by one or more alkoxy groups as defined above. Preferred alkoxyalkyl is alkoxy-$C_{1-3}$ alkyl. Non-limiting examples of "alkoxyalkyl" include, but are not limited to: methoxymethyl, methoxyethyl, ethoxyethyl, etc.

The term "$C_{2-4}$ alkenyl" used in the present application refers to an alkenyl group having 2-4 carbon atoms. The alkenyl group has 1, 2, or 3 carbon-carbon double bonds, and when it has one or more carbon-carbon double bonds, the carbon-carbon double bonds are conjugated or non-conjugated. Non-limiting examples of "$C_{2-4}$ alkenyl" include, but are not limited to vinyl, and ethylene dienyl.

The term "$C_{2-4}$ alkynyl" used in the present application refers to an alkynyl group having 2-6 carbon atoms. The alkynyl group has 1, 2, or 3 carbon-carbon triple bonds, and when it has one or more carbon-carbon triple bonds, the carbon-carbon triple bonds are conjugated or non-conjugated. Non-limiting examples of "$C_{2-4}$ alkynyl" include, but are not limited to, ethynyl.

The term "cycloalkyl" used in the present application refers to a saturated carbocyclic group having 3, 4, 5, 6, 7, 8, 9, or 10 carbon atoms. The cycloalkyl may be a monocyclic or polycyclic fused system, and may be fused on an aromatic ring. Non-limiting examples of "cycloalkyl" include, but are not limited to, cyclopropyl, cyclobutyl, cyclopentyl, cyclohexyl, etc.

The term "halogen" used in the present application refers to fluorine, chlorine, bromine, and iodine atoms.

The term "aromatic hydrocarbon group" used in the present application refers to a monocyclic or bicyclic aromatic system containing at least one unsaturated aromatic ring, preferably an aromatic group having 6-10, i.e. 6, 7, 8, 9, or 10 carbon atoms. Non-limiting examples of "aromatic hydrocarbon group" include, but are not limited to, phenyl, naphthyl, 1,2,3,4-tetrahydronaphthyl, indenyl, etc.

The term "heteroaryl" used in the present application refers to a monocyclic or bicyclic unsaturated aromatic ring system optionally substituted by at least one heteroatom independently selected from N, O, or S, preferably an aromatic heterocyclic group having 5-10, i.e. 5, 6, 7, 8, 9, or 10 atoms. Non-limiting examples of "heteroaryl" include, but are not limited to, thienyl, 2-pyridyl, 3-pyridyl, thiazolyl, isothiazolyl, furyl, pyrrolyl, triazolyl, imidazolyl, etc.

The term "heterocycloalkyl" used in the present application refers to a monocyclic or bicyclic saturated cyclic system optionally substituted by at least one and at most four heteroatoms independently selected from N, O, or S, preferably a heterocyclic group having 4-10, i.e. 4, 5, 6, 7, 8, 9, or 10 atoms, provided that the ring of the heterocyclic group does not contain two adjacent O or S atoms. Non-limiting examples of "heterocycloalkyl" include, but are not

limited to, pyrrolidyl, piperidyl, morpholinyl, or piperazinyl, etc.

The term "haloalkyl" used in the present application means that an alkyl group is substituted by one or more halogens, wherein the alkyl group is as defined above.

The term "haloalkoxy" used in the present application means that an alkoxy group is substituted by one or more halogens, wherein the alkoxy group is as defined above.

The term "hydroxyl" used in the present application refers to -OH.

The term "amino" used in the present application refers to $-NH_2$.

The term "cyano" used in the present application refers to -CN.

The term "nitro" used in the present application refers to $-NO_2$.

The term "ester group" used in the present application refers to -C(O)O (alkyl) or -C(O)O (cycloalkyl), wherein alkyl and cycloalkyl are as defined above.

"Optional" or "optionally" means that the subsequently described event or environment may, but not necessarily occur, and the description includes occasions where the event or environment occurs or does not occur. For example, "aryl optionally substituted by alkyl" means that alkyl may, but does not have to, exist, and the description includes cases where aryl is substituted by alkyl and cases where aryl is not substituted by alkyl.

The term "mixture form thereof' used in the present application refers to a mixture form of tautomers, a mixture form of geometric isomers (e.g. trans-cis or E-Z mixture form), a mixture form of enantiomers, or a mixture form of diastereomers, or a mixture form of at least two of tautomers, geometric isomers, enantiomers, and diastereomers.

The term "long-acting" used in the present application means that the single-drug anesthesia duration is greater than that of a standard concentration of levobupivacaine hydrochloride.

The present application provides a quaternary ammonium salt compound with a brand-new structural type. Experiments have proven that a single dose of the compound of the present application has a long-term local anesthetic effect, does not cause motor nerve injury, and has good safety, fast acting and high selectivity. The present application provides a kind of long-acting local anesthetics with a brand-new structure, to overcome the shortcomings of existing clinical local anesthetics, such as short duration, tissue and nerve toxicity, and inseparability of sensory and motor blocks, providing a new medical strategy for anesthesia and analgesia treatment.

Additional features and advantages of the present application will be set forth in the description which follows, and in part will become apparent from the description, or may be learned by practice of the present application. Other advantages of the present application may be realized and obtained by the solutions described in the specification.

**Examples**

To further clarify the purpose, technical solutions and advantages of the present application, examples of the present application will be described in detail below. The following examples are intended to illustrate the present application only and should not be considered as limiting the scope of the present application. Where specific conditions are not indicated in the examples, conventional conditions or conditions recommended by the manufacturer shall be followed. The reagents or instruments used without indication of the manufacturer are all conventional products that are commercially available.

TLC is thin layer chromatography;

HPLC is High Performance Liquid Chromatography;

MS: mass spectrum;

$^{1}H$ NMR: Proton Nuclear Magnetic Resonance Spectroscopy.

$^{13}C$ NMR: Carbon Nuclear Magnetic Resonance Spectroscopy;

TBTU: O-benzotriazole-N,N,N',N'-tetramethyluronium tetrafluoroborate;

NMM: N-methylmorpholine;

HATU: 2-(7-azabenzotriazole)-N,N,N',N'-tetramethyluronium hexafluorophosphate;

V/m: volume-mass ratio, indicating that the preceding equivalent (eq) is a ratio of volume to mass;

m/m: mass-mass ratio, indicating that the preceding equivalent (eq) is a ratio of mass to mass.

In this experiment, Waters 2545-2767-2489 HPLC was used, Epic Polar 5u 120A 25cm*30mm chromatographic column was used, detection wavelength was 210 nm, and acetonitrile/0.05% TFA/$H_2O$ (30%/70%) was used for gradient elution, with a flow rate of 15 ml/min;

For NMR, Bruker UltrashiedTM 400 MHz Plus nuclear magnetic resonance spectrometer was used for measurement (with TMS as internal standard, $CDCl_3$ or $CD_3OD$ as solvent); for LC-MS, waters Qda MS KAD3195 portable mass spectrometer was used.

**Example 1**

Synthesis of compound 1b

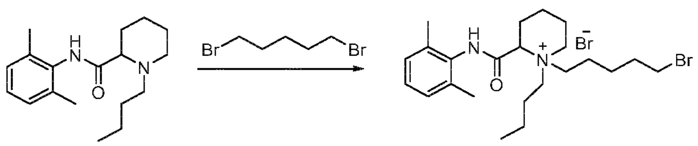

1a 1b

Bupivacaine 1a (17.36 mmol, 1.0 eq, 5 g) was weighed and added to 1,5-dibromopentane (2.0 eq of 1a , V/m, 10 mL), and the mixture was stirred and heated to 75°C, and detected by TCL until the reaction was complete. Silica gel column chromatography was performed, with the eluant: $CH_2Cl_2$:MeOH = 20:1, the eluate was collected and concentrated by rotary evaporation to obtain orange-yellow oily liquid compound 1b (4.5 g, with a yield of 59.1%, HPLC>90%). MS M/z (ESI) = 437.22 $[M]^+$, 439.22 $[M+2H]^+$.

Synthesis of compound 1

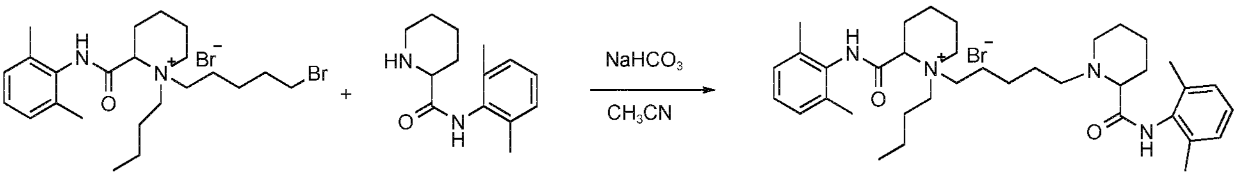


1b 1c 1

Compound 1b (1.14 mmol, 1.0 eq, 0.60 g) was weighed and added to analytically pure acetonitrile (10 eq of compound 1b, V/m, 5.0 mL) and stirred to dissolve, analytically pure sodium bicarbonate (1 eq of compound 1b, m/m, 0.60 g) was added, N-(2',6'-dimethylphenyl)-2-piperidinformamide 1c (1.34 mmol, 1.2 eq, 0.31 g) was added, the mixture was heated to 75°C for reaction, and HPLC detection was carried out until the reaction was completed. The reaction solution was filtered, the filter cake was washed with acetonitrile (30 eq of 1b, V/m, 15 mL, in three times), the filtrate was collected, and white powdery solid compound 1 (0.19 g, with a yield of 24.9%, HPLC>98%) was obtained by preparative separation. $^1H$ NMR (400MHz, $CDCl_3$)$\delta$ (ppm): 10.48 (s, 1H), 9.39 (s, 1H), 7.15-6.97 (m, 6H), 4.10 (s, 2H), 3.74-3.62 (m, 2H), 3.46-3.24 (m, 10H), 2.26-2.21 (m, 6H), 2.18 (s, 12H), 1.56-1.01 (m, 17H). MS m/z (ESI) = 589.37 $[M]^+$.

**Example 2**

Synthesis of compound 2c

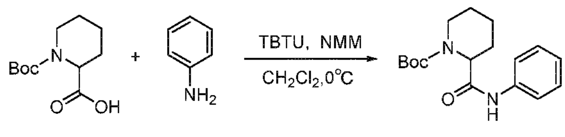


2a 2b 2c

N-Boc-2-piperidinecarboxylic acid 2a (4.37 mmol, 1.0 eq, 1.0 g) was added to analytically pure dichloromethane (25 eq of 2a, V/m, 25 mL), and stirred to dissolve, after the mixture was cooled to 0°C in an ice water bath, O-benzotriazole-N,N,N',N'-tetramethyluronium tetrafluoroborate (TBTU, 5.24 mmol, 1.2 eq, 1.68 g) was added. After stirring for about 30 minutes at 0°C, aniline (5.24 mmol, 1.2 eq, 0.48 mL) and N-methylmorpholine (NMM, 8.74 mmol, 2.0 eq, 0.96 mL) were added, and after stirring for about 1 hour at 0°C, the mixture was stirred at room temperature and detected by HPLC until the reaction was completed. The reaction solution was distilled under reduced pressure to obtain a yellow oily liquid. The organic phase was dissolved with a mixed solvent of dichloromethane:methanol = 20:1 (70 eq of 2a, V/m, mL/g, 70 mL), and washed with 5% $NaHSO_4$ aqueous solution (70 eq of N-Boc-2-piperidinecarboxylic acid, V/m, 70 mL, in three times). The organic phase was collected and distilled under reduced pressure to obtain a yellow oily viscous liquid. Column chromatography purification was performed, with the eluant: $CH_2Cl_2$:$CH_3OH$ = 40:1, the eluate was collected and distilled under reduced pressure to obtain pale yellow solid compound 2c (1.21 g, with a yield of 91.0%, HPLC>95%). MS m/z (ESI) = 305.18 $[M + H]^+$.

Synthesis of compound 2d

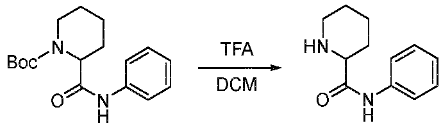


2c 2d

Compound 2c (3.28 mmol, 1 eq, 1.00 g) was weighed and added to analytically pure dichloromethane (5 eq of 2c, V/m, 5 mL) and stirred to dissolve. Trifluoroacetic acid (7.0 eq of compound 2c, V/m, 7 mL) was dissolved in analytically pure dichloromethane (10 eq of 2c, V/m, 10 mL) and was added dropwise, and the mixture was stirred for about 2 hours and detected by HPLC until the reaction was completed. The reaction solution was distilled under reduced pressure by adding analytically pure dichloromethane (10 eq of compound 2c, V/m, 10 mL, in three times) and analytically pure methanol (10 eq of compound 2c, V/m, 10 mL, in three times) to obtain white solid compound 2d (0.62 g, with a yield of 92.5%, HPLC>90%). MS m/z (ESI) = 205.13 $[M + H]^+$.

Synthesis of compound 2

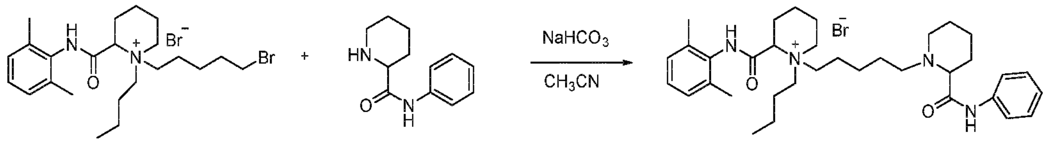


1b 2d 2

Compound 1b (1.14 mmol, 1 eq, 0.60 g) was weighed and was added to analytically pure acetonitrile (10 eq of compound 1b, V/m, 5.0 mL) and stirred to dissolve, analytically pure sodium bicarbonate (1 eq of compound 1b, m/m, 0.50 g) was added, compound 2d (1.34 mmol, 1.2 eq, 0.27 g) was added, the mixture was heated to 75°C for reaction, and HPLC detection was carried out until the reaction was completed. The reaction solution was filtered, the filter cake was washed with acetonitrile (30 eq of compound 1b, V/m, 15 mL, in three times), the filtrate was collected, and white powdery solid compound 2 (0.13 g, with a yield of 17.8%, HPLC>98%) was obtained by preparative separation. $^1H$ NMR (400MHz, $CDCl_3$)$\delta$ (ppm): 10.58 (s, 1H), 9.21 (s, 1H), 7.32-7.11 (m, 8H), 4.20 (s, 2H), 3.70-3.55 (m, 2H), 3.48-3.00 (m, 10H), 2.25-2.09 (m, 6H), 1.81 (s, 6H), 1.68-1.11 (m, 17H). MS m/z (ESI) = 561.40 $[M]^+$.

**Example 3**

Synthesis of compound 3b

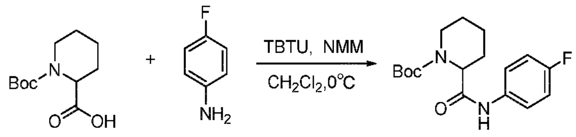


2a 3a 3b

N-Boc-2-piperidinecarboxylic acid (4.37 mmol, 1.0 eq, 1 g) was added to analytically pure dichloromethane (25 eq of N-Boc-2-piperidinecarboxylic acid, V/m, 25 mL), and stirred to dissolve, after the mixture was cooled to 0°C in an ice water bath, O-benzotriazole-N,N,N',N'-tetramethyluronium tetrafluoroborate (TBTU, 5.24 mmol, 1.2 eq, 1.68 g) was added. After stirring at 0°C for about 30 min, 4-fluoroaniline (5.24 mmol, 1.2 eq, 0.50 mL) and N-methylmorpholine (NMM, 8.74 mmol, 2 eq, 0.96 mL) were added. After stirring at 0°C for about 1h, the mixture was stirred at room temperature and detected by HPLC until the reaction was completed. The reaction solution was distilled under reduced pressure to obtain a yellow oily liquid. The organic phase was dissolved with a mixed solvent of dichloromethane:methanol = 20:1 (70 eq of N-Boc-2-piperidinecarboxylic acid, V/m, mL/g, 70 mL), and washed with 5% $NaHSO_4$ aqueous solution (70 eq of N-Boc-2-piperidinecarboxylic acid, V/m, 70 mL, in three times). The organic phase was collected and distilled under reduced pressure to obtain a yellow oily viscous liquid. Column chromatography purification was performed, with the eluant: $CH_2Cl_2$:$CH_3OH$ = 40:1, the eluate was collected and distilled under reduced pressure to obtain a pale yellow solid (1.33 g, with a yield of 94.3%, HPLC>95%). MS m/z=323.17 $[M + H]^+$.

Synthesis of compound 3c

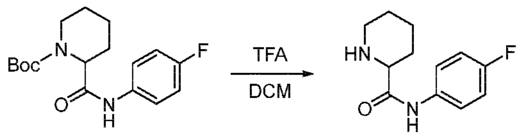


3b 3c

Compound 3b (3.10 mmol, 1 eq, 1.00 g) was weighed and added to analytically pure dichloromethane (5 eq of compound 3b, V/m, 5 mL) and stirred to dissolve. Trifluoroacetic acid (7.0 eq of compound 3b, V/m, 7 mL) was dissolved in analytically pure dichloromethane (10 eq of compound 3b, V/m, 10 mL) dropwise, and the mixture was stirred for about 2 h and detected by HPLC until the reaction was completed. The reaction solution was distilled under reduced pressure by adding analytically pure dichloromethane (10 eq of compound 3b, V/m, 10 mL, in three times) and analytically pure methanol (10 eq of compound 3b, V/m, 10 mL, in three times) to obtain a white solid (0.64 g, with a yield of 92.75%, HPLC>90%). MS m/z=223.12 $[M + H]^+$.

Synthesis of compound 3

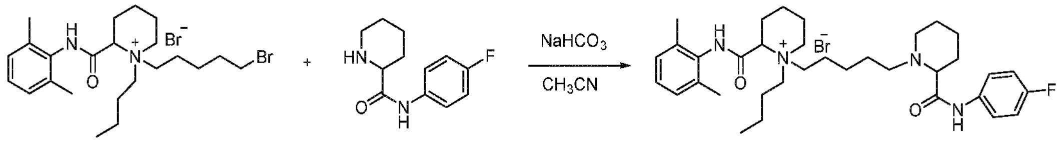


1b 3c 3

Compound 1b (1.14 mmol, 1 eq, 0.60 g) was weighed and added toanalytically pure acetonitrile (10 eq of compound 1b, V/m, 5.0 mL) and stirred to dissolve, analytically pure sodium bicarbonate (1 eq of compound 1b, m/m,

0.60 g) was added, 3c (1.34 mmol, 1.2 eq, 0.30 g) was added, the mixture was heated to 75°C for reaction, and HPLC detection was carried out until the reaction was completed. The reaction solution was filtered, the filter cake was washed with acetonitrile (30 eq of 1b, V/m, 15 mL, in three times), the filtrate was collected, and white powdery solid compound 3 (0.15 g, with a yield of 19.9%, HPLC>98%) was obtained by preparative separation. $^1$H NMR (400MHz, $CDCl_3$)$\delta$ (ppm): 10.53 (s, 1H), 9.22 (s, 1H), 7.90-7.31 (m, 7H), 4.40-4.31 (m, 2H), 3.72-3.50 (m, 2H), 3.48-3.20 (m, 10H), 2.15-2.09 (m, 6H), 1.90 (s, 6H), 1.44-1.11 (m, 17H). MS m/z (ESI) = 579.41 $[M]^+$.

**Example 4**

Synthesis of compound 4b

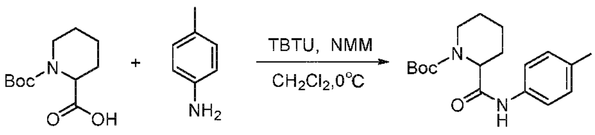


2a 4a 4b

N-Boc-2-piperidinecarboxylic acid 2a (4.37 mmol, 1.0 eq, 1 g) was weighed and added to analytically pure dichloromethane (25 eq of N-Boc-2-piperidinecarboxylic acid, V/m, 25 mL) , and stirred to dissolve, after the mixture was cooled to 0°C in an ice water bath, O-benzotriazole-N,N,N',N'-tetramethyluronium tetrafluoroborate (TBTU, 5.24 mmol, 1.2 eq, 1.68 g) was added. After stirring for about 30 minutes at 0°C, 4-methylaniline (5.24 mmol, 1.2 eq, 0.58 mL) and N-methylmorpholine (NMM, 8.74 mmol, 2 eq, 0.96 mL) were added, and after stirring for about 1 hour at 0°C, the mixture was stirred at room temperature and detected by HPLC until the reaction was completed. The reaction solution was distilled under reduced pressure to obtain a yellow oily liquid. The organic phase was dissolved with a mixed solvent of dichloromethane:methanol = 20:1 (70 eq of N-Boc-2-piperidinecarboxylic acid, V/m, mL/g, 70 mL), and washed with 5% NaHSO4 aqueous solution (70 eq of N-Boc-2-piperidinecarboxylic acid, V/m, 70 mL, in three times). The organic phase was collected and distilled under reduced pressure to obtain a yellow oily viscous liquid. Column chromatography purification was performed, with the eluant: $CH_2Cl_2$:$CH_3OH$ = 40:1, the eluate was collected and distilled under reduced pressure to obtain pale yellow solid compound 4b (1.25 g, with a yield of 91.9%, HPLC>95%). MS m/z (ESI) = 319.19 $[M + H]^+$.

Synthesis of compound 4c

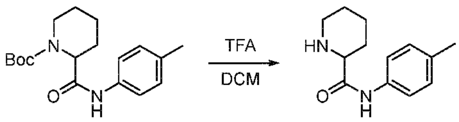


4b 4c

Compound 4b (3.14 mmol, 1 eq, 1.00 g) was weighed and added to analytically pure dichloromethane (5 eq of compound 4b, V/m, 5 mL) and stirred to dissolve. Trifluoroacetic acid (7.0 eq of compound 4b, V/m, 7 mL) was dissolved in analytically pure dichloromethane (10 eq of 4b, V/m, 10 mL) dropwise, and the mixture was stirred for about 2 hours and detected by HPLC until the reaction was completed. The reaction solution was distilled under reduced pressure by adding analytically pure dichloromethane (10 eq of compound 4b, V/m, 10 mL, in three times) and analytically pure methanol (10 eq of 4b, V/m, 10 mL, in three times) to obtain white solid compound 4c (0.63 g, with a yield of 92.6%, HPLC>90%). MS m/z (ESI) = 219.14 $[M + H]^+$.

Synthesis of compound 4

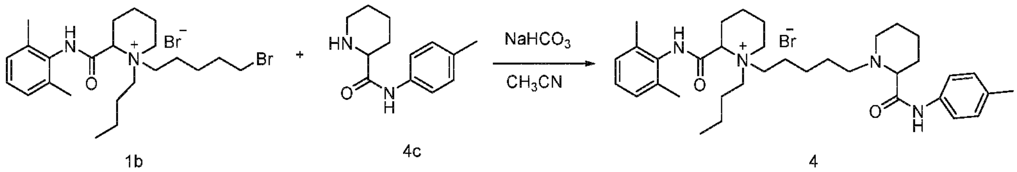


1b 4c 4

Compound 1b (1.14 mmol, 1 eq, 0.60 g) was weighed and added to analytically pure acetonitrile (10 eq of compound 1b, V/m, 5.0 mL) and stirred to dissolve, analytically pure sodium bicarbonate (1 eq of compound 1b, m/m, 0.50 g) was added, 4c (1.34 mmol, 1.2 eq, 0.29 g) was added, the mixture was heated to 75°C for reaction, and HPLC detection was carried out until the reaction was completed. The reaction solution was filtered, the filter cake was washed with acetonitrile (30 eq of 1b, V/m, 15 mL, in three times), the filtrate was collected, and white powdery solid compound 4 (0.15 g, with a yield of 20.0%, HPLC>98%) was obtained by preparative separation. $^1$H NMR (400MHz, $CDCl_3$)$\delta$ (ppm): 10.57 (s, 1H), 9.28 (s, 1H), 7.54-7.29 (m, 7H), 4.38 (s, 2H), 3.88-3.71 (m, 2H), 3.44-3.19 (m, 10H), 2.27-2.14 (m, 6H), 2.01 (s, 9H), 1.79-1.24 (m, 17H). MS m/z (ESI) = 575.35 $[M]^+$.

**Example 5**

Synthesis of compound 5b

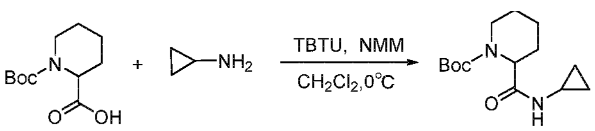


2a 5a 5b

N-Boc-2-piperidinecarboxylic acid (4.37 mmol, 1.0 eq, 1 g) was weighed and added to analytically pure dichloromethane (25 eq of N-Boc-2-piperidinecarboxylic acid, V/m, 25 mL), and stirred to dissolve, after the mixture was cooled to 0°C in an ice water bath, O-benzotriazole-N,N,N',N'-tetramethyluronium tetrafluoroborate (TBTU, 5.24 mmol, 1.2 eq, 1.68 g) was added. After stirring for about 30 minutes at 0°C, cyclopropylamine (5.24 mmol, 1.2 eq, 0.36 mL) and N-methylmorpholine (NMM, 8.74 mmol, 2 eq, 0.96 mL) were added, and after stirring for about 1 hour at 0°C, the mixture was stirred at room temperature and detected by HPLC until the reaction was completed. The reaction solution was distilled under reduced pressure to obtain a yellow oily liquid. The organic phase was dissolved with a mixed solvent of dichloromethane:methanol=20:1 (70 eq of N-Boc-2-piperidinecarboxylic acid, V/m, mL/g, 70 mL) and washed with 5% $NaHSO_4$ aqueous solution (70 eq of N-Boc-2-piperidinecarboxylic acid, V/m, 70 mL, in three times). The organic phase was collected and distilled under reduced pressure to obtain a yellow oily viscous liquid. Column chromatography purification was performed, with the eluant: $CH_2Cl_2$:$CH_3OH$ = 40:1, the eluate was collected and distilled under reduced pressure to obtain pale yellow solid compound 5b (1.03 g, with a yield of 88.0%, HPLC>95%). MS m/z (ESI) = 269.18 $[M + H]^+$.

Synthesis of 5c

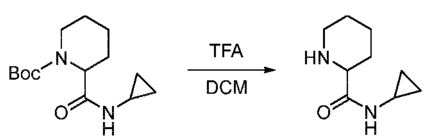


5b 5c

Compound 5b (3.72 mmol, 1 eq, 1.00 g) was weighed and added to analytically pure dichloromethane (5 eq of compound 5b, V/m, 5 mL) was added and stirred to dissolve. Trifluoroacetic acid (7.0 eq of compound 5b, V/m, 7 mL) was dissolved in analytically pure dichloromethane (10 eq of compound 5b, V/m, 10 mL) dropwise, and the mixture was

stirred for about 2 hours and detected by HPLC until the reaction was completed. The reaction solution was distilled under reduced pressure by adding analytically pure dichloromethane (10 eq of compound 5b, V/m, 10 mL, in three times) and analytically pure methanol (10 eq of compound 5b, V/m, 10 mL, in three times) to obtain white solid compound 5c (0.59 g, with a yield of 93.7%, HPLC>90%). MS m/z (ESI) = 169.13 [M + H]$^+$.

Synthesis of compound 5

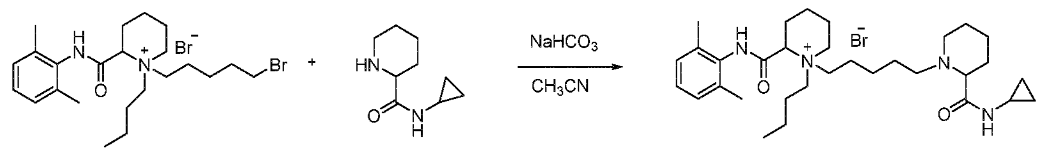


1b 5c 5

Compound 1b (1.14 mmol, 1 eq, 0.60 g) was weighed and added to analytically pure acetonitrile (10 eq of compound 1b, V/m, 5.0 mL) and stirred to dissolve, analytically pure sodium bicarbonate (1 eq of compound 1b, m/m, 0.60 g) was added, 5c (1.34 mmol, 1.2 eq, 0.22 g) was added, the mixture was heated to 75°C for reaction, and HPLC detection was carried out until the reaction was completed. The reaction solution was filtered, the filter cake was washed with acetonitrile (30 eq of compound 1b, V/m, 15 mL, in three times), the filtrate was collected, and white powdery solid compound 5 (0.18 g, with a yield of 26.1%, HPLC>98%) was obtained by preparative separation. $^1$H NMR (400MHz, $CDCl_3$) $\delta$ (ppm): 10.58 (s, 1H), 8.95 (s, 1H), 7.14-7.07 (m, 3H), 4.28 (s, 1H), 4.04-3.83 (m, 4H), 3.44-3.19 (m, 10H), 2.27-2.14 (m, 6H), 2.22 (s, 6H), 1.10-0.60 (m, 21H). MS m/z (ESI) = 525.40 [M]$^+$

**Example 6**

Synthesis of compound 6b

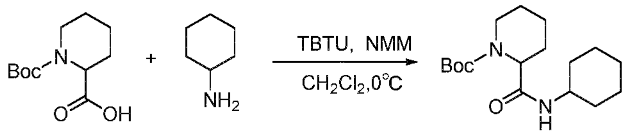


2a 6a 6b

N-Boc-2-piperidinecarboxylic acid (4.37 mmol, 1.0 eq, 1 g) was added to analytically pure dichloromethane (25 eq of N-Boc-2-piperidinecarboxylic acid, V/m, 25 mL) , and stirred to dissolve, after the mixture was cooled to 0°C in an ice water bath, O-benzotriazole-N,N,N',N'-tetramethyluronium tetrafluoroborate (TBTU, 5.24 mmol, 1.2 eq, 1.68 g) was added. After stirring for about 30 minutes at 0°C, cyclohexylamine (5.24 mmol, 1.2 eq, 0.60 mL) and N-methylmorpholine (NMM, 8.74 mmol, 2 eq, 0.96 mL) were added, and after stirring for about 1 hour at 0°C, the mixture was stirred at room temperature and detected by HPLC until the reaction was completed. The reaction solution was distilled under reduced pressure to obtain a yellow oily liquid. The organic phase was dissolved with a mixed solvent of dichloromethane:methanol=20:1 (70 eq of N-Boc-2-piperidinecarboxylic acid, V/m, mL/g, 70 mL) and washed with 5% NaHSO4 aqueous solution (70 eq of N-Boc-2-piperidinecarboxylic acid, V/m, 70 mL, in three times). The organic phase was collected and distilled under reduced pressure to obtain a yellow oily viscous liquid. Column chromatography purification was performed, with the eluant: $CH_2Cl_2$:$CH_3OH$ = 40:1. The eluate was collected and distilled under reduced pressure to obtain pale yellow solid compound 6b (1.27 g, with a yield of 93.4%, HPLC>95%). MS m/z (ESI) = 311.23 [M + H]$^+$.

Synthesis of compound 6c

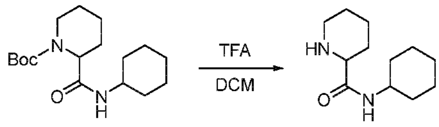


6b 6c

Compound 6b (3.22 mmol, 1 eq, 1.00 g) was weighed and added to analytically pure dichloromethane (5 eq of 6b, V/m, 5 mL) and stirred to dissolve. Trifluoroacetic acid (7.0 eq of compound 6b, V/m, 7 mL) was dissolved in analytically pure dichloromethane (10 eq of 6b, V/m, 10 mL) dropwise, and the mixture was stirred for about 2 hours and detected by HPLC until the reaction was completed. The reaction solution was distilled under reduced pressure by adding analytically pure dichloromethane (10 eq of compound 6b, V/m, 10 mL, in three times) and analytically pure methanol (10 eq of compound 6b, V/m, 10 mL, in three times) to obtain white solid compound 6c (0.61 g, with a yield of 91.0%, HPLC>90%). MS m/z (ESI) = 211.17 $[M + H]^+$.

Synthesis of compound 6

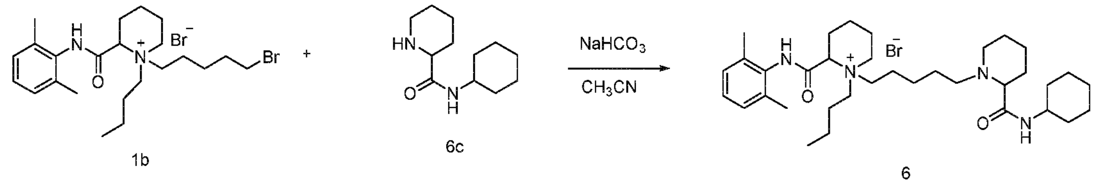


1b 6c 6

Compound 1b (1.14 mmol, 1 eq, 0.60 g) was weighed and added to analytically pure acetonitrile (10 eq of compound 1b, V/m, 5.0 mL) and stirred to dissolve, analytically pure sodium bicarbonate (1 eq of compound 1b, m/m, 0.60 g) was added, compound 6c (1.34 mmol, 1.2 eq, 0.28 g) was added, the mixture was heated to 75°C for reaction, and HPLC detection was carried out until the reaction was completed. The reaction solution was filtered, the filter cake was washed with acetonitrile (30 eq of 1b, V/m, 15 mL, in three times), the filtrate was collected, and white powdery solid compound 6 was obtained by preparative separation (0.20 g, with a yield of 27.1%, HPLC>98%). $^1H$ NMR (400MHz, $CDCl_3$) $\delta$ (ppm): 10.59 (s, 1H), 8.95 (s, 1H), 7.14-7.07 (m, 3H), 5.14 (s, 1H), 4.28 (s, 2H), 4.04-3.11 (m, 20H), 2.27-2.14 (m, 8H), 2.22 (s, 6H), 2.05-1.39 (m, 17H). MS m/z (ESI) = 567.38 $[M]^+$.

## Example 7

Synthesis of compound 7a

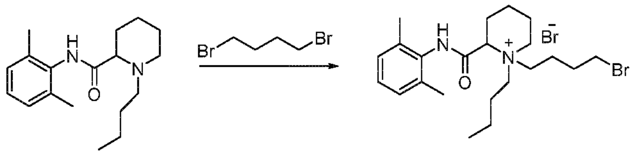

1a 7a

Bupivacaine (17.36 mmol, 1.0 eq, 5 g) was weighed and added to 1,4-dibromobutane (2.0 eq of bupivacaine, V/m, 10 mL), and the mixture was stirred and heated to 75°C, and detected by TCL until the reaction was complete. Silica gel column chromatography was performed, with the eluant: $CH_2Cl_2$:MeOH = 20:1, the eluate was collected and concentrated by rotary evaporation to obtain orange-yellow oily liquid compound 7a (4.3 g, with a yield of 49.1%, HPLC>95%). MS m/z (ESI) = 423.20 $[M]^+$, 425.20 $[M+2H]^+$.

Synthesis of compound 7

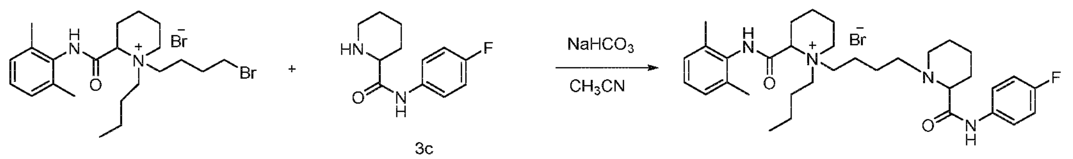


7a 3c 7

Compound 7a (1.18 mmol, 1 eq, 0.60 g) was weighed and added to analytically pure acetonitrile (10 eq of 7a, V/m, 5.0 mL) and stirred to dissolve, analytically pure sodium bicarbonate (1 eq of 7a, m/m, 0.60 g) was added, compound 3c (1.41 mmol, 1.2 eq, 0.32 g) was added, the mixture was heated to 75°C for reaction, and HPLC detection was carried out until the reaction was completed. The reaction solution was filtered, the filter cake was washed with acetonitrile (30 eq of 7a, V/m, 15 mL, in three times), the filtrate was collected, and white powdery solid compound 7 (0.13 g, with a yield of 17.1%, HPLC>98%) was obtained by preparative separation. $^1$H NMR (400MHz, $CDCl_3$) $\delta$ (ppm): 10.59 (s, 1H), 8.95 (s, 1H), 7.90-7.31 (m, 7H), 4.40-4.31 (m, 2H), 3.72-3.50 (m, 2H), 3.35-3.20 (m, 8H), 2.15-2.10 (m, 6H), 1.90 (s, 6H), 1.44-1.11 (m, 17H). MS m/z (ESI)= 565.31[M]$^+$.

**Example 8**

Synthesis of compound 8

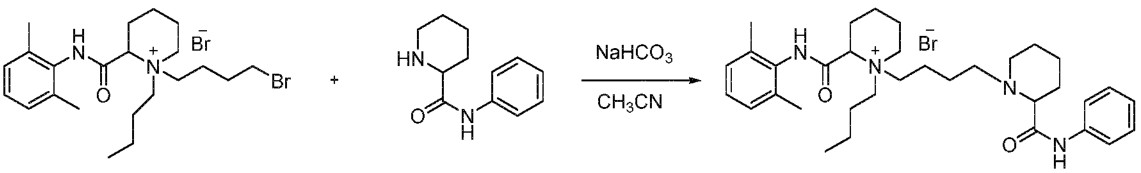


7a 2d 8

Compound 7a (1.18 mmol, 1 eq, 0.60 g) was weighed and added to analytically pure acetonitrile (10 eq of 7a, V/m, 6.0 mL) and stirred to dissolve, analytically pure sodium bicarbonate (1 eq of 7a, m/m, 0.60 g) was added, 2d (1.41 mmol, 1.2 eq, 0.29 g) was added, the mixture was heated to 75°C for reaction, and HPLC detection was carried out until the reaction was completed. The reaction solution was filtered, the filter cake was washed with acetonitrile (30 eq of 7a, V/m, 15 mL, in three times), the filtrate was collected, and white powdery solid compound 8 was obtained by preparative separation (0.16 g, with a yield of 21.6%, HPLC>98%). $^1$H NMR (400MHz, $CDCl_3$) $\delta$ (ppm): 10.61 (s, 1H), 9.90 (s, 1H), 7.33-7.10 (m, 8H), 4.94 (s, 1H), 4.16 (s, 1H), 3.70-3.55 (m, 2H), 3.48-3.12 (m, 8H), 2.25-2.09 (m, 6H), 1.81 (s, 6H), 1.68-1.11 (m, 17H). MS m/z (ESI) = 547.40 [M]$^+$.

**Example 9**

Synthesis of compound 9

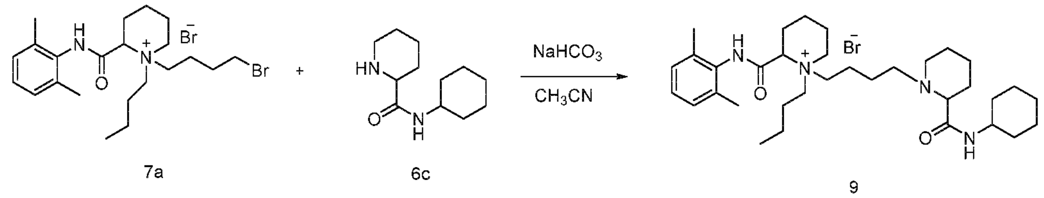


7a 6c 9

Compound 7a (1.18 mmol, 1 eq, 0.60 g) was weighed and added to analytically pure acetonitrile (10 eq of 7a, V/m, 6.0 mL) and stirred to dissolve, analytically pure sodium bicarbonate (1 eq of 7a, m/m, 0.60 g) was added, compound 6c (1.41 mmol, 1.2 eq, 0.30 g) was added, the mixture was heated to 75°C for reaction, and HPLC detection was carried out until the reaction was completed. The reaction solution was filtered, the filter cake was washed with acetonitrile (30 eq of 7a, V/m, 15 mL, in three times), the filtrate was collected, and white powdery solid compound 9 was obtained by

preparative separation (0.18 g, with a yield of 24.1%, HPLC>98%). $^1$H NMR (400MHz, $CDCl_3$) δ (ppm): 10.56 (s, 1H), 8.54 (s, 1H), 7.16-7.09 (m, 3H), 4.65 (s, 1H), 4.27 (s, 2H), 4.04-3.3. 34 (m, 18H), 2.27-2.14 (m, 8H), 2.22 (s, 6H), 2.05-1.39 (m, 17H). MS m/z (ESI) = 553.43 [M]$^+$.

**Example 10**

Synthesis of compound 10b

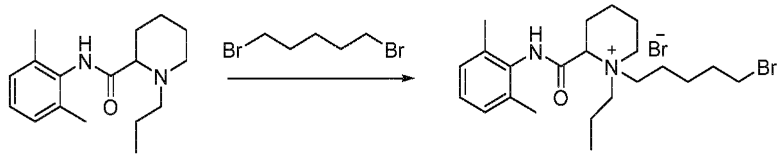

10a 10b

Ropivacaine (18.22 mmol, 1.0 eq, 5 g) was weighed and added to 1,5-dibromopentane (2.0 eq of Ropivacaine , V/m, 10 mL), and the mixture was stirred and heated to 75°C, and detected by TCL until the reaction was complete. Silica gel column chromatography was performed, with the eluant: $CH_2Cl_2$:MeOH = 20:1, the eluate was collected and concentrated by rotary evaporation to obtain orange-yellow oily liquid compound 10b (5.1 g, with a yield of 55.5%, HPLC>90%). MS m/z (ESI) = 423.20 [M]$^+$, 425.20 [M + H]$^+$.

Synthesis of compound 10

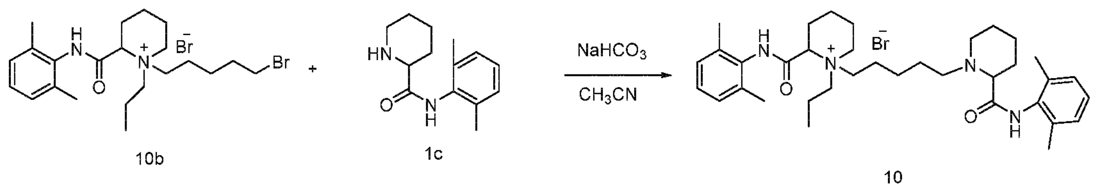


10b 1c 10

Compound 10b (1.18 mmol, 1 eq, 0.60 g) was weighed and added to analytically pure acetonitrile (10 eq of 10b, V/m, 6.0 mL) and stirred to dissolve, analytically pure sodium bicarbonate (1 eq of 7a, m/m, 0.60 g) was added, N-(2',6'-dimethylphenyl)-2-piperidinformamide 1c (1.41 mmol, 1.2 eq, 0.33 g) was added, the mixture was heated to 75°C for reaction, and HPLC detection was carried out until the reaction was completed. The reaction solution was filtered, the filter cake was washed with acetonitrile (30 eq of compound 10b, V/m, 15 mL, in three times), the filtrate was collected, and white powdery solid compound 10 was obtained by preparative separation (0.21 g, with a yield of 27.14%, HPLC>98%). $^1$H NMR (400MHz, $CDCl_3$) δ (ppm): 10.49 (s, 1H), 9.35 (s, 1H), 7.15-6.98 (m, 6H), 4.10 (s, 2H), 3.74-3.59 (m, 2H), 3.44-3.20 (m, 10H), 2.26-2.21 (m, 6H), 2.19 (s, 12H), 1.56-1.01 (m, 15H). MS m/z (ESI) = 575.45 [M]$^+$.

**Example 11**

Synthesis of compound 11

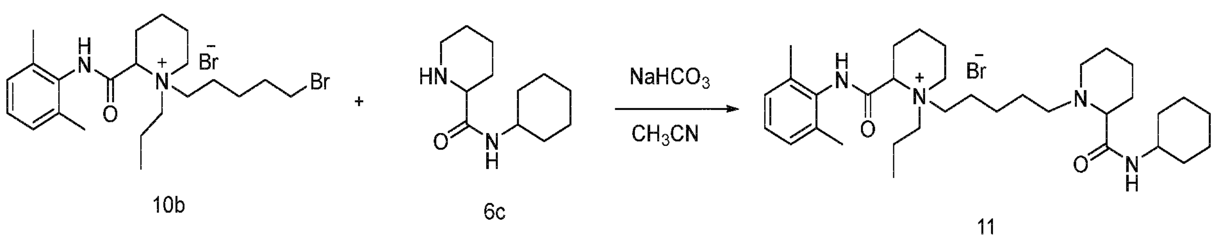


10b 6c 11

Compound 10b (1.18 mmol, 1 eq, 0.60 g) was weighed and added to analytically pure acetonitrile (10 eq of compound 10b, V/m, 6.0 mL) and stirred to dissolve, analytically pure sodium bicarbonate (1 eq of compound 10b, m/m, 0.60 g) was added, compound 6c (1.41 mmol, 1.2 eq, 0.30 g) was added, the mixture was heated to 75°C for reaction, and HPLC detection was carried out until the reaction was completed. The reaction solution was filtered, the filter cake was washed with acetonitrile (30 eq of 10b, V/m, 15 mL, in three times), the filtrate was collected, and white powdery solid compound 11 was obtained by preparative separation (0.11 g, with a yield of 14.7%, HPLC>98%). $^1H$ NMR (400MHz, $CDCl_3$) δ (ppm): 10.58 (s, 1H), 9.33 (s, 1H), 7.14-7.07 (m, 3H), 5.14 (s, 1H), 4.26 (s, 2H), 4.04-3.20 (m, 20H), 2.27-2.14 (m, 8H), 2.23 (s, 6H), 2.05-1.34 (m, 15H). MS m/z (ESI) = 553.41 $[M]^+$.

**Example 12**

Synthesis of compound 12

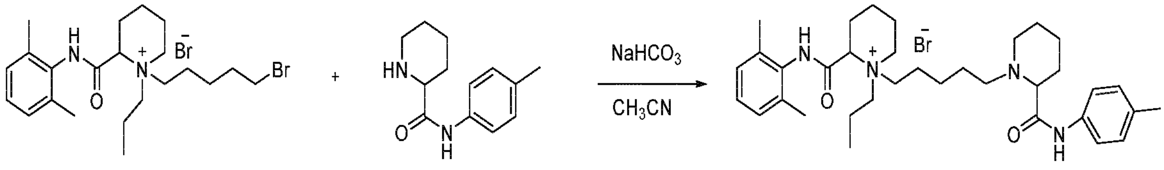


10b 4c 12

Compound 10b (1.18 mmol, 1 eq, 0.60 g) was weighed and added to analytically pure acetonitrile (10 eq of compound 10b, V/m, 6.0 mL) and stirred to dissolve, analytically pure sodium bicarbonate (1 eq of compound 10b, m/m, 0.60 g) was added, compound 4c (1.41 mmol, 1.2 eq, 0.31 g) was added, the mixture was heated to 75°C for reaction, and HPLC detection was carried out until the reaction was completed. The reaction solution was filtered, the filter cake was washed with acetonitrile (30 eq of 10b, V/m, 15 mL, in three times), the filtrate was collected, and white powdery solid compound 12 was obtained by preparative separation (0.15 g, with a yield of 19.8%, HPLC>98%). $^1H$NMR (400MHz, $CDCl_3$) δ (ppm): 10.57 (s, 1H), 9.30 (s, 1H), 7.56-7.31 (m, 7H), 4.36 (s, 2H), 3.88-3.66 (m, 2H), 3.51-3.15 (m, 10H), 2.55-2.34 (m, 6H), 2.24 (s, 9H), 1.84-1.42 (m, 15H). MS m/z (ESI) = 561.48 $[M]^+$.

**Example 13**

Synthesis of compound 13

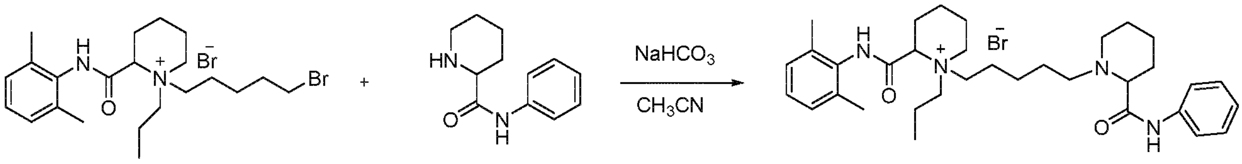


10b 2d 13

Compound 10b (1.18 mmol, 1 eq, 0.60 g) was weighed and added to analytically pure acetonitrile (10 eq of compound 10b, V/m, 6.0 mL) and stirred to dissolve, analytically pure sodium bicarbonate (1 eq of compound 10b, m/m, 0.60 g) was added, compound 2d (1.41 mmol, 1.2 eq, 0.29 g) was added, the mixture was heated to 75°C for reaction, and HPLC detection was carried out until the reaction was completed. The reaction solution was filtered, the filter cake was washed with acetonitrile (30 eq of 10b, V/m, 15 mL, in three times), the filtrate was collected, and white powdery solid compound 13 was obtained by preparative separation (0.18 g, with a yield of 24.3%, HPLC>98%). $^1H$ NMR (400MHz, $CDCl_3$) ClMHz%): 10.58 (s, 1H), 9.23 (s, 1H), 7.32-7.11 (m, 8H), 4.20 (s, 2H), 3.70-3.55 (m, 2H), 3.48-3.11 (m, 10H), 2.25-2.09 (m, 6H), 1.85 (s, 6H), 1.68-1.23 (m, 15H). MS m/z (ESI) = 547.32 $[M]^+$.

**Example 14**

Synthesis of 8b

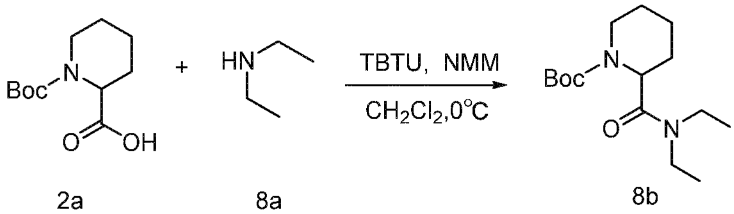


2a 8a 8b

N-Boc-2-piperidinecarboxylic acid (4.37 mmol, 1.0 eq, 1 g) was weighed and added to analytically pure dichloromethane (25 eq of N-Boc-2-piperidinecarboxylic acid, V/m, 25 mL), and stirred to dissolve, after the mixture was cooled to 0°C in an ice water bath, O-benzotriazole-N,N,N',N'-tetramethyluronium tetrafluoroborate (TBTU, 5.24 mmol, 1.2 eq, 1.68 g) was added. After stirring for about 30 minutes at 0°C, diethylamine (5.24 mmol, 1.2 eq, 0.54 mL) and N-methylmorpholine (NMM, 8.74 mmol, 2 eq, 0.96 mL) were added, and after stirring for about 1 hour at 0°C, the mixture was stirred at room temperature and detected by HPLC until the reaction was completed. The reaction solution was distilled under reduced pressure to obtain a yellow oily liquid. The organic phase was dissolved with a mixed solvent of dichloromethane:methanol=20:1 (70 eq of N-Boc-2-piperidinecarboxylic acid, V/m, mL/g, 70 mL) and washed with 5% $NaHSO_4$ aqueous solution (70 eq of N-Boc-2-piperidinecarboxylic acid, V/m, 70 mL, in three times). The organic phase was collected and distilled under reduced pressure to obtain a yellow oily viscous liquid. Column chromatography purification was performed, with the eluant: $CH_2Cl_2$:$CH_3OH$ = 40:1. The eluate was collected and distilled under reduced pressure to obtain pale yellow solid 8b (1.12 g, with a yield of 90.3%, HPLC>95%). MS m/z (ESI) = 285.21 $[M + H]^+$.

Synthesis of 8c

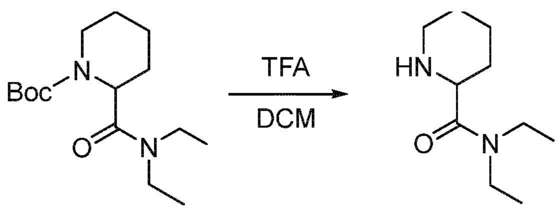


8b 8c

8b (3.51 mmol, 1 eq, 1.00 g) was weighed and added to analytically pure dichloromethane (5 eq of 8b, V/m, 5 mL) and stirred to dissolve. Trifluoroacetic acid (7.0 eq of 8b, V/m, 7 mL) was dissolved in analytically pure dichloromethane (10 eq of 8b, V/m, 10 mL) dropwise, and the mixture was stirred for about 2 hours and detected by HPLC until the reaction was completed. The reaction solution was distilled under reduced pressure by adding analytically pure dichloromethane (10 eq of 8b, V/m, 10 mL, in three times) and analytically pure methanol (10 eq of 8b, V/m, 10 mL, in three times) to obtain white solid 8c (0.61 g, with a yield of 93.8%, HPLC>90%). MS m/z (ESI) = 185.16 $[M + H]^+$.

Synthesis of compound 14

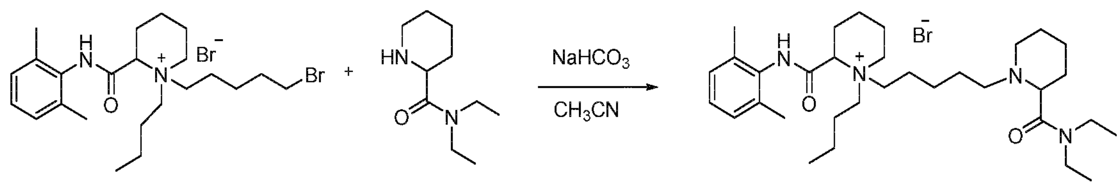


1b 8c 14

1b (1.14 mmol, 1 eq, 0.60 g) was weighed and added to analytically pure acetonitrile (10 eq of 1b, V/m, 6.0 mL) and stirred to dissolve, analytically pure sodium bicarbonate (1 eq of 1b, m/m, 0.60 g) was added, 8c (1.37 mmol, 1.2 eq, 0.25 g) was added, the mixture was heated to 75°C for reaction, and HPLC detection was carried out until the reaction was completed. The reaction solution was filtered, the filter cake was washed with acetonitrile (30 eq of 1b, V/m, 15 mL, in three times), the filtrate was collected, and white powdery solid compound 14 was obtained by preparative separation (0.15 g, with a yield of 21.1%, HPLC>98%). $^1$H NMR (400MHz, $CD_3OD$): 7.05 (s, 3H), 4.61-4.56 (m, 1H), 3.27-3.18 (m, 11H), 2.51-2.41 (m, 4H), 2.17-1.92 (m, 8H), 1.86-1.08 (m, 26H), 0.92-0.87 (m, 3H). MS m/z (ESI) = 541.45 [M] +.

**Example 15**

Synthesis of 9b

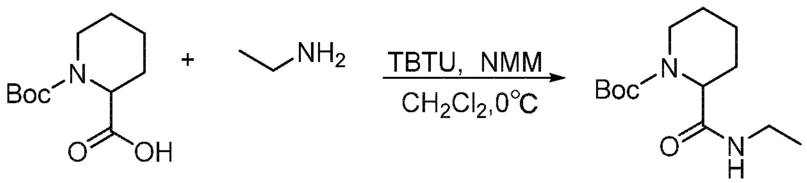


2a 9a 9b

N-Boc-2-piperidinecarboxylic acid (4.37 mmol, 1.0 eq, 1 g) was weighed and added to analytically pure dichloromethane (25 eq of N-Boc-2-piperidinecarboxylic acid, V/m, 25 mL), and stirred to dissolve, after the mixture was cooled to 0°C in an ice water bath, O-benzotriazole-N,N,N',N'-tetramethyluronium tetrafluoroborate (TBTU, 6.55 mmol, 1.5 eq, 2.10 g) was added. After stirring for about 30 minutes at 0°C, 70% aqueous ethylamine solution (13.10 mmol, 3 eq, 1.06 mL) and N-methylmorpholine (NMM, 8.74 mmol, 2 eq, 0.96 mL) were added, and after stirring for about 1 hour at 0°C, the mixture was stirred at room temperature and detected by HPLC until the reaction was completed. The reaction solution was distilled under reduced pressure to obtain a yellow oily liquid. The organic phase was dissolved with a mixed solvent of dichloromethane:methanol=20:1 (70 eq of N-Boc-2-piperidinecarboxylic acid, V/m, mL/g, 70 mL) and washed with 5% $NaHSO_4$ aqueous solution (70 eq of N-Boc-2-piperidinecarboxylic acid, V/m, mL/g, 70 mL, in three times). The organic phase was collected and distilled under reduced pressure to obtain a yellow oily viscous liquid. Column chromatography purification was performed, eluant: $CH_2Cl_2$:$CH_3OH$ = 40:1, the eluate was collected and distilled under reduced pressure to obtain white solid 9b (1.03 g, with a yield of 92.0%, HPLC>95%). MS m/z (ESI) = 257.18 $[M + H]^+$.

Synthesis of 9c

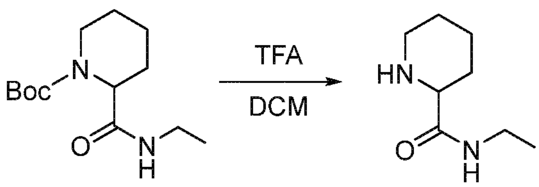


9b 9c

9b (3.90 mmol, 1 eq, 1.00 g) was weighed and added to analytically pure dichloromethane (5 eq of 9b, V/m, mL/g, 5 mL) and stirred to dissolve. Trifluoroacetic acid (7.0 eq of 9b, V/m, mL/g, 7 mL) was dissolved in analytically pure dichloromethane (10 eq of 9b, V/m, mL/g, 10 mL) dropwise, and the mixture was stirred for about 2 hours and detected by HPLC until the reaction was completed. The reaction solution was distilled under reduced pressure by adding analytically pure dichloromethane (10 eq of 9b, V/m, mL/g, 10 mL, in three times) and analytically pure methanol (10 eq of 9b, V/m, mL/g, 10 mL, in three times) to obtain white solid 9c (0.57 g, with a yield of 95.0%, HPLC>90%). MS m/z (ESI) = 157.13 $[M + H]^+$.

Synthesis of compound 15

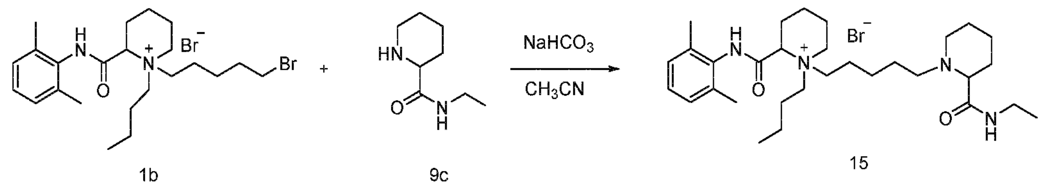


1b 9c 15

1b (1.14 mmol, 1 eq, 0.60 g) was weighed and added to analytically pure acetonitrile (10 eq of 1b, V/m, 6.0 mL) and stirred to dissolve, analytically pure sodium bicarbonate (1 eq of 1b, m/m, 0.60 g) was added, 9c (1.34 mmol, 1.2 eq, 0.21 g) was added, the mixture was heated to 75°C for reaction, and HPLC detection was carried out until the reaction was completed. The reaction solution was filtered, the filter cake was washed with acetonitrile (15 mL, in three times), the filtrate was collected, and white powdery solid compound 15 was obtained by preparative separation (0.11 g, with a yield of 16.4%, HPLC>98%). $^1$H NMR (400MHz,$CD_3OD$) δ (ppm): 8.57 (s, 1H), 8.45 (s, 1H), 7.15-7.07 (m, 3H), 5.05-4.89 (m, 1H), 4.28 (s, 1H), 3.87-3.84 (m, 2H), 3.67-3.23 (m, 10H), 2.21 (s, 6H), 1.92-1.86 (m, 9H), 1.48-1.37 (m, 8H), 1.16-0.98 (m, 11H). MS m/z (ESI) = 513.34 $[M]^+$.

**Example 16**

Synthesis of 11b

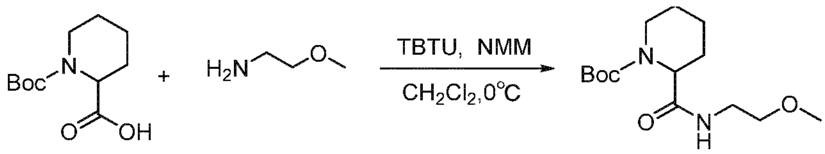


2a 11a 11b

N-Boc-2-piperidinecarboxylic acid (4.37 mmol, 1.0 eq, 1 g) was added to analytically pure dichloromethane (25 eq of N-Boc-2-piperidinecarboxylic acid, V/m, 25 mL), and stirred to dissolve, after the mixture was cooled to 0°C in an ice water bath, O-benzotriazole-N,N,N',N'-tetramethyluronium tetrafluoroborate (TBTU, 5.24 mmol, 1.2 eq, 1.68 g) was added. After stirring for about 30 minutes at 0°C, 2-methoxyethylamine (5.24 mmol, 1.2 eq, 0.46 mL) and N-methylmorpholine (NMM, 8.74 mmol, 2 eq, 0.96 mL) were added, and after stirring for about 1 hour at 0°C, the mixture was stirred at room temperature and detected by HPLC until the reaction was completed. The reaction solution was distilled under reduced pressure to obtain a yellow oily liquid. The organic phase was dissolved with a mixed solvent of dichloromethane: methanol = 20:1 (70 eq of N-Boc-2-piperidinecarboxylic acid, V/m, mL/g, 70 mL), and washed with 5% $NaHSO_4$ aqueous solution (70 eq of N-Boc-2-piperidinecarboxylic acid, V/m, 70 mL, in three times). The organic phase was collected and distilled under reduced pressure to obtain a yellow oily viscous liquid. Column chromatography purification was performed, eluant: $CH_2Cl_2$:$CH_3OH$ = 40:1, the eluate was collected and distilled under reduced pressure to obtain a pale yellow solid 11b (1.11 g, with a yield of 88.8%, HPLC>95%). MS m/z (ESI) = 287.19 $[M + H]^+$.

Synthesis of 11c

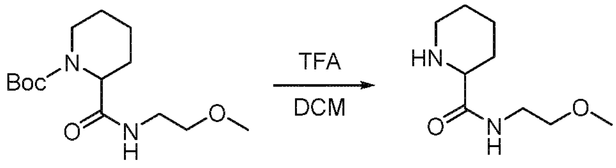


11b 11c

11b (3.49 mmol, 1 eq, 1.00 g) was weighed and added to analytically pure dichloromethane (5 eq of 11b, V/m, 5 mL) and stirred to dissolve. Trifluoroacetic acid (7.0 eq of 11b, V/m, 7 mL) was dissolved in analytically pure dichloromethane (10 eq of 11b, V/m, 10 mL) dropwise, and the mixture was stirred for about 2 hours and detected by HPLC until the reaction was completed. The reaction solution was distilled under reduced pressure by adding analytically pure dichloromethane (10 mL, in three times) and analytically pure methanol to obtain a white solid 11c (0.60 g, with a yield of 92.3%, HPLC>90%). MS m/z (ESI) = 187.14 [M + H]$^+$.

Synthesis of compound 16

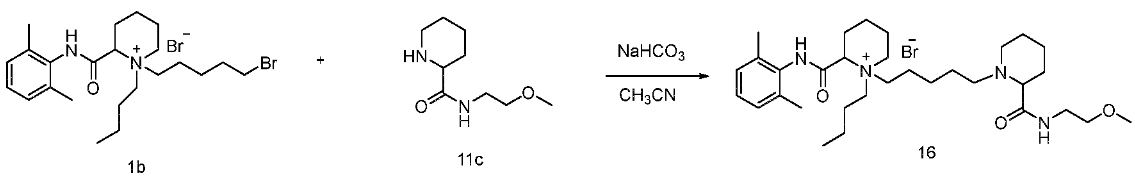


1b 11c 16

1b (1.14 mmol, 1 eq, 0.60 g) was weighed and added to analytically pure acetonitrile (10 eq of 1b, V/m, 6.0 mL) and stirred to dissolve, analytically pure sodium bicarbonate (1 eq of 1b, m/m, 0.60 g) was added, 11c (1.37 mmol, 1.2 eq of 1b, 0.25 g) was added, the mixture was heated to 75°C for reaction, and HPLC detection was carried out until the reaction was completed. The reaction solution was filtered, the filter cake was washed with acetonitrile (15 mL, in three times), the filtrate was collected, and white powdery solid compound 16 was obtained by preparative separation (0.14 g, with a yield of 19.7%, HPLC>98%). $^1$H NMR (400MHz, CD$_3$OD): 8.01 (s, 1H), 7.05 (s, 3H), 4.61-4.56 (m, 1H), 3.67 (m, 2H), 3.37-3.17 (m, 12H), 2.51-2.41 (m, 4H), 2.17-1.92 (m, 8H), 1.86-1.20 (m, 20H), 0.92-0.87 (m, 3H). MS m/z (ESI) = 543.43 [M]$^+$.

**Example 17**

Synthesis of 17e

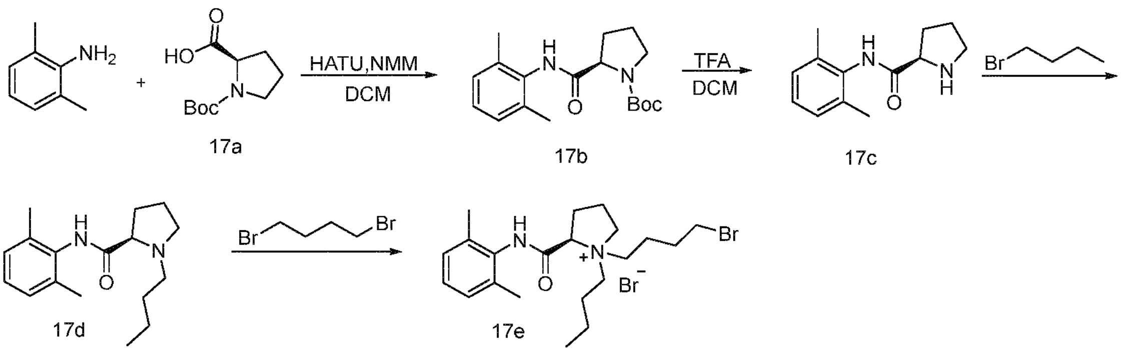


17a 17b 17c 17d 17e

Boc-N-proline 17a (10.0 mmol, 1.0 eq, 2.15 g) was weighed and added to analytically pure dichloromethane (10 eq of 17a, V/m, 20 mL) at 0°C and stirred to dissolve,2-(7-azabenzotriazole)-N,N,N',N'-tetramethyluronium hexafluorophosphate (HATU, 10.0 mmol, 7.6 g) was added and stirred at 0°C for 1h, then 2,6-dimethylaniline (15 mL) and

N-methylmorpholine (NMM, 8.6 mL) were added, and after stirring for 1h at 0°C, the mixture was moved to room temperature and detected by TCL until the reaction was complete, followed by extraction with $NH_4Cl$ saturated solution (50 mL×3) and then with NaHCOs saturated solution (50 mL×3) to collect the organic layer. Silica gel column chromatography was performed, with the eluant: $CH_2Cl_2$:MeOH = 20:1, the eluate was collected and concentrated by rotary evaporation to obtain orange-yellow oily liquid compound 17b (3.10 g, with a yield of 98.0%, HPLC>90%). MS m/z (ESI) = 218.5 $[M + H]^+$.

17b (5.0 mmol, 1.0 eq, 1.59 g) was weighed and added to 16 mL of analytically pure dichloromethane and stirred to dissolve, 2 mL of trifluoroacetic acid was added dropwise, and TCL detection was carried out until the reaction was complete. Orange-yellow oily liquid compound 17c was obtained by rotary evaporation and concentration (1.2 g, with a yield of 98%, HPLC>90%). MS m/z (ESI) = 218.5 $[M + H]^+$.

17c (5.0 mmol, 1.0 eq, 1.2 g) was weighed and added to 12 mL of analytically pure acetonitrile to dissolve, 1.4 g potassium carbonate and 1.4 g bromobutane were added for reaction at 80°C. TCL detection was carried out until the reaction was complete. The reaction solution was filtered, the filter cake was washed with acetonitrile (15 mL, in three times), and pale yellow solid compound 17d was obtained by rotary evaporation and concentration (1.3 g, with a yield of 95%, HPLC>90%). MS m/z (ESI) = 275. 5 $[M + H]^+$.

17d (5.0 mmol, 1.0 eq, 2.75 g) was weighed and added to 6 mL of 1,4-dibromobutane, and the mixture was stirred and heated to 100°C, and detected by TCL until the reaction was complete. Silica gel column chromatography was performed, eluant: $CH_2Cl_2$:MeOH = 20:1. The eluate was collected and concentrated by rotary evaporation to obtain orange-yellow oily liquid compound 17e (1.5 g, with a yield of 73.17%, HPLC>90%). MS m/z (ESI) = 409.19 $[M]^+$, 410.99 $[M+2H]^+$.

Synthesis of compound 17

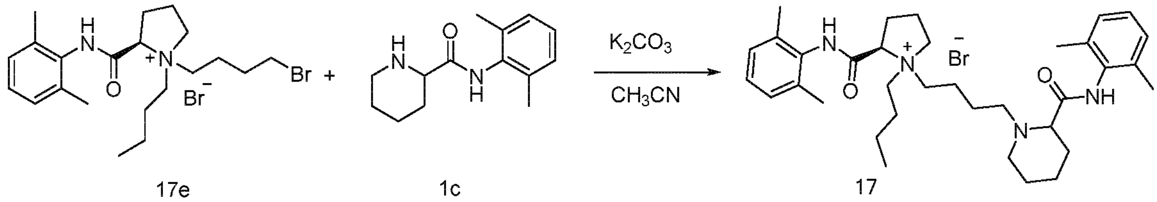


17e 1c 17

17e (1.14 mmol, 1 eq, 0.56 g) was weighed and added to analytically pure acetonitrile (10 eq of 17e, V/m, 5.6 mL) and stirred to dissolve, analytically pure potassium carbonate (1 eq of 17e, m/m, 0.56 g) was added, and 1c (2.28 mmol, 2 eq of 17e, 0.53 g) was added. The mixture was heated to 80°C, and the reaction was monitored by TLC until the reaction was completed. The reaction solution was filtered, the filter cake was washed with acetonitrile (18 mL, in three times), the filtrate was collected, and white solid compound 17 was obtained by preparative separation (0.36 g, with a yield of 49.3%, HPLC>98%). $^1H$ NMR (400MHz, $CD_3OD$) δ (ppm): 10.51 (m, 1H), 9.35 (m, 1H), 7.15-6.98 (m, 6H), 4.10 (s, 2H), 3.74-3.59 (m, 2H), 3.43-3.21 (m, 10H), 2.27-2.22 (m, 6H), 2.18-2.01 (m, 12H), 1.57-1.02 (m, 13H), MS m/z (ESI) = 561.41 $[M]^+$.

### Example 18

Synthesis of compound 18

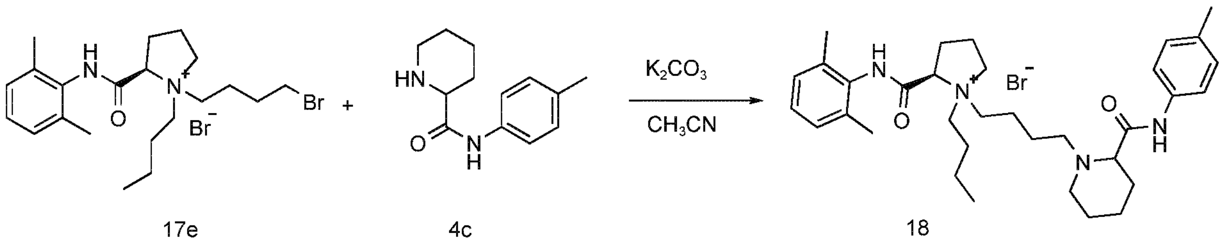


17e 4c 18

17e (1.14 mmol, 1 eq, 0.56 g) was weighed and added to analytically pure acetonitrile (10 eq of 17e, V/m, 5.0

mL) and stirred to dissolve, analytically pure potassium carbonate (1 eq of 17e, m/m, 0.56 g) was added, and 4c (2.28 mmol, 2 eq of 17e, 0.50 g) was added. The mixture was heated to 80°C, and the reaction was monitored by thin layer chromatography until the reaction was completed. The reaction solution was filtered, the filter cake was washed with acetonitrile (18 mL, in three times), the filtrate was collected, and white solid compound 18 was obtained by preparative separation (0.43 g, with a yield of 59.7%, HPLC>98%). $^1H$ NMR (400MHz, $CD_3OD$) $\delta$ (ppm): 10.53 (s, 1H), 9.32 (s, 1H), 7.45-7.27 (m, 7H), 4.25 (m, 2H), 3.80-3.66 (m, 2H), 3.49-3.11 (m, 10H), 2.55-2.34 (m, 6H), 2.20-2.10 (m, 9H), 1.79-1.45 (m, 13H). MS m/z (ESI) = 547.38 $[M]^+$.

**Example 19**

Synthesis of 12c

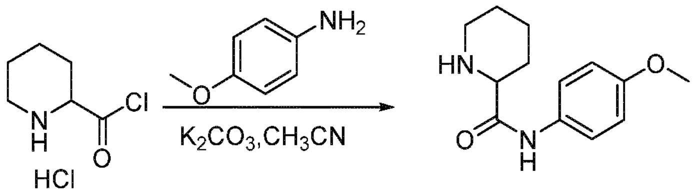


12c

Piperidinecarbonyl chloride hydrochloride (2.0 mmol, 1 eq, 0.36 g) was weighed and added to 3.0 mL of analytically pure acetonitrile and stirred to dissolve, 0.55 g of analytically pure potassium carbonate was added, and p-methoxyaniline (4.0 mmol, 0.42 g) was added. The mixture was heated to 80°C, and the reaction was monitored by thin layer chromatography until the reaction was completed. The reaction solution was filtered, the filter cake was washed with acetonitrile (15 mL, in three times), the filtrate was collected and concentrated by rotary evaporation, and silica gel column chromatography was performed, with the eluant: $CH_2Cl_2$:MeOH = 40: 1. The eluate was collected and concentrated by rotary evaporation to obtain yellowish-brown solid compound 12c (0.40 g, with a yield of 89.8%, HPLC>98%). MS m/z (ESI) = 235.2 $[M + H]^+$.

Synthesis of compound 19

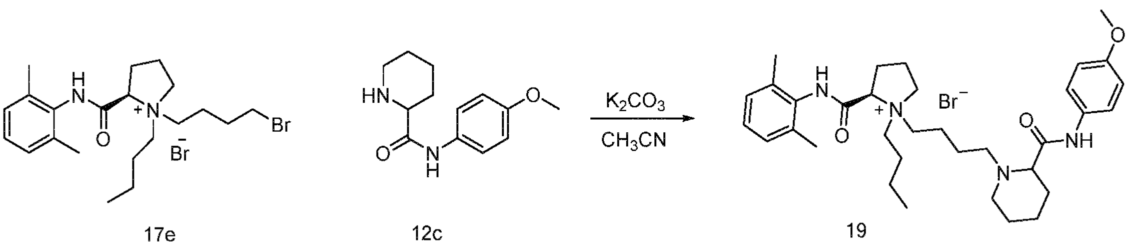


17e 12c 19

17e (1.14 mmol, 1 eq, 0.56 g) was weighed and added to analytically pure acetonitrile (10 eq of 17e, V/m, 5.0 mL) and stirred to dissolve, analytically pure potassium carbonate (1 eq of 17e, m/m, 0.56 g) was added, and 12c (2.28 mmol, 2 eq of 17e, 0.53 g) was added. The mixture was heated to 80°C, and the reaction was monitored by thin layer chromatography until the reaction was completed. The reaction solution was filtered, the filter cake was washed with acetonitrile (18 mL, in three times), the filtrate was collected, and white solid compound 19 was obtained by preparative separation (0.49 g, with a yield of 66.7%, HPLC>98%). $^1H$ NMR (400MHz, $CD_3OD$) $\delta$ (ppm): 10.50 (s, 1H), 9.01 (s, 1H), 7.45-7.27 (M, 5H), 7.10-7.00 (M, 2H), 4.25-4.00 (M, 2H), 3.79-3.66 (M, 2H), 3.50-3.10 (M, 10H), 2.54-2.33 (M, 6H), 2.19-2.10 (M, 9H), 1.79-1.45 (M, 13H), MS m/z (ESI) = 563.32 $[M]^+$.

**Example 20**

Synthesis of 13c

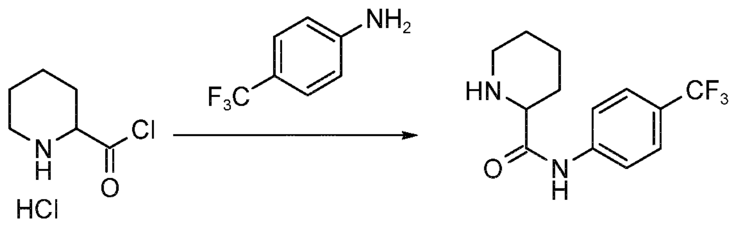

13c

Piperidinecarbonyl chloride hydrochloride (2.0 mmol, 1 eq, 0.36 g) was weighed and added to 3.0 mL of analytically pure acetonitrile and stirred to dissolve, 0.55 g of analytically pure potassium carbonate was added, and p-trifluoromethylaniline (4.0 mmol, 0.42 g) was added. The mixture was heated to 80°C, and the reaction was monitored by thin layer chromatography until the reaction was completed. The reaction solution was filtered, the filter cake was washed with acetonitrile (15 mL, in three times), the filtrate was collected and concentrated by rotary evaporation, and silica gel column chromatography was performed, with the eluant: $CH_2Cl_2$:MeOH = 40:1. The eluate was collected and concentrated by rotary evaporation to obtain yellowish-brown solid compound 13c (0.41 g, with a yield of 90.3%, HPLC>98%). MS m/z (ESI) = 273.2 $[M + H]^+$.

Synthesis of compound 20

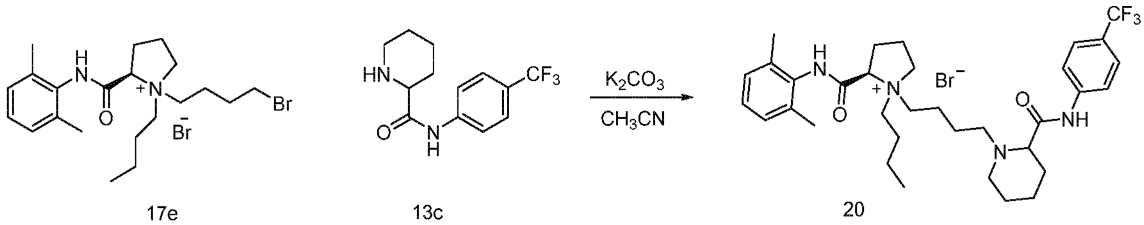


17e 13c 20

17e (1.14 mmol, 1 eq, 0.56 g) was weighed and added to analytically pure acetonitrile (10 eq of 17e, V/m, 5.0 mL) and stirred to dissolve, analytically pure potassium carbonate (1 eq of 17e, m/m, 0.56 g) was added, and 13c (2.28 mmol, 2 eq of 17e, 0.62 g) was added. The mixture was heated to 80°C, and the reaction was monitored by thin layer chromatography until the reaction was completed. The reaction solution was filtered, the filter cake was washed with acetonitrile (18 mL, in three times), the filtrate was collected, and white solid compound 20 was obtained by preparative separation (0.30 g, with a yield of 38.6%, HPLC>98%). $^1H$ NMR (400MHz, $CD_3OD$) δ (ppm): 10.54 (s, 1H), 9.47 (s, 1H), 7.78-7.30 (m, 7H), 4.26-4.01 (m, 2H), 3.81-3.70 (m, 2H), 3.53-3.14 (m, 10H), 2.57-2.32 (m, 6H), 2.25-2.10 (m, 9H), 1.85-1.54 (m, 13H). MS m/z (ESI) = 601.29 $[M]^+$.

**Example 21**

Synthesis of compound 21

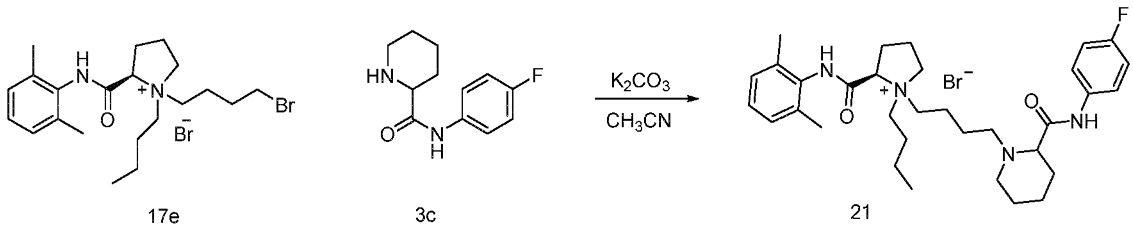


17e 3c 21

17e (1.14 mmol, 1 eq, 0.56 g) was weighed and added to analytically pure acetonitrile (10 eq of 17e, V/m, 5.6 mL) and stirred to dissolve, analytically pure potassium carbonate (1 eq of 17e, m/m, 0.56 g) was added, and 3c (2.28 mmol, 2 eq of 17e, 0.50 g) was added. The mixture was heated to 80°C, and the reaction was monitored by TLC until the reaction was completed. The reaction solution was filtered, the filter cake was washed with acetonitrile (18 mL, in three times), the filtrate was collected, and white solid compound 21 was obtained by preparative separation (0.36 g, with a yield of 54.2%, HPLC>98%). $^1$H NMR (400MHz, $CD_3OD$) δ (ppm): 10.53 (s, 1H), 9.41 (s, 1H), 7.68-7.26 (m, 7H), 4.25-4.00 (m, 2H), 3.83-3.69 (m, 2H), 3.55-3.20 (m, 10H), 2.67-2.30 (m, 6H), 2.23-2.10 (m, 9H), 1.81-1.52 (m, 13H). MS m/z (ESI) = 551.48 $[M]^+$.

**Example 22**

Synthesis of 18e

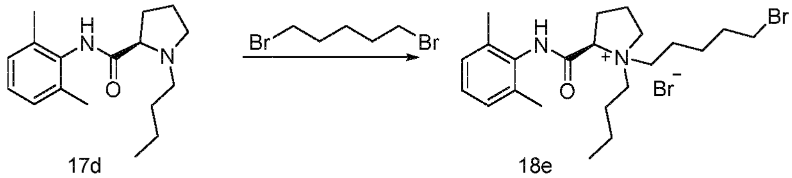

17d 18e

17d (5.0 mmol, 1.0 eq, 1.37 g) was weighed and added to 6 mL of 1,5-dibromopentane, and the mixture was stirred and heated to 100°C, and detected by TCL until the reaction was complete. Silica gel column chromatography was performed, with the eluant: $CH_2Cl_2$:MeOH = 20:1. The eluate was collected and concentrated by rotary evaporation to obtain yellowish-brown oily liquid compound 18e (1.8 g, with the yield of 71.4%, HPLC>90%). MS m/z (ESI) = 422.19 $[M]^+$, 424.28 $[M+2H]^+$.

Synthesis of compound 22

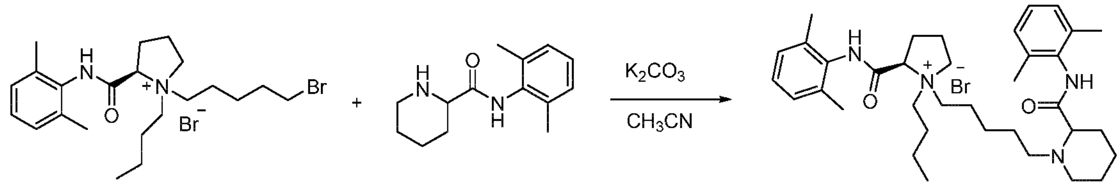


18e 1c 22

18e (1.14 mmol, 1 eq, 0.57 g) was weighed and added to analytically pure acetonitrile (10 eq of 18e, V/m, 5.7 mL) and stirred to dissolve, analytically pure potassium carbonate (1 eq of 18e, m/m, 0.57 g) was added, and 1c (2.28 mmol, 2 eq of 18e, 0.53 g) was added. The mixture was heated to 80°C, and the reaction was monitored by TLC until the reaction was completed. The reaction solution was filtered, the filter cake was washed with acetonitrile (18 mL, in three times), the filtrate was collected, and white solid compound 22 was obtained by preparative separation (0.38 g, with a yield of 51.0%, HPLC>98%). $^1$H NMR (400MHz, $CD_3OD$): 7.05 (s, 6H), 4.61-4.56 (m, 1H), 3.47-3.42 (m, 1H), 3.25-3.20 (m, 4H), 2.51-2.41 (m, 4H), 2.20-2.13 (m, 14H), 1.97-1.55 (m, 7H), 1.49-1.29 (m, 11H), 0.92-0.87 (m, 3H). MS m/z (ESI) = 574.40 $[M]^+$.

**Example 23**

Synthesis of compound 23

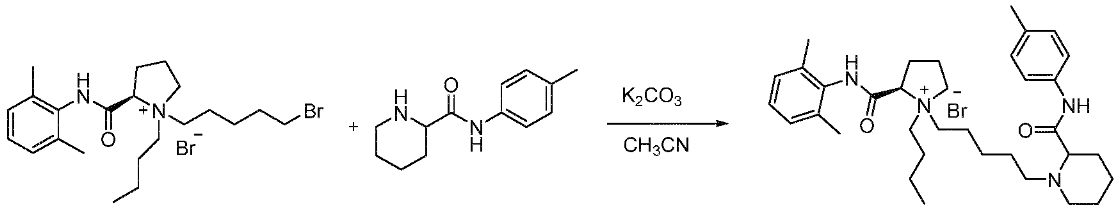


18e 4c 23

18e (1.14 mmol, 1 eq, 0.57 g) was weighed and added to analytically pure acetonitrile (10 eq of 18e, V/m, 5.7 mL) and stirred to dissolve, analytically pure potassium carbonate (1 eq of 18e, m/m, 0.57 g) was added, and 4c (2.28 mmol, 2 eq of 18e, 0.50 g) was added. The mixture was heated to 80°C, and the reaction was monitored by TLC until the reaction was completed. The reaction solution was filtered, the filter cake was washed with acetonitrile (18 mL, in three times), the filtrate was collected, and white solid compound 23 was obtained by preparative separation (0.45 g, with a yield of 61.7%, HPLC>98%). $^1H$ NMR (400MHz, $CD_3OD$): 9.70 (s, 1H), 7.44 (d, 2H), 7.10-7.05 (m, 5H), 4.61-4.56 (m, 1H), 3.47-3.42 (m, 1H), 3.25-3.20 (m, 4H), 2.51-2.13 (m, 15H), 1.97-1.45 (m, 7H), 1.55-1.29 (m, 11H), 0.92-0.87 (m, 3H). MS m/z (ESI) = 561.38 $[M]^+$.

**Example 24**

Synthesis of compound 24

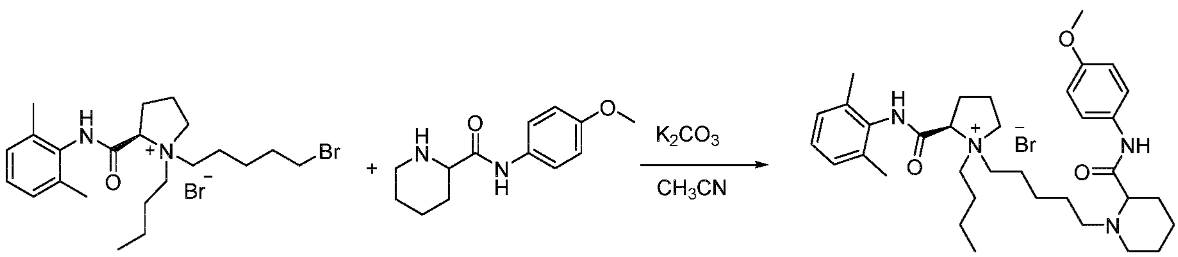


18e 12c 24

18e (1.14 mmol, 1 eq, 0.57 g) was weighed and added to analytically pure acetonitrile (10 eq of 18e, V/m, 5.7 mL) and stirred to dissolve, analytically pure potassium carbonate (1 eq of 18e, m/m, 0.57 g) was added, and 12c (2.28 mmol, 2 eq of 18e, 0.55 g) was added. The mixture was heated to 80°C, and the reaction was monitored by TLC until the reaction was completed. The reaction solution was filtered, the filter cake was washed with acetonitrile (18 mL, in three times), the filtrate was collected, and white solid compound 24 was obtained by preparative separation (0.49 g, with a yield of 65.4%, HPLC>98%). $^1H$ NMR (400MHz, $CD_3OD$): 7.41 (d, 2H), 7.13-7.02 (m, 3H), 6.86 (d, 2H), 4.04-3.94 (m, 1H), 3.89-3.80 (m, 1H), 3.79-3.62 (m, 5H), 3.59-3.48 (m, 1H), 3.44-3.33 (m, 2H), 3.30-3.14 (m, 3H), 3.12-3.01 (m, 1H), 2.77-2.60 (m, 1H), 2.40-2.12 (m, 1H), 1.97-1.48 (m, 10H), 1.40-1.16 (m, 3H), 0.92-0.87 (m, 3H). $^{13}C$ NMR (400 MHz, $CD_3OD$): δ 172.0, 158.9, 137.1, 130.8, 130.7, 127.7, 126.8, 122.6, 114.5, 83.9, 68.6, 64.7, 56.5, 56.2, 55.8, 53.7, 52.5, 27.6, 25.9, 25.8, 25.5, 25.3, 23.1, 21.2, 19.6, 19.2, 19.0, 17.6, 13.8. MS m/z (ESI) = 577.39 $[M]^+$.

**Example 25**

Synthesis of compound 25

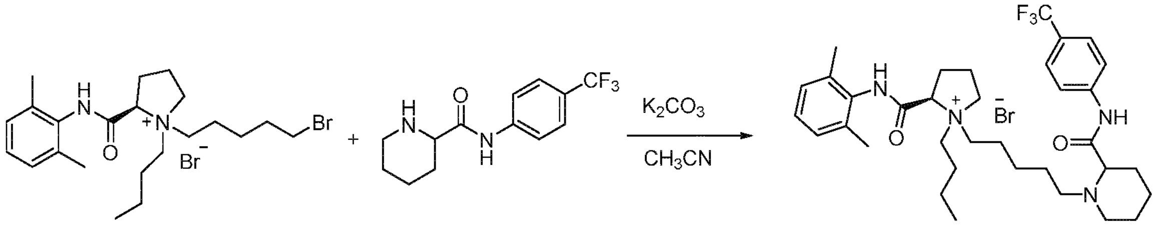


18e 13c 25

18e (1.14 mmol, 1 eq, 0.57 g) was weighed and added to analytically pure acetonitrile (10 eq of 18e, V/m, 5.7 mL) and stirred to dissolve, analytically pure potassium carbonate (1 eq of 18e, m/m, 0.57 g) was added, and 13c (2.28 mmol, 2 eq of 18e, 0.62 g) was added. The mixture was heated to 80°C, and the reaction was monitored by TLC until the reaction was completed. The reaction solution was filtered, the filter cake was washed with acetonitrile (18 mL, in three times), the filtrate was collected, and white solid compound 25 was obtained by preparative separation (0.42 g, with a yield of 53.2%, HPLC>98%). $^1H$ NMR (400MHz, $CD_3OD$): 7.57 (d, 2H), 7.46 (d, 2H), 7.05 (s, 3H), 4.61-4.56 (m, 1H), 3.47-3.42 (m, 1H), 3.27-3.17 (m, 6H), 2.51-2.41 (m, 4H), 2.17-1.92 (m, 9H), 1.76-1.29 (m, 17H), 0.92-0.87 (m, 3H). MS m/z (ESI) = 615.41 $[M]^+$.

**Example 26**

Synthesis of compound 26

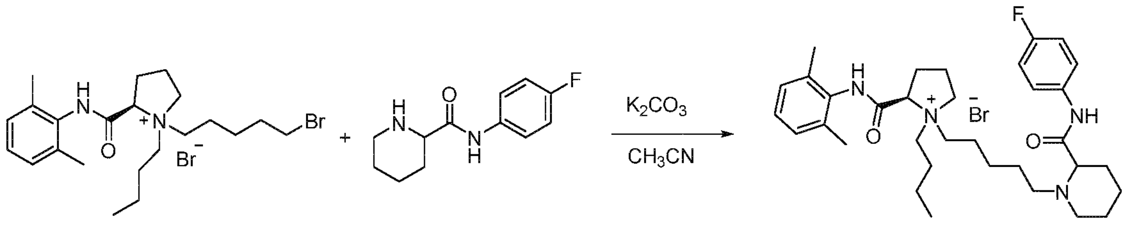


18e 3c 26

18e (1.14 mmol, 1 eq, 0.57 g) was weighed and added to analytically pure acetonitrile (10 eq of 18e, V/m, 5.7 mL) and stirred to dissolve, analytically pure potassium carbonate (1 eq of 18e, m/m, 0.57 g) was added, and 3c (2.28 mmol, 2 eq of 2e, 0.51 g) was added. The mixture was heated to 80°C, and the reaction was monitored by TLC until the reaction was completed. The reaction solution was filtered, the filter cake was washed with acetonitrile (18 mL, in three times), the filtrate was collected, and white solid compound 26 was obtained by preparative separation (0.30 g, with a yield of 40.8%, HPLC>98%). $^1H$ NMR (400MHz, $CD_3OD$): 7.59 (dd, 2H), 7.17-7.12 (m, 2H), 7.05 (s, 3H), 4.61-4.56 (m, 1H), 3.47-3.42 (m, 1H), 3.27-3.17 (m, 6H), 2.51-2.41 (m, 4H), 2.17-1.92 (m, 9H), 1.76-1.29 (m, 17H), 0.92-0.87 (m, 3H). MS m/z (ESI) = 565.39 $[M]^+$.

**Example 27**

Synthesis of 19e

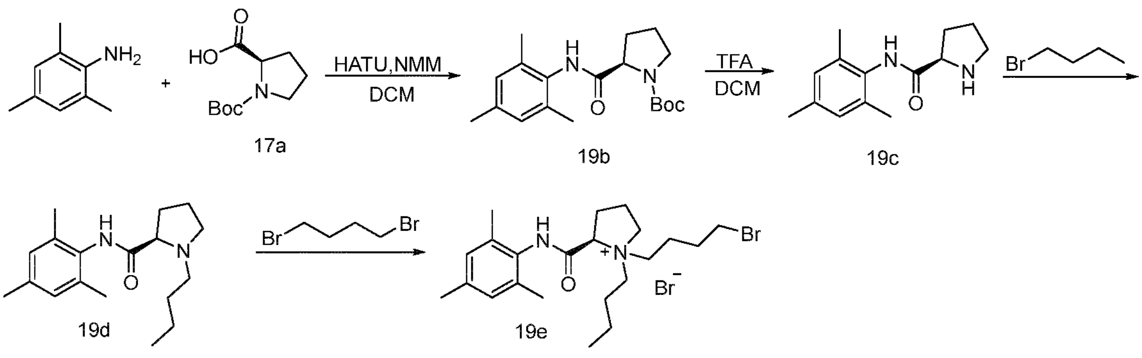


17a 19b 19c 19d 19e

Boc-N-proline 17a (10.0 mmol, 1.0 eq, 2.15 g) was weighed and added to analytically pure dichloromethane (10 eq of 17a, V/m, 20 mL) at 0°C and stirred to dissolve, 2-(7-azabenzotriazole)-N,N,N',N'-tetramethyluronium hexafluorophosphate (HATU, 20.0 mmol, 7.6 g) was added and stirred at 0°C for 1h, then 2,4,6-trimethylaniline (15 mL) and N-methylmorpholine (NMM, 8.6 mL) were added, and after stirring for 1h at 0°C, the mixture was moved to room temperature and detected by TCL until the reaction was complete, followed by extraction with $NH_4Cl$ saturated solution (50 mL×3) and then with $NaHCO_3$ saturated solution (50 mL×3) to collect the organic layer. Silica gel column chromatography was performed, with the eluant: $CH_2Cl_2$:MeOH = 20:1. The eluate was collected and concentrated by rotary evaporation to obtain orange-yellow oily liquid compound 19b (3.10 g, with a yield of 98%, HPLC>90%). MS m/z (ESI) = 233.5 $[M + H]^+$.

19b (5.0 mmol, 1.0 eq, 1.59 g) was weighed and added to 16 mL of analytically pure dichloromethane and stirred to dissolve, 2 mL of trifluoroacetic acid was added dropwise, and TCL detection was carried out until the reaction was complete. Orange-yellow oily liquid compound 19c was obtained by rotary evaporation and concentration (1.2 g, with a yield of 98%, HPLC>90%). MS m/z (ESI) = 233.5 $[M + H]^+$.

19c (5.0 mmol, 1.0 eq, 1.2 g) was weighed and added to 12 mL of analytically pure acetonitrile to dissolve, 2.4 g potassium carbonate and 1.4 g bromobutane were added for reaction at 80°C. TCL detection was carried out until the reaction was complete. The reaction solution was filtered, the filter cake was washed with acetonitrile (15 mL, in three times), and pale yellow solid compound 19d was obtained by rotary evaporation and concentration (1.3 g, with a yield of 95%, HPLC>90%). MS m/z (ESI) = 289.5 $[M + H]^+$.

19d (5.0 mmol, 1.0 eq, 2.75 g) was weighed and added to 6 mL of 1,4-dibromobutane, and the mixture was stirred and heated to 100°C, and detected by TCL until the reaction was complete. Silica gel column chromatography was performed, with the eluant: $CH_2Cl_2$: MeOH = 20:1. The eluate was collected and concentrated by rotary evaporation to obtain orange-yellow oily liquid compound 19e (1.5 g, with a yield of 70.2%, HPLC>90%). MS m/z (ESI) = 422.79 $[M]^+$, 424.79 $[M+2H]^+$.

Synthesis of compound 27

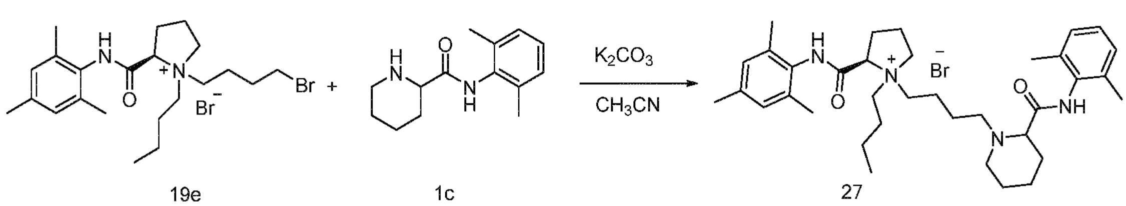


19e 1c 27

19e (1.14 mmol, 1 eq, 0.57 g) was weighed and added to analytically pure acetonitrile (10 eq of 19e, V/m, 5.7 mL) and stirred to dissolve, analytically pure potassium carbonate (1 eq of 19e, m/m, 0.57 g) was added, and 1c (2.28 mmol, 2 eq of 19e, 0.53 g) was added. The mixture was heated to 80°C, and the reaction was monitored by TLC until the reaction was completed. The reaction solution was filtered, the filter cake was washed with acetonitrile (30 eq of 19e, V/m, 18 mL, in three times), the filtrate was collected, and white solid compound 27 was obtained by preparative separation (0.39 g, with a yield of 52.3%, HPLC>98%). $^1H$ NMR (400MHz, $CD_3OD$): 7.05 (s, 3H), 6.90 (s, 2H), 4.61-4.56 (m, 1H), 3.47-3.42 (m, 1H), 3.27-3.17 (m, 6H), 2.51-2.41 (m, 4H), 2.26-1.92 (m, 18H), 1.76-1.30 (m, 15H), 0.92-0.87 (m, 3H). MS m/z (ESI) = 575.35 $[M]^+$.

**Example 28**

Synthesis of compound 28

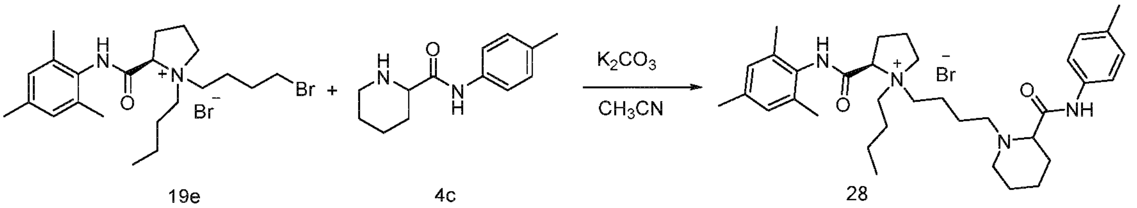


19e 4c 28

19e (1.14 mmol, 1 eq, 0.57 g) was weighed and added to analytically pure acetonitrile (10 eq of 19e, V/m, 5.07 mL) and stirred to dissolve, analytically pure potassium carbonate (1 eq of 19e, m/m, 0.57 g) was added, and 4c (2.28 mmol, 2 eq of 1e, 0.50 g) was added. The mixture was heated to 80°C, and the reaction was monitored by TLC until the reaction was completed. The reaction solution was filtered, the filter cake was washed with acetonitrile (30 eq of 19e, V/m, 18 mL, in three times), the filtrate was collected, and white solid compound 28 was obtained by preparative separation (0.40 g, with a yield of 54.6%, HPLC>98%). $^1$H NMR (400MHz, $CD_3OD$): 7.44 (d, 2H), 7.10 (d, 2H), 6.90 (s, 2H), 4.61-4.56 (m, 1H), 3.47-3.42 (m, 1H), 3.27-3.17 (m, 6H), 2.51-1.92 (m, 19H), 1.76-1.30 (m, 15H), 0.92-0.87 (m, 3H). MS m/z (ESI) = 561.40 $[M]^+$.

**Example 29**

Synthesis of compound 29

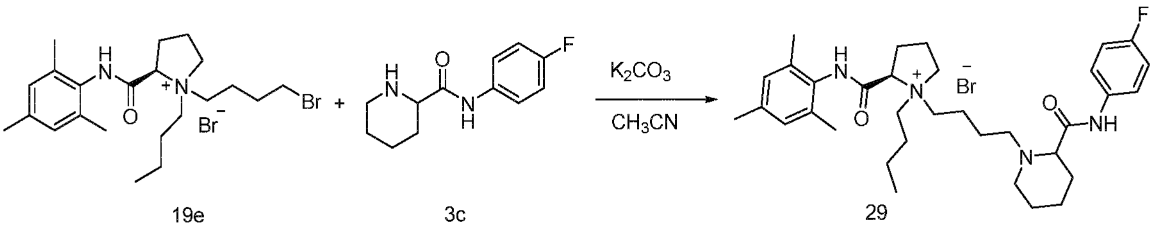


19e 3c 29

19e (1.14 mmol, 1 eq, 0.57 g) was weighed and added to analytically pure acetonitrile (10 eq of 19e, V/m, 5.7 mL) and stirred to dissolve, analytically pure potassium carbonate (1 eq of 19e, m/m, 0.57 g) was added, and 3c (2.28 mmol, 2 eq of 19e, 0.51 g) was added. The mixture was heated to 80°C, and the reaction was monitored by TLC until the reaction was completed. The reaction solution was filtered, the filter cake was washed with acetonitrile (30 eq of 19e, V/m, 18 mL, in three times), the filtrate was collected, and white solid compound 29 was obtained by preparative separation (0.36 g, with a yield of 48.9%, HPLC>98%). $^1$H NMR (400MHz, $CD_3OD$): 7.59-7.52 (m, 2H), 7.14-7.11 (m, 2H), 6.90 (s, 2H), 4.58-4.51 (m, 1H), 3.44-3.37 (m, 1H), 3.27-3.22 (m, 6H), 2.43-2.41 (m, 4H), 2.26-1.92 (m, 11H), 1.97-1.30 (m, 16H), 0.99-0.91 (m, 3H). MS m/z (ESI) = 565.31 $[M]^+$.

**Example 30**

Synthesis of 20e

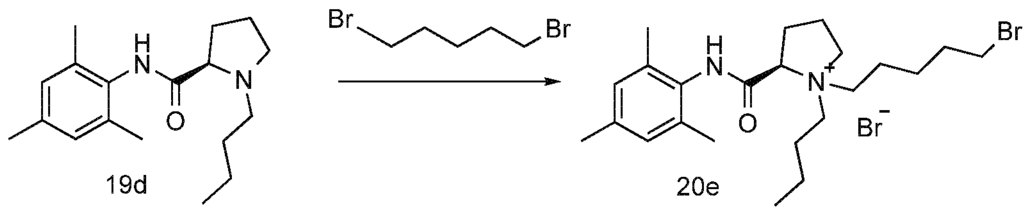

19d 20e

19d (5.0 mmol, 1.0 eq, 2.75 g) was weighed and added to 1,5-dibromopentane (2.0 eq of 19e, V/m, 6 mL), and the mixture was stirred and heated to 100°C, and detected by TCL until the reaction was complete. Silica gel column chromatography was performed, with the eluant: $CH_2Cl_2$:MeOH = 20:1. The eluate was collected and concentrated by rotary evaporation to obtain yellowish-brown oily liquid compound 20e (1.8 g, with the yield of 76.3%, HPLC>90%). MS m/z (ESI) = 437.22 $[M]^+$, 439.21 [M+2H].

Synthesis of compound 30

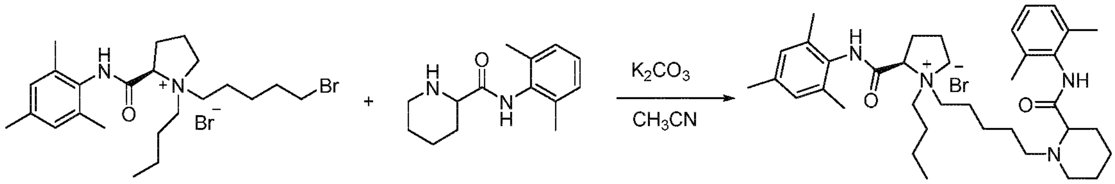


20e 1c 30

20e (1.14 mmol, 1 eq, 0.59 g) was weighed and added to analytically pure acetonitrile (10 eq of 20e, V/m, 5.0 mL) and stirred to dissolve, analytically pure potassium carbonate (1 eq of 20e, m/m, 0.59 g) was added, and 1c (2.28 mmol, 2 eq of 20e, 0.53 g) was added. The mixture was heated to 80°C, and the reaction was monitored by TLC until the reaction was completed. The reaction solution was filtered, the filter cake was washed with acetonitrile (30 eq of 20e, V/m, 18 mL, in three times), the filtrate was collected, and white solid compound 30 was obtained by preparative separation (0.38 g, with a yield of 50.0%, HPLC>98%). $^1H$ NMR (400MHz, $CD_3OD$): δ 10.02 (s, 2H), 7.05 (s, 3H), 6.90 (s, 2H), 4.61-4.58 (m, 1H), 3.44-3.39 (m, 1H), 3.22-3.16 (m, 4H), 2.51-2.41 (m, 4H), 2.26-2.13 (m, 17H), 1.97-1.55 (m, 7H), 1.49-1.29 (m, 11H), 0.95-0.90 (m, 3H). MS M/z (ESI) = 588.44 $[M]^+$.

**Example 31**

Synthesis of compound 31

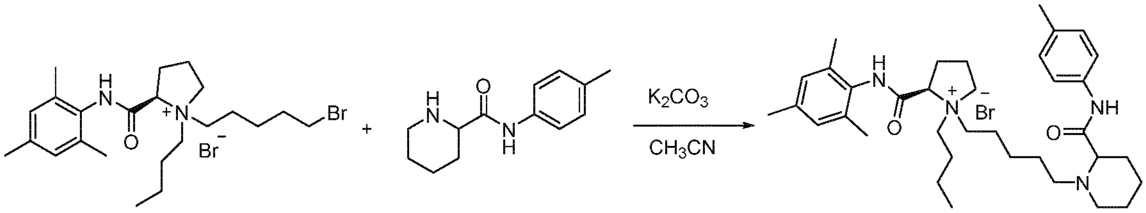


20e 4c 31

20e (1.14 mmol, 1 eq, 0.59 g) was weighed and added to analytically pure acetonitrile (10 eq of 20e, V/m, 5.9 mL) and stirred to dissolve, analytically pure potassium carbonate (1 eq of 20e, m/m, 0.59 g) was added, and 4c (2.28 mmol, 2 eq of 20e, 0.50 g) was added. The mixture was heated to 80°C, and the reaction was monitored by TLC until the reaction was completed. The reaction solution was filtered, the filter cake was washed with acetonitrile (30 eq of 20e, V/m, 18 mL, in three times), the filtrate was collected, and white solid compound 31 was obtained by preparative separation (0.45 g, with a yield of 60.4%, HPLC>98%). $^1H$ NMR (400MHz, $CD_3OD$): 7.44 (d, 2H), 7.10 (d, 2H), 6.90 (s, 2H), 4.58-4.51 (m, 1H), 3.44-3.38 (m, 1H), 3.22-3.10 (m, 4H), 2.51-2.00 (m, 18H), 1.97-1.55 (m, 7H), 1.49-1.29 (m, 11H),

0.98-0.93 (m, 3H). MS m/z (ESI) = 575.37 $[M]^+$.

**Example 32**

Synthesis of compound 32

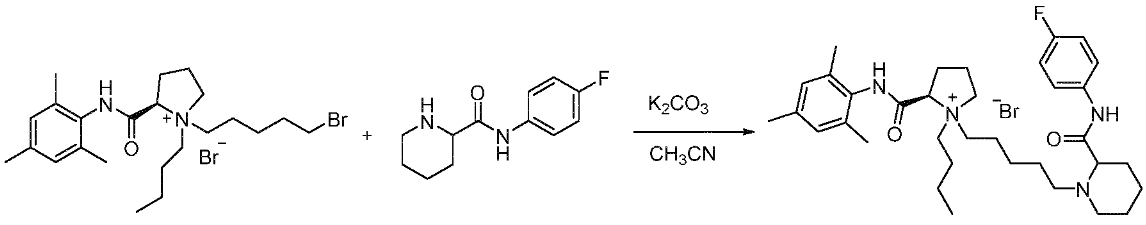


20e 3c 32

20e (1.14 mmol, 1 eq, 0.59 g) was weighed and added to analytically pure acetonitrile (10 eq of 20e, V/m, 5.9 mL) and stirred to dissolve, analytically pure potassium carbonate (1 eq of 20e, m/m, 0.59 g) was added, and 3c (2.28 mmol, 2 eq of 20e, 0.51 g) was added. The mixture was heated to 80°C, and the reaction was monitored by TLC until the reaction was completed. The reaction solution was filtered, the filter cake was washed with acetonitrile (30 eq of 20e, V/m, 18 mL, in three times), the filtrate was collected, and white solid compound 32 was obtained by preparative separation (0.31 g, with a yield of 41.3%, HPLC>98%). $^1H$ NMR (400MHz, $CD_3OD$): 7.59-7.51 (m, 2H), 7.14-7.10 (m, 2H), 6.90 (s, 2H), 4.58-4.53 (m, 1H), 3.44-3.38 (m, 1H), 3.27-3.17 (m, 6H), 2.51-2.41 (m, 4H), 1.26-1.92 (m, 12H), 1.76-1.29 (m, 17H), 0.89-0.85 (m, 3H). MS m/z (ESI) = 579.39 $[M]^+$.

**Biological test**

The experimental methods in the following test examples are all routine experimental methods unless otherwise specified. The test materials used in the following test examples were all purchased from conventional biochemical reagent stores, unless otherwise specified.

**Test example 1. Local anesthetic effect of nerve block of the compound of the present application**

Three groups of fully acclimatized rats, one in each group, were given to the example compounds and levobupivacaine hydrochloride positive control, respectively.

Solution preparation: 2 mg/mL levobupivacaine hydrochloride injection: 2.670 mL of 7.5 mg/mL levobupivacaine hydrochloride injection (batch number: 92S0702) was pipetted by a pipette and added into a 10 mL volumetric flask, 0.9% NaCl solution was added until the concave liquid level was flush with the scale line, and the volumetric flask was turned upside down to mix the solution well to prepare 2 mg/mL levobupivacaine hydrochloride injection. 6 mg/mL (the molar concentration calculated from the molecular weight of the example compound ranged from 8.6 to 10.1 mmol/L) example compound solution: 19.80 mg of example compound was accurately weighed and added with 3.3 mL of 0.9% NaCl solution to prepare 6 mg/mL example compound solution.

Each rat was administered or control injected at 5 mL/Kg, in a single dose through right sciatic nerve, and thereafter, the rat was placed on a hot plate of a preheated 50±0.1°C smart hot plate apparatus. The time required for the occurrence of the behavioral phenomena of avoiding thermal stimuli, such as obvious foot lifting and foot licking on the administered side of the rat was recorded, as the hot incubation period. The allowable maximum value of the hot incubation period was controlled at 60s. If the rat still has no foot licking after 60s, the hind foot of the rat was lifted manually to avoid tissue injury or hyperalgesia, and 60s was taken as the hot incubation period of the rat. After each measurement, the hot plate was wiped in time to remove possible urine and feces, and was dried for subsequent testing.

Analytical method

The results of the hot incubation period were expressed with maximum proportionality effect (MPE) and calculated using the following equation:

$$MPE (\%) = (C-B)/(P-B) * 100\%$$

wherein B represents the basic hot incubation period of rats, P represents the allowable maximum hot incubation period, and C represents the hot incubation period at the detection time point.

It was specified that the tested time below 50% of the maximum proportionality effect was determined to be invalid, and the test was stopped if two consecutive tests were both below 50% of the maximum proportionality effect.

Test results:

Table 1 shows data of local anesthetic effect of the compounds in the present application

| Drug to be tested | Onset time /min | Duration/hour |
|---|---|---|
| Compound 1 | 1 | 24 |
| Compound 2 | 1 | 15 |
| Compound 3 | 1 | 18 |
| Compound 4 | 1 | 48 |
| Compound 6 | 1 | 12 |
| Compound 7 | 1 | 30 |
| Compound 8 | 1 | 48 |
| Compound 9 | 1 | 24 |
| Compound 11 | 1 | 11 |
| Compound 12 | 1 | 48 |
| Compound 17 | 1 | 12-24 |
| Compound 18 | 1 | 20 |
| Compound 19 | 2 | 24 |
| Compound 20 | 1 | 12-24 |
| Compound 21 | 1 | 24-48 |
| Compound 22 | 1 | 24-48 |
| Compound 23 | 2 | 12-24 |
| Compound 24 | 1 | 60 |
| Compound 25 | 1 | 24 |
| Compound 27 | 1 | 12-24 |
| Compound 28 | 1 | 12-24 |
| Compound 29 | 1 | 24-48 |
| Compound 30 | 2 | 24-48 |
| Compound 31 | 1 | 12-24 |
| Compound 32 | 1 | 12-24 |
| Levobupivacaine hydrochloride | | 3 |

The experimental results showed that the compound of the present application could produce relatively good local anesthetic effect in sciatic nerve block models, with a fast onset of action. In the range of equivalent molar concentrations of 8.6-10.1 mmol/L, the single-drug anesthesia duration was greater than or equal to 11 hours, which was significantly longer than that of the positive control levobupivacaine hydrochloride. The single-drug anesthesia duration

of some compounds was greater than 24 hours, and the single-drug local anesthesia duration of some other compounds was greater than 48 hours, with the onset time of 1 min.

**Test example 2. Subcutaneous infiltration local anesthetic effect of the compounds of the present application**

SD rats (half male and half female) with a body weight of 190-210 grams were shaved and sterilized on the back, then a circle with a diameter of about 1.5 cm was drawn on one side of the bare back, and 0.5 mL of a drug-containing solution was subcutaneously injected into the skin in the center: normal saline was used as the solvent, 2 mg/mL levobupivacaine hydrochloride, 9 compounds of the present application were selected, at a concentration of 6 mg/mL (the equivalent molar concentration ranged from 8.6 to 10.1 mmol/L according to the corresponding molecular weights of the example compounds), with 10 rats in each group. Von Frey fiber filaments with 100 g strength were bound to a needle for topical skin stimulation. 1 min after drug injection, response of rats to stinging stimulus was tested. The test sites were six different points of the circle of the administration site, and whether behaviors such as skin contraction and avoidance occurred after stimulus was observed and recorded. Three points in the center of the circle and three points on the periphery of the circle were stimulated separately, and the number of occurrences of skin contraction and avoidance behavior was recorded as N/6. In terms of judgment, if there were 4 or more occurrences of avoidance and skin contraction behaviors, it was considered that the drug was ineffective; if there was a reaction on the periphery (≤3) and no reaction (≥1) in the center, it was considered that the drug was still effective; and if there was a reaction only at the points in the center, it was considered that the drug was ineffective. Each compound was tested with 10 rats.

Test results:

Table 2 shows subcutaneous infiltration local anesthetic effect of the compounds (6 mg/mL) of the present application

| Drug to be tested | Onset time/min | Duration/h |
|---|---|---|
| Compound 1 | 1 | 55 |
| Compound 4 | 1 | 72-96 |
| Compound 7 | 2 | 52 |
| Compound 8 | 1 | 72-96 |
| Compound 12 | 1 | 72-96 |
| Compound 21 | 1 | 57 |
| Compound 22 | 1 | 60 |
| Compound 24 | 2 | 72-96 |
| Compound 29 | 1 | 50 |
| Compound 30 | 1 | 56 |
| Levobupivacaine hydrochloride | | 4 |

The experimental results showed that the compounds of the present application, when at a concentration of 6 mg/mL (equivalent molar concentration ranged from 8.6 to 10.1 mmol/L), could produce local anesthetic effect for more than 48 hours in rat subcutaneous infiltration models, with a fast onset of action. Some of the compounds could produce local anesthetic effect for more than 72 hours, with the onset time of 1 min.

Since the local anesthesia time longer than 72 hours may cause irreversible nerve injury, we subsequently halved (3 mg/mL) the dose of the compounds with the duration of action greater than 72 hours and then conducted an activity test to determine whether the local anesthesia effect could be restored in the skin infiltration models.

Table 3 shows subcutaneous infiltration local anesthetic effect of the compounds (3 mg/mL) of the present application

| Drug to be tested | Onset time/min | Duration/h |
|---|---|---|
| Compound 4 | 1 | 56 |
| Compound 8 | 1 | 52 |

(continued)

| Drug to be tested | Onset time/min | Duration/h |
|---|---|---|
| Compound 12 | 1 | 60 |
| Compound 24 | 1 | 63 |
| Levobupivacaine hydrochloride | | 3 |

The experimental results showed that the compounds of the present application, when at a concentration of 3 mg/mL (equivalent molar concentration ranged from 4.4 to 5.1 mmol/L), could still produce local anesthetic effect for more than 48 hours in rat subcutaneous infiltration models, and the local anesthetic effect could be restored.

**Test example 3. Evaluation of neuropathological injury of the compounds of the present application**

Fully acclimatized SD rats (half male and half female) with a body weight of 190-210 g were given to the example compounds 4, 8, 12, 24, levobupivacaine hydrochloride positive control group, and solvent control group, 8 rats in each group.

Administration concentration: normal saline was used as the solvent, 2 mg/mL levobupivacaine hydrochloride, two concentrations of the compounds of the present application: 6 mg/mL and 12 mg/mL, and the solvent control group: normal saline.

The injection volume for each rat was 1.0 ml, and the injection site was the vicinity of the sciatic nerve of rats. On the 7th and 14th days after sciatic nerve injection, the experimental rats were euthanized by injecting levobupivacaine hydrochloride into their hearts under anesthesia with isoflurane. About 1.5 cm of the sciatic nerve at the injection site was taken, preserved in 10% formaldehyde solution for 48 h, HE stained and cut into 5 $\mu$m thick slices.

Fully acclimatized SD rats (half male and half female) with a body weight of 190-210 g were given to the compounds 4, 8, 12, 24 prepared in the examples, levobupivacaine hydrochloride positive control group, and solvent control group, 8 rats in each group.

Administration concentration: normal saline was used as the solvent, 2 mg/mL levobupivacaine hydrochloride, two concentrations of the compounds of the present application: 3 mg/mL and 6 mg/mL, and the solvent control group: normal saline.

Each rat was injected with an injection volume of 1 mL, which was injected subcutaneously in the back of rats. On the 7th and 14th days after subcutaneous injection, the experimental rats were euthanized by injecting levobupivacaine hydrochloride into their hearts under anesthesia with isoflurane. Skin tissue was taken from the injection site, preserved in 10% formaldehyde solution for 48 h, HE stained and cut into 5 $\mu$m thick slices.

The evaluation results of neuropathological injury showed: compared with levobupivacaine hydrochloride positive control group and solvent control group, the example compounds had good safety, with no significant differences in nerve injury, vascular proliferation, degree of demyelination, muscle inflammation and degree of connective tissue inflammation.

**Test example 4. Study of toxicity of the compounds in the present application in single dose**

Example compounds 4, 8, 12, 24 were administered to fully acclimatized SD test rats with a body weight of 190-210 g, 4 rats in each group.

Dosage: normal saline was used as the solvent, and the concentration of the compounds 4, 8, 12 and 24 in the present application was 6mg/kg.

Administration mode: each rat was administered through tail vein according to the administration volume of 5 mL/kg.

Fully acclimatized SD test rats with a body weight of 190-210 g were given to the example compounds 4, 8, 12, 24, levobupivacaine hydrochloride positive control group, 4 rats in each group.

Dosage: normal saline was used as the solvent, and the concentration of the compounds 4, 8, 12 and 24 in the present application was 30mg/kg.

Administration mode: each rat was injected subcutaneously according to the administration volume of 5 mL/kg.

The results showed that after administration of the compounds of the present application at a dose of 6 mg/kg in tail vein and 30 mg/kg subcutaneously, the rats showed no obvious abnormality and showed active behavior, and were not significantly different from normal rats.

To sum up, the present application provides a quaternary ammonium salt compound with a novel structure, a preparation method and use thereof. The compound takes effect quickly, has long-term local anesthesia effect after

single administration, and has good safety.

Multiple examples are described in the present application, but the description is exemplary rather than limiting, and it is obvious for those of ordinary skill in the art that there may be more examples and implementation solutions within the scope of the examples described in the present application.

## Claims

1. A quaternary ammonium salt compound represented by Formula (I), or a tautomer, geometric isomer, enantiomer, diastereomer thereof, or a mixture form thereof, or a pharmaceutically acceptable salt thereof:

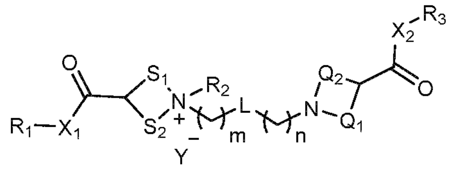

(I)

wherein:

$R_1$ is selected from an aromatic hydrocarbon group and heteroaryl, herein the aromatic hydrocarbon group and heteroaryl are optionally substituted by one or more of the following groups: $C_{1-6}$ alkyl, $C_{1-6}$ alkoxy, cyano, halogen, hydroxyl, amino, nitro, an ester group, mono $C_{1-6}$ alkyl amino, di-$C_{1-6}$ alkyl amino, $C_{2-4}$ alkenyl, $C_{2-4}$ alkynyl, $C_{1-6}$ haloalkyl, and $C_{1-6}$ haloalkoxy;
$R_2$ is selected from $C_{1-18}$ alkyl and $C_{3-12}$ cycloalkyl, herein, the $C_{1-18}$ alkyl and $C_{3-12}$ cycloalkyl are optionally substituted by one or more of the following groups: $C_{1-6}$ alkyl, $C_{1-6}$ alkoxy, $C_{1-6}$ haloalkyl, $C_{3-6}$ cycloalkyl, halogen, hydroxyl, cyano, and amino;
$R_3$ is selected from $C_{1-8}$ alkyl, $C_{3-12}$ cycloalkyl, an aromatic hydrocarbon group, heteroaryl and heterocycloalkyl, wherein optionally, herein, the $C_{1-8}$ alkyl, $C_{3-12}$ cycloalkyl, aromatic hydrocarbon group, heteroaryl, and heterocycloalkyl are optionally substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, $C_{2-4}$ alkynyl, an optionally substituted aromatic hydrocarbon group, optionally substituted heteroaryl, optionally substituted heterocycloalkyl; herein, the optionally substituted aromatic hydrocarbon group, optionally substituted heteroaryl, optionally substituted heterocycloalkyl refer to an unsubstituted aromatic hydrocarbon group, unsubstituted heteroaryl, and unsubstituted heterocycloalkyl; or the aromatic hydrocarbon group, heteroaryl, and heterocycloalkyl are substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, and $C_{2-4}$ alkynyl;
$X_1$ and $X_2$ are each independently selected from O, S, and $NR_4$, wherein $R_4$ is hydrogen, deuterium, $C_{1-8}$ alkyl, $C_{3-8}$ cycloalkyl, $C_{1-8}$ alkoxy $C_{1-8}$ alkyl, or $NR_4$ and $R_1$ or $R_3$ connected thereto together form azacycloalkyl;
m and n are each independently selected from an integer of 0-8, and m and n are the same or different;
L is selected from $C_{1-8}$ alkylene, $C_{2-8}$ alkenylene, $C_{2-8}$ alkynylene, and $C_{3-8}$ cycloalkylene, wherein optionally, herein, a carbon atom on the main chain of the $C_{1-8}$ alkylene and $C_{3-8}$ cycloalkylene is optionally substituted by 1-3 heteroatoms selected from O, S, and N, wherein N may be substituted by $R_5$, $R_5$ is hydrogen, deuterium, $C_{1-4}$ alkyl; the $C_{1-8}$ alkylene, $C_{2-8}$ alkenylene, $C_{2-8}$ alkynylene and $C_{3-8}$ cycloalkylene are optionally substituted by one or more of the following groups: $C_{1-4}$ alkyl, $C_{1-4}$ alkoxy, $C_{1-4}$ haloalkyl, $C_{1-4}$ haloalkoxy, $C_{3-6}$ cycloalkyl, halogen, hydroxyl, cyano, amino, an ester group, nitro, mono $C_{1-4}$ alkyl amino, di-$C_{1-4}$ alkyl amino, $C_{2-4}$ alkenyl, and $C_{2-4}$ alkynyl;
$S_1$, $S_2$, $Q_1$, and $Q_2$ are each independently selected from a single bond and $C_{1-6}$ alkylene, herein, carbon atoms on the main chain of $C_{1-6}$ alkylene are optionally substituted by a heteroatom selected from O, S and N, wherein N may be substituted by $R_6$, $R_6$ is hydrogen or deuterium, and it is specified that $S_1$ and $S_2$ are not a single bond at the same time, $Q_1$ and $Q_2$ are not a single bond at the same time; and
$Y^-$ represents a pharmaceutically acceptable anion.

2. The compound according to claim 1, wherein in the Formula (I), $R_1$ is phenyl or naphthyl; herein, the phenyl and naphthyl are optionally substituted by one or more of the following groups: $C_{1-6}$ alkyl, $C_{1-6}$ alkoxy, cyano, halogen, hydroxyl, amino, nitro, an ester group, mono $C_{1-6}$ alkyl amino, di-$C_{1-6}$ alkyl amino, $C_{2-4}$ alkenyl, $C_{2-4}$ alkynyl, $C_{1-4}$

haloalkyl, $C_{1-6}$ haloalkoxy;

preferably, $R_1$ is phenyl, optionally substituted by one or more of the following groups: methyl, ethyl, n-propyl, isopropyl, n-butyl, sec-butyl, tert-butyl, trifluoromethyl, methoxy, trifluoromethoxy, fluorine, chlorine, bromine, iodine, hydroxyl, amino, nitro, methyl ester, ethyl ester;
more preferably, $R_1$ is phenyl, 2-methylphenyl, 2-methoxyphenyl, 4-methylphenyl, 4-methoxyphenyl, 4-fluorophenyl, 2-chlorophenyl, 4-chlorophenyl, 2-bromophenyl, 3-bromophenyl, 4-bromophenyl, 3-hydroxyphenyl, 4-trifluoromethylphenyl, 2,6-dimethylphenyl, 2,6-dimethoxyphenyl, 3-nitrophenyl, 2,6-difluorophenyl, 3-chloro-2-methylphenyl, 2,3-dichlorophenyl, 4-hydroxyphenyl, 2,4,6-trimethylphenyl, 2,4,6-trimethoxyphenyl, and 2,4,6-trifluorophenyl.

**3.** The compound according to claim 1, wherein in the Formula (I), $R_2$ is $C_{1-8}$ alkyl or $C_{3-8}$ cycloalkyl; herein, the $C_{1-8}$ alkyl, $C_{3-8}$ cycloalkyl are optionally substituted by one or more of the following groups: $C_{1-6}$ alkyl, $C_{1-6}$ alkoxy, $C_{1-6}$ haloalkyl, $C_{3-6}$ cycloalkyl, halogen, hydroxyl, cyano, and amino; more preferably, $R_2$ is methyl, ethyl, n-propyl, isopropyl, n-butyl, sec-butyl, tert-butyl, n-amyl, isoamyl, n-hexyl, cyclopropyl, cyclobutyl, cyclopropylmethylene, cyclobutyl methylene, cyclopentylmethylene, n-octyl, n-heptyl.

**4.** The compound according to claim 1, wherein in the Formula (I), $R_3$ is $C_{1-8}$ alkyl, $C_{3-7}$ cycloalkyl, an aromatic hydrocarbon group, heteroaryl, three- to eight-membered heterocycloalkyl, or $R_3$ and $X_2$, which is $NR_4$, together form azacycloalkyl, herein, the $C_{1-8}$ alkyl, $C_{3-7}$ cycloalkyl, an aromatic hydrocarbon group, heteroaryl, or three- to eight-membered heterocycloalkyl is optionally substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, $C_{2-4}$ alkynyl, optionally substituted phenyl; herein, the optionally substituted phenyl refers to unsubstituted phenyl, or phenyl is substituted by one or more of the following groups: $C_{1-6}$ alkyl, $C_{1-6}$ alkoxy, $C_{1-6}$ haloalkyl, $C_{1-6}$ haloalkoxy, $C_{3-6}$ cycloalkyl, halogen, hydroxyl, cyano, amino, an ester group, nitro, mono $C_{1-6}$ alkyl amino, di-$C_{1-6}$ alkyl amino, $C_{2-4}$ alkenyl, and $C_{2-4}$ alkynyl;
optionally, $R_3$ is methyl, ethyl, propyl, cyclopropyl, cyclobutyl, cyclopentyl, cyclohexyl, methoxyethyl, methoxypropyl, phenyl, phenylethyl, benzyl, 4-fluorobenzyl, 2-methylphenyl, 2-methoxyphenyl, 2-fluorophenyl, 2-chlorophenyl, 2-bromophenyl, 2-hydroxyphenyl, 3-methylphenyl, 3-methoxyphenyl, 3-fluorophenyl, 3-chlorophenyl, 3-bromophenyl, 3-hydroxyphenyl, 4-methylphenyl, 4-methoxyphenyl, 4-fluorophenyl, 4-chlorophenyl, 4-bromophenyl, 4-hydroxyphenyl, 4-trifluoromethylphenyl, 2,4-dimethylphenyl, 2,4-dimethoxyphenyl, 2,4-difluorophenyl, 2,4-dichlorophenyl, 2,4-dihydroxyphenyl, 2,6-dimethylphenyl, 2,6-dimethoxyphenyl, 2,6-difluorophenyl, 2,6-dichlorophenyl, 2,6-dihydroxyphenyl, 2,4,6-trimethylphenyl, 2,4,6-trimethoxyphenyl, 2,4,6-trifluorophenyl, or $R_3$ and $X_2$, which is $NR_4$, together form piperidine or pyrrolidine.

**5.** The compound according to claim 1, wherein in the Formula (I), L is selected from: $-CH_2-$, $-(CH_2)_2-$, $-(CH_2)_3-$, $-(CH_2)_4-$, $-(CH_2)_5-$, $-(CH_2)_6-$, -O-, $-CH_2OCH_2-$, $-OCH_2-$, $-CH_2O-$, $-OCH_2O-$, $-O(CH_2)_2O-$, $-O(CH_2)_3O-$, $-O(CH_2)_4O-$, $-CH_2OCH_2CH_2-$, $-CH_2CH_2OCH_2-$, $-OCH_2OCH_2O-$, $-CH_2CH_2OCH_2CH_2-$, -S-, $-CH_2SCH_2-$, $-SCH_2-$, $-CH_2S-$, $-CH_2SCH_2CH_2-$, $-CH_2CH_2SCH_2-$, $-CH_2CH_2SCH_2CH_2-$, -CH=CH-,

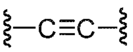,
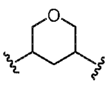,
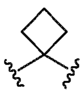,
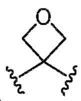,
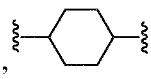,

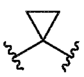,
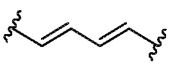,
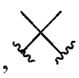,
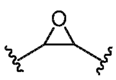,
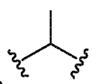.

**6.** The compound according to claim 1, wherein in the Formula (I), $X_1$ and $X_2$ are each independently O, NH, $NCH_3$, $NCH_2CH_3$, $N(CH_2)_2CH_3$, or, when $X_2$ is $NR_4$, it forms piperidine together with $R_3$;

optionally, when one of $S_1$ and $S_2$ is a single bond (i.e., atoms connected thereto are directly bond-connected), the other is $-(CH)_2)_3-$, $-(CH_2)_4-$,$-(CH_2)_5-$, $-(CH_2)_6-$, $-CH_2OCH_2CH_2-$, $-CH_2CH_2OCH_2-$, $-CH_2SCH_2CH_2-$;
optionally, when one of $Q_1$ and $Q_2$ is a single bond (i.e., atoms connected thereto are directly bond-connected), the other is $-(CH_2)_3-$, $-(CH_2)_4-$, $-(CH_2)_5-$, $-(CH_2)_6-$.

**7.** The compound according to any one of claims 1-6, wherein in the Formula (I), $Y^-$ is halogen anion, sulfate, acetate,

tartrate, p-toluenesulfonate, mesylate, citrate, preferably $Cl^-$, $Br^-$, $I^-$, $CH_3COO^-$; more preferably, $Br^-$.

**8.** The compound according to claim 1, wherein the compound of Formula (I) is selected from one of the following compounds:

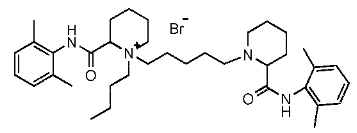
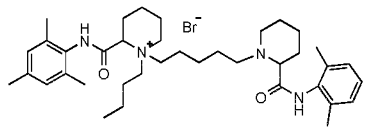
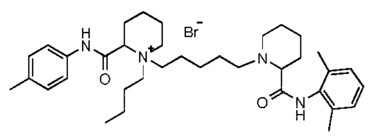
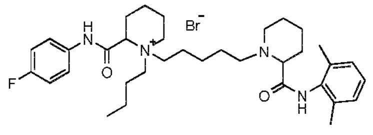
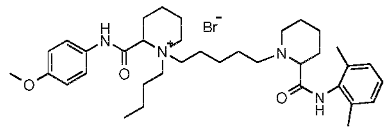
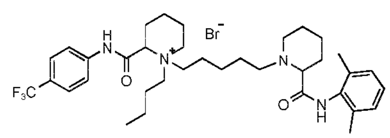
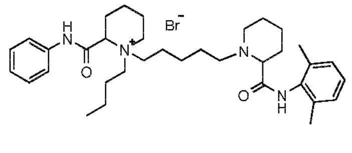
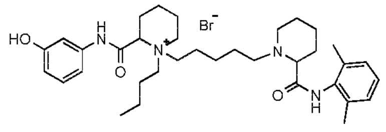
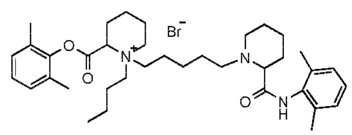
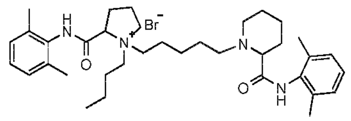
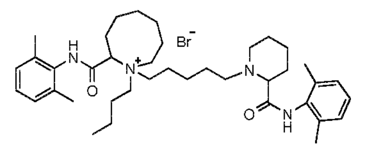
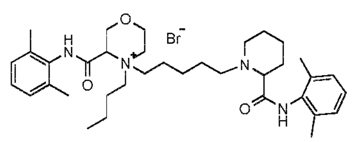
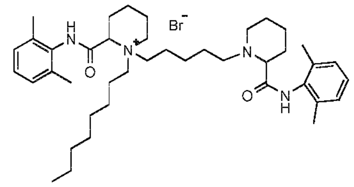
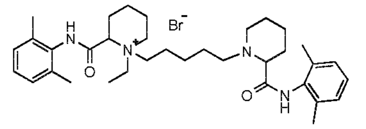
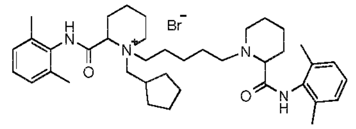
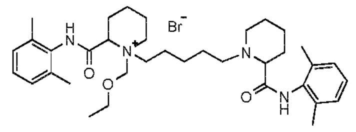
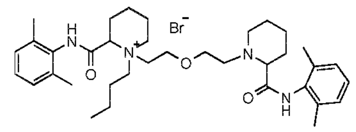
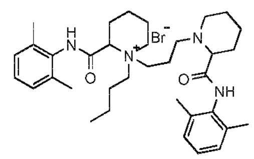
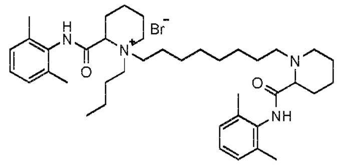
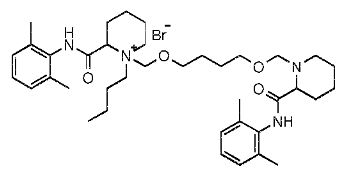
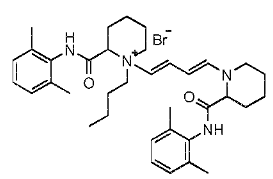

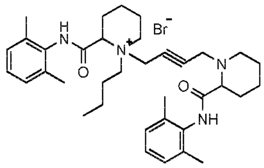
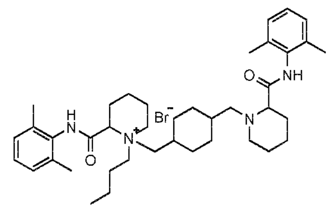
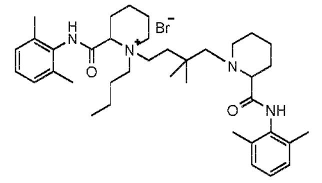

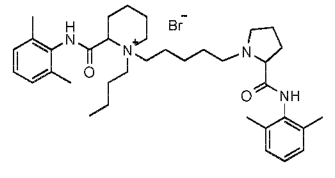
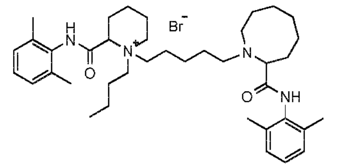
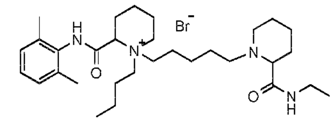

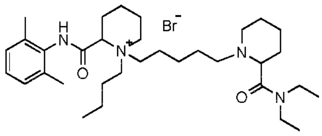
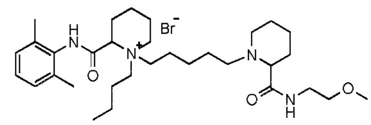
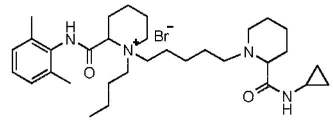

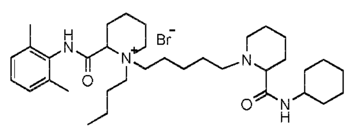
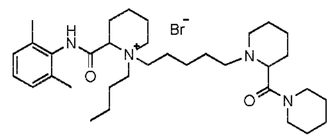
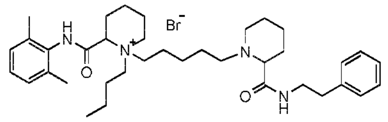

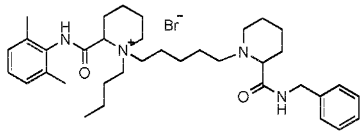
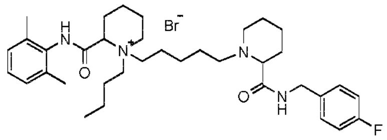
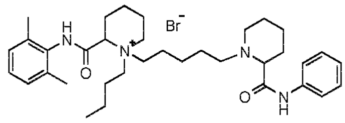

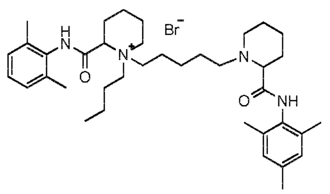
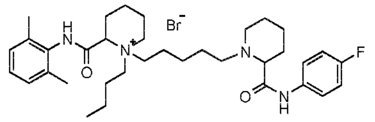
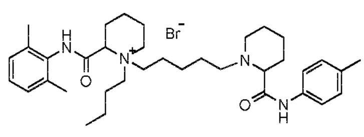

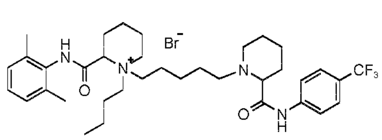
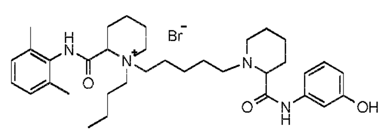
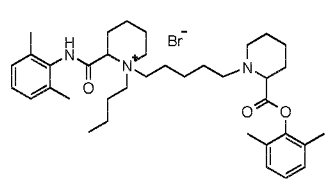

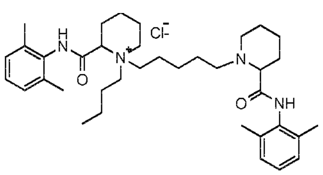
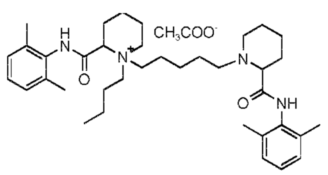

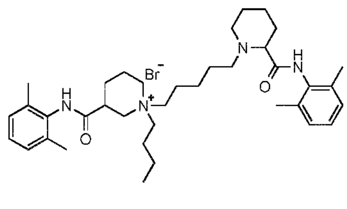

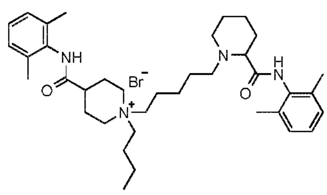

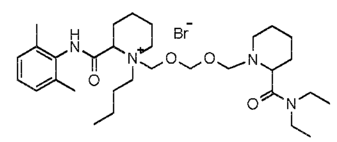

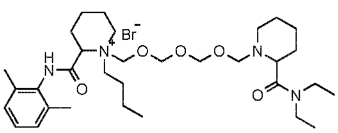

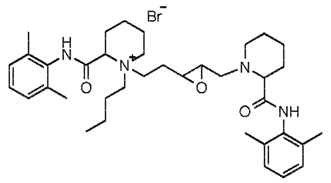

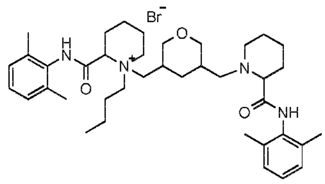

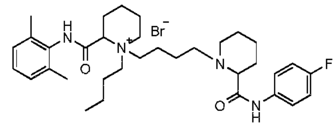

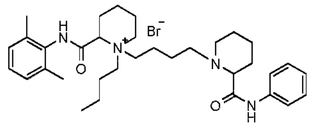

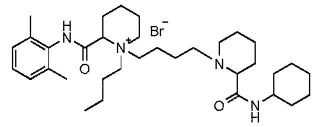

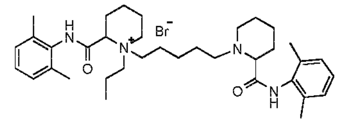

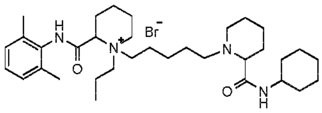

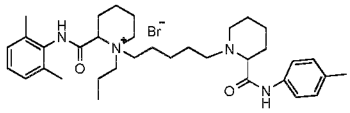

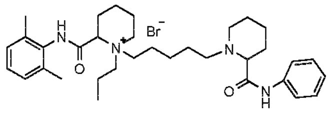

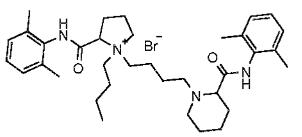

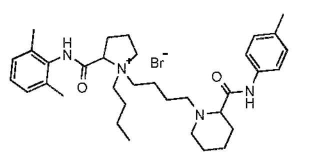

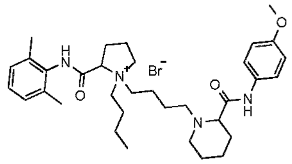

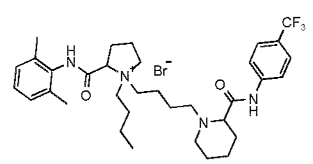

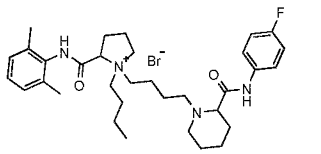

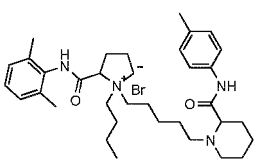

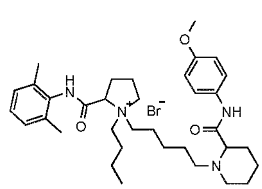

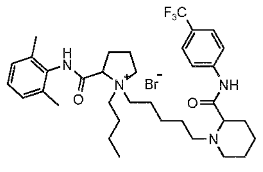
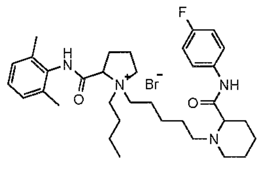
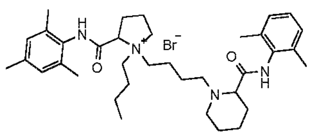

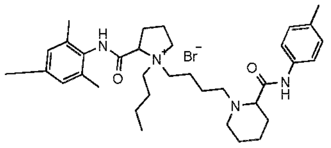
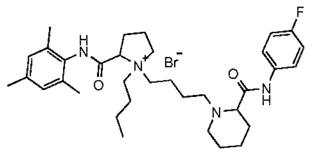
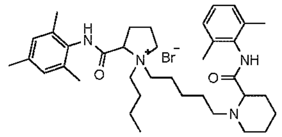

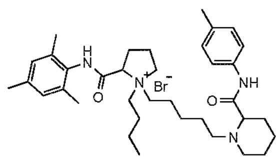
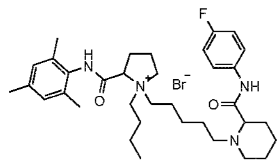

9. The compound according to claim 8, wherein the compound of Formula (I) is selected from one of the following compounds:

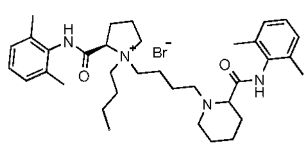
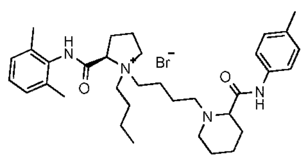

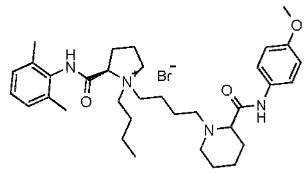
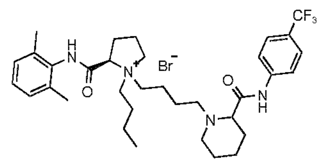
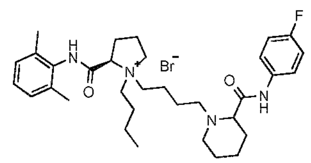

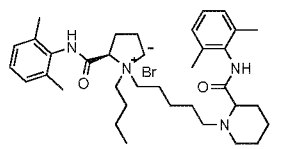
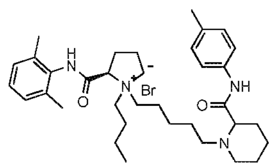
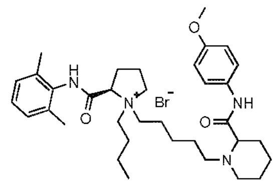

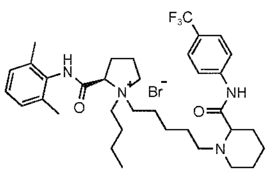
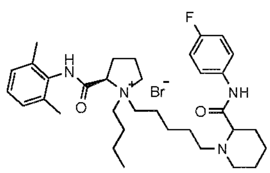
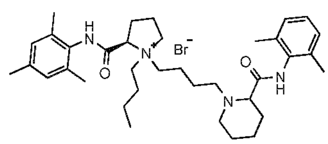

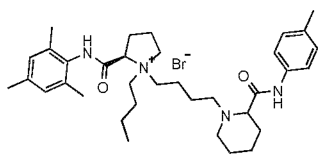
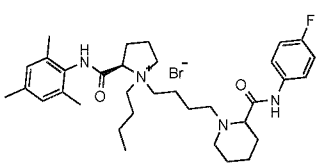
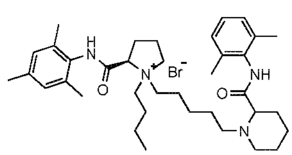

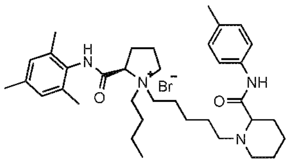

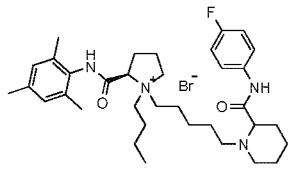

**10.** A preparation method for the compound according to any one of claims 1-9, comprising the following steps:

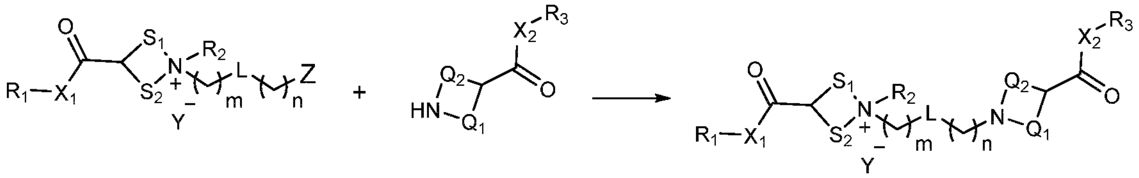

(II) (III) (I)

reacting a compound of Formula (II) with a compound of Formula (III) to obtain the compound of Formula (I); here, Z in Formula (II) is an electron-withdrawing leaving group, optionally, the leaving group is bromine, chlorine or sulfonate, and definitions of groups in Formula (II) and Formula (III) are the same as those in Formula (I).

**11.** A pharmaceutical composition, comprising the compound of Formula (I) according to any one of claims 1-9, a tautomer, geometric isomer, enantiomer, diastereomer thereof, or a mixture form thereof, or a pharmaceutically acceptable salt thereof, and a pharmaceutically acceptable carrier excipient or a diluent.

**12.** The compound of Formula (I) according to any one of claims 1-9, a tautomer, geometric isomer, enantiomer, diastereomer thereof, or a mixture form thereof, or a pharmaceutically acceptable salt thereof, for use as a medication.

**13.** Use of the compound of Formula (I) according to any one of claims 1-9, a tautomer, geometric isomer, enantiomer, diastereomer thereof, or a mixture form thereof, or a pharmaceutically acceptable salt thereof, or the pharmaceutical composition according to claim 11 in the preparation of anesthetic or analgesic drugs.

**14.** The use according to claim 13, wherein the anesthesia is local anesthesia; pain suitable for the analgesia is chronic pain, acute pain, inflammatory pain, cancer pain, neuropathic pain, musculoskeletal pain, primary pain, intestinal pain or idiopathic pain; preferably, the anesthesia is conduction anesthesia, topical anesthesia or infiltration anesthesia.

**15.** A method for anesthesia or analgesia, comprising administering the compound of General Formula (I) according to any one of claims 1-9, a tautomer, geometric isomer, enantiomer, diastereomer thereof, or a mixture form thereof, or a pharmaceutically acceptable salt thereof, or the pharmaceutical composition according to claim 11 to a patient in need thereof.

**16.** The method according to claim 15, wherein the anesthesia is local anesthesia.

**INTERNATIONAL SEARCH REPORT**

International application No.
**PCT/CN2022/095336**

**A. CLASSIFICATION OF SUBJECT MATTER**

C07D 211/60(2006.01)i; C07D 225/02(2006.01)i; C07D 401/06(2006.01)i; A61K 31/452(2006.01)i; A61P 23/02(2006.01)i

According to International Patent Classification (IPC) or to both national classification and IPC

**B. FIELDS SEARCHED**

Minimum documentation searched (classification system followed by classification symbols)

C07D211/-; C07D225/-; C07D401/-; A61K31/-; A61P23/-

Documentation searched other than minimum documentation to the extent that such documents are included in the fields searched

Electronic data base consulted during the international search (name of data base and, where practicable, search terms used)

WPI, EPODOC, CNPAT, CNKI, Caplus(STN), Registry(STN): 季铵盐, 麻醉, 酰胺, 苯, quaternary ammonium salt, anesthetic, amide, phenyl, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | CN 114075184 A (WEST CHINA HOSPITAL, SICHUAN UNIVERSITY) 22 February 2022 (2022-02-22)<br>claims 1, 11, and 23-26 | 1-16 |
| A | CN 112574098 A (WEST CHINA HOSPITAL, SICHUAN UNIVERSITY) 30 March 2021 (2021-03-30)<br>claims 1-10 | 1-16 |
| A | CN 111153851 A (WEST CHINA HOSPITAL, SICHUAN UNIVERSITY) 15 May 2020 (2020-05-15)<br>claims 1-11 | 1-16 |
| A | US 2021107872 A1 (WEST CHINA HOSPITAL, SICHUAN UNIVERSTRY) 15 April 2021 (2021-04-15)<br>claims 1-24 | 1-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 August 2022** | **02 September 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/095336**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15-16**
because they relate to subject matter not required to be searched by this Authority, namely:

[1] Claims 15-16 relate to a disease treatment method. However, a search was made on the basis that said claims set forth an application of a compound in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/095336**

| Patent document cited in search report | Publication date (day/month/year) | Patent family member(s) | Publication date (day/month/year) |
|---|---|---|---|
| CN 114075184 A | 22 February 2022 | WO 2022037590 A1 | 24 February 2022 |
| CN 112574098 A | 30 March 2021 | None | |
| CN 111153851 A | 15 May 2020 | WO 2020156359 A1 | 06 August 2020 |
| | | JP 2022518599 A | 15 March 2022 |
| | | EP 3919476 A1 | 08 December 2021 |
| | | US 2022135526 A1 | 05 May 2022 |
| US 2021107872 A1 | 15 April 2021 | CN 110156666 A | 23 August 2019 |
| | | EP 3750870 A1 | 16 December 2020 |
| | | JP 2021518839 A | 05 August 2021 |
| | | WO 2019154287 A1 | 15 August 2019 |
| | | WO 2019154288 A1 | 15 August 2019 |
| | | CN 110156665 A | 23 August 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110587550
- CN 110156665 B
- CN 110156666 A
- CN 101050200 A


### Non-patent literature cited in the description


- **BECKER D E. et al.** Local anesthetics: review of pharmacological considerations. *Anesth Prog,* 2012, vol. 59 (2), 90-102
- **COURTNEY KR.** Mechanism of frequency-dependent inhibition of sodium currents in frog myelinated nerve by the lidocaine derivative GEA. *J Pharmacol Exp Ther,* 1975, vol. 195, 225-236
- **ALSALEM M et al.** Anti-nociceptive and desensitizing effects of olvanil on capsaicin-induced thermal hyperalgesia in the rat. *BMC Pharmacol Toxicol.,* 2016, vol. 17 (1), 31